# EUROPEAN PATENT APPLICATION

(11) **EP 4 501 906 A1**
(43) Date of publication of application: **05.02.2025**
(21) Application number: 23780886.0
(22) Date of filing: 30.03.2023
(51) Int. Cl.: C07C 229/50, A61K 31/136, A61K 31/343, A61K 31/4418, A61K 31/47, A61K 51/00, A61P 35/00, C07D 213/24, C07D 215/20, C07D 307/83, A61K 101/02

(54) **TETRAHYDRONAPHTHALENE DERIVATIVE**

(30) Priority: 31.03.2022 JP 2022058114
(71) Applicant: Atransen Pharma Ltd., Osaka-shi, Osaka 541-0048 (JP); Stella Pharma Corporation, Osaka-shi, Osaka 541-0043 (JP); OSAKA UNIVERSITY, Suita-shi Osaka 565-0871 (JP)
(72) Inventor: KANAI,Yoshikatsu, Suita-shi, Osaka 565-0871 (JP); UEHARA,Kohki, Osaka-shi, Osaka 541-0043 (JP); OHTA,Yoichiro, Osaka-shi, Osaka 541-0043 (JP); TAKENAKA,Hiroshi, Osaka-shi, Osaka 541-0043 (JP); MASUI,Shin, Osaka-shi, Osaka 541-0048 (JP)
(74) Representative: Cabinet Beau de Loménie
(86) International application number: PCT/JP2023/013197
(87) International publication number: WO 2023/190878

(57) **Abstract**

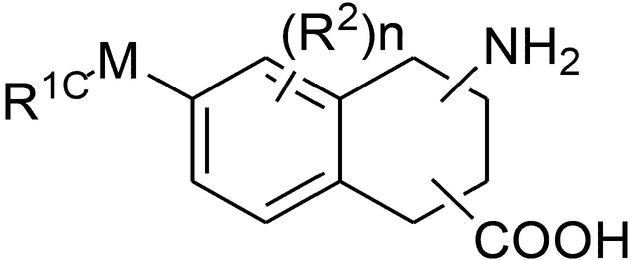

Provided is a tetrahydronaphthalene derivative that can be used as an LAT1-selective inhibitor and an LAT1-selective substrate. The present invention relates to a compound represented by formula (I) or a pharmaceutically acceptable salt thereof. In formula (I), M represents S, O, NH or the like; R^{1C} represents any one structure selected from the group consisting of a 5- to 7-membered aromatic heterocyclic group having a substituent, a C5- to 7-membered aromatic cyclic group having a substituent and a substituted or unsubstituted 8- to 16-membered polycyclic group, or the like; and n represents an integer of 0 to 5. The present invention also relates to a composition for treating cancer, a medicine for BNCT, a diagnostic drug for cancer, an LAT1-selective inhibitor, or a composition for enhancing a BNCT effect, each containing the compound or the pharmaceutically acceptable salt thereof.

## Description

### BACKGROUND OF THE INVENTION

### Field of the Invention

The present invention relates to a tetrahydronaphthalene derivative.

### Description of the Related Art

Among amino acid transporters that are membrane proteins required for intracellular uptake of amino acids, an L-type amino acid transporter 1 (LAT1) is an amino acid transporter discovered by one of the inventors in 1998, and is a membrane protein responsible for uptake of amino acids into cancer cells. Since LAT1 is expressed with high specificity in cancer cells, many attempts have been made in Japan and other countries as targets for diagnosis and treatment of cancer. Although not present in normal cells in most tissues, LAT1 is specifically expressed in cancer cells and is responsible for supplying amino acids as nutrients in cancer tissues (Non-Patent Document 1 and Non-Patent Document 2). The amino acid taken up in the cancer cell by LAT1 does not serve as a nutrient but is responsible for regulating protein synthesis, intracellular metabolism, and cell growth via an amino acid signal system (Non-Patent Document 2).

In addition, amino acids incorporated into cancer cells by LAT1 are responsible for regulating protein synthesis, intracellular metabolism, and cell growth through an amino acid signaling system and not only as nutrients.

LAT1 inhibitory drugs that inhibit the function of LAT1 specifically expressed in cancer cells to induce cancer-specific nutrient starvation and suppress protein synthesis, intracellular metabolism, and cell growth via an amino acid signal system to reduce cancer tissues have been developed.

Compounds that are transported by LAT1 to exhibit accumulability in cancer cells and can release α rays have also been developed (Non-Patent Document 3 and Non-Patent Document 4).

A boron compound that is transported by LAT1 to exhibit accumulability in cancer cells and is used for boron delivery to cancer cells in boron neutron capture therapy (BNCT) has also been developed (Non-Patent Document 2) .

Radioactive compounds that are transported by LAT1 to exhibit accumulability in cancer cells and are used for detection and diagnosis of cancer by PET or SPECT have also been developed (Non-Patent Document 1 and Non-Patent Document 2).

### Prior Art Document

### Non-Patent Documents

Non-Patent Document 1: Kanai Y, Segawa H, Miyamoto Ki, Uchino H, Takeda E, Endou H. Expression cloning and characterization of a transporter for large neutral amino acids activated by the heavy chain of 4F2 antigen (CD98). J Biol Chem. 1998 Sep 11;273(37):23629-32. doi: 10.1074/jbc.273.37.23629.
Non-Patent Document 2: Kanai Y. Amino acid transporter LAT1 (SLC7A5) as a molecular target for cancer diagnosis and therapeutics. Pharmacol Ther 230: Feb. 2022, 107964.
Non-Patent Document 3: Kaneda-Nakashima K, Zhang Z, Manabe Y, Shimoyama A, Kabayama K, Watabe T, Kanai Y, Ooe K, Toyoshima A, Shirakami Y, Yoshimura T, Fukuda M, Hatazawa J, Nakano T, Fukase K, Shinohara A. α-Emitting cancer therapy using 211At-AAMT targeting LAT1.Cancer Sci. 2021 Mar;112(3):1132-1140. doi: 10.1111/cas.14761.
Non-Patent Document 4: Watabe T, Kaneda-Nakashima K, Shirakami Y, Liu Y, Ooe K, Teramoto T, Toyoshima A, Shimosegawa E, Nakano T,Kanai Y, Shinohara A, Hatazawa J. Targeted alpha therapy using astatine (211At)-labeled phenylalanine: A preclinical study in glioma bearing mice. Oncotarget. 2020 Apr 14;11(15):1388-1398. doi: 10.18632/oncotarget.27552.

### SUMMARY OF THE INVENTION

### Problems to be solved

A drug having an ability to inhibit the uptake of amino acids into cancer cells via LAT1 and exhibiting anticancer action is also known, but development of a drug having high LAT1 affinity, a drug having a sustained LAT1 inhibitory effect, or a drug capable of effectively maintaining a blood concentration is required.

LAT1 has an exchange transport function, and when there is an amino acid outside the cell, even if an existing drug such as L-BPA is once delivered into the cell by exchange transport, LAT1 is rapidly released outside the cell.

Therefore, there is a demand for the development of a compound that has high LAT1 affinity, has a sustained LAT1 inhibitory effect, and can maintain an effective blood concentration for a long time, or that inhibits the release of a substance having cancer cell accumulability such as L-BPA to the outside of cells by inhibiting the uptake in cancer cells by LAT1.

An object of the present invention is to provide a tetrahydronaphthalene derivative that can be useful for cancer treatment, cancer diagnosis, or the like.

### Means to solve the problem

The present inventors have conducted intensive studies to solve the above problems, and as a result, have found a new tetrahydronaphthalene derivative, thereby completing the present invention.

That is, the present invention provides the following compound.
[1] A compound represented by Formula (I) below or a pharmaceutically acceptable salt thereof: 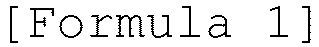 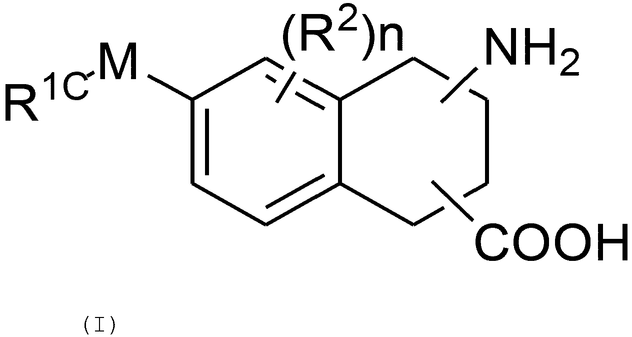
   wherein in Formula (I),
   M represents S, O, or NH;
   R^{1C} represents any structure selected from the group consisting of a 5- to 7-membered aromatic heterocyclic group having a substituent, a C5- to 7-membered aromatic cyclic group having a substituent, and a substituted or unsubstituted 8- to 16-membered polycyclic group,
   where, the substituent of the 5- to 7-membered aromatic heterocyclic group having the substituent or the C5- to 7-membered aromatic cyclic group having the substituent is 1 to 5 groups independently selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted biphenyl, substituted or unsubstituted pyridyl, substituted or unsubstituted naphthyl, halogen, hydroxy, cyano, amino, C1-6 alkylamino, di-(C1-6 alkyl)amino, C1-6 alkyl, C2-6 alkenyl, C2-6 alkynyl, C1-6 alkoxyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, sulfamoyl, ¹⁸F, ¹²³I, boron containing substituents, alpha-emitting nuclides and beta-emitting nuclides
   where, the substituent of the polycyclic group is 1 to 6 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, sulfamoyl, ¹⁸F, ¹²³I, boron containing substituents, alpha-emitting nuclides and beta-emitting nuclides;
      or,
   M is S, O, NH, or absent (simply represents a single bond), R^{1C} represents ¹⁸F, ¹²³I, a substituent including boron, an α radioactive nucleus and/or a β radioactive nucleus;
   R² represents a group independently selected from the group consisting of halogen, hydroxy, cyano, amino, C1-6 alkylamino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl; and
   n represents any integer of 0 to 5.
[2] The compound or pharmaceutically acceptable salt thereof described in [1], wherein the R^{1C} is a substituted or unsubstituted 8- to 16-membered polycyclic group, and the polycyclic group is a ring which may optionally contain N, O and/or S in addition to a C atom.
[3] The compound or pharmaceutically acceptable salt thereof described in [1], wherein
   the R^{1C} is a 5- to 7-membered aromatic group having a substituent, and necessarily contains, as the substituent, at least one group selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted pyridyl, substituted or unsubstituted naphthyl, and substituted or unsubstituted biphenyl, and
   where, when the benzyloxy, benzyl, phenyl, phenyl C1-6 alkyl, pyridyl, naphthyl, or biphenyl is substituted, the benzyloxy, benzyl, phenyl, phenyl C1-6 alkyl, pyridyl, naphthyl, or biphenyl is substituted with 1 to 5 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl.
[4] The compound or pharmaceutically acceptable salt thereof described in [1], wherein
   the R^{1C} is one selected from the group consisting of substituted or unsubstituted pyridylphenyl, substituted or unsubstituted biphenyl, substituted or unsubstituted biphenyl-substituted phenyl, substituted or unsubstituted naphthyl-substituted phenyl, substituted or unsubstituted naphthyl, substituted or unsubstituted quinoline-substituted phenyl, substituted or unsubstituted isoquinoline, substituted or unsubstituted isoquinoline-substituted phenyl, substituted or unsubstituted anthracene, substituted or unsubstituted anthracene-substituted phenyl, substituted or unsubstituted dithiazole ylideneaminonaphthyl, substituted or unsubstituted dithiazole ylideneaminobiphenyl, substituted or unsubstituted benzofuranyl, substituted or unsubstituted benzothiophenyl, substituted or unsubstituted acenaphthene, and substituted or unsubstituted dithiazole ylideneaminophenyl, and
   the substituent in the case of the substitution is 1 to 5 groups selected from the group consisting of halogen, hydroxy, cyano, amino, C1-6 alkylamino, C1-6 alkyl, C2-6 alkenyl, C2-6 alkynyl, C1-6 alkoxy, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino), morpholinocarbonyl, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, C3-8 cycloalkyl, 3- to 8-membered non-aromatic heterocycle, C3-8 cycloalkylamino, 3- to 8-membered non-aromatic heterocycle-substituted amino, dialkylamino, and sulfamoyl.
[5] The compound or pharmaceutically acceptable salt thereof described in [1], wherein in Formula (I),
   M represents O,
   R^{1C} is a 5- to 7-membered aromatic group having a substituent, and necessarily contains, as the substituent, at least one group selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted pyridyl, substituted or unsubstituted naphthyl, and substituted or unsubstituted biphenyl,
   the substituent in the case of substitution is 1 to 5 groups selected from the group consisting of halogen, hydroxy, cyano, amino, C1-6 alkylamino, C1-6 alkyl, C2-6 alkenyl, C2-6 alkynyl, C1-6 alkoxy, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino), morpholinocarbonyl, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, C3-8 cycloalkyl, 3- to 8-membered non-aromatic heterocycle, C3-8 cycloalkylamino, 3- to 8-membered non-aromatic heterocycle-substituted amino, dialkylamino, and sulfamoyl, and
   n represents 0.
[6] An L-type amino acid transporter LAT1 selective inhibitor containing the compound or pharmaceutically acceptable salt thereof described in any one of [1] to [5].
[7] A pharmaceutical composition for cancer treatment, containing the compound or pharmaceutically acceptable salt thereof described in any one of [1] to [5].
[8] A composition for retaining L-BPA in a cell, containing the compound or pharmaceutically acceptable salt thereof described in any one of [1] to [5].
[9] A composition for cancer treatment, containing a compound represented by Formula (II) below or a pharmaceutically acceptable salt thereof: 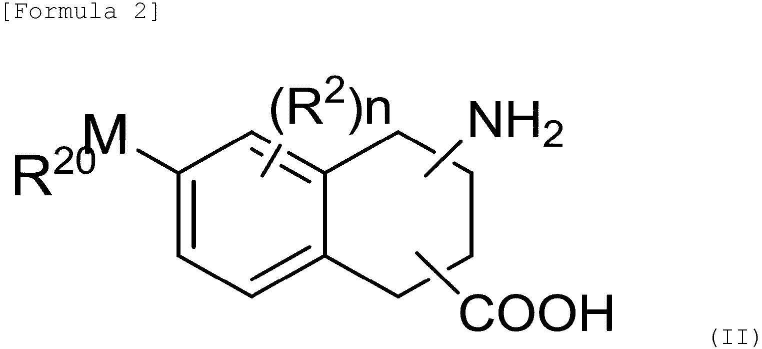
   wherein, in Formula (II),
   M represents S, O, NH, or absent (single bond); and
   R²⁰ represents a radioisotope.
[10] The composition for cancer treatment described in [9], wherein the R²⁰ represents an α radioactive nucleus and/or a β radioactive nucleus.
[11] The composition for cancer treatment described in [10], wherein the α radioactive nucleus is ²¹¹At, and the β radioactive nucleus is ¹³¹I.
[12] A drug for BNCT, containing a compound represented by Formula (III) below or a pharmaceutically acceptable salt thereof: 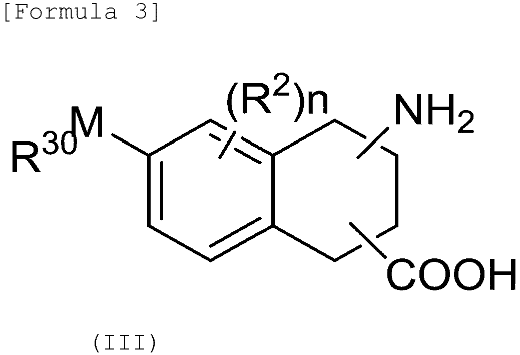
   wherein, in Formula (III),
   M represents S, O, NH, or absent (single bond); and
   R³⁰ represents a substituent including boron.
[13] The drug for BNCT described in [12], wherein the R³⁰ represents boronic acid (-B(OH)₂), a boronic acid ester, a boronic acid amide, or a boron cluster.
[14] A cancer diagnostic agent, containing a compound represented by Formula (IV) below or a pharmaceutically acceptable salt thereof: 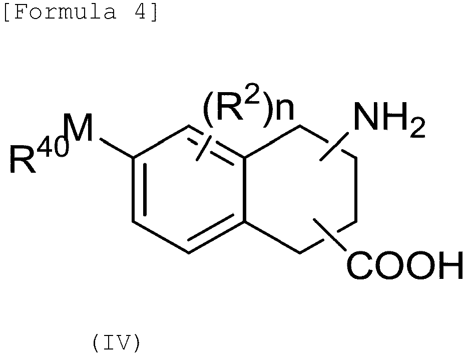
   wherein, in Formula (IV),
   M represents S, O, NH, or absent (single bond); and
   R⁴⁰ represents a radioisotope.
[15] The cancer diagnostic agent described in [14], wherein the R⁴⁰ is ¹⁸F or ¹²³I.
[16] The cancer diagnostic agent described in [14] or [15], wherein the cancer diagnostic agent is a probe for PET or a probe for SPECT.
[17] A sustained inhibitor of LAT1, containing the compound or pharmaceutically acceptable salt thereof described in any one of [1] to [5].
[18] A composition for enhancing a BNCT effect, containing the compound or pharmaceutically acceptable salt thereof described in any one of [1] to [5].

The novel compound of the present invention can be used singly or in combination with another compound as a drug for cancer treatment. The compound of the present invention can be useful for a method of treating or diagnosing a tumor including BNCT or nuclear medicine treatment.

### BRIEF DESCRIPTION OF THE DRAWINGS

Fig. 1A is a graph showing the results of a test for suppression of [¹⁴C]L-leucine uptake using a human-LAT1 stably expressing cell line for a compound of Example and BCH (2-aminobicyclo-(2,2,1)-heptane-2-carboxylic acid);
Fig. 1B is a graph showing the results of a test for suppression of [¹⁴C]L-alanine uptake using a human-LAT2 stably expressing cell line for the compound of Example and BCH;
Fig. 2 is a graph showing the uptake amount of [¹⁴C]L-leucine after 30 minutes from when the compound of Example or BCH was caused to act on a human-LAT1 highly expressing cell line and removed, which represents the suppression action remaining after removal of the compound;
Fig. 3A shows the results of examination of a BNCT-enhancing effect of a compound of Example evaluated using cultured cells; and
Fig. 3B shows the results of examination of the BNCT-enhancing effect of a compound of Example evaluated using a mouse allograft tumor model.

### DETAILED DESCRIPTION OF THE PREFERRED EMBODIMENTS

The present invention relates to a new tetrahydronaphthalene derivative.

In the present specification, when a compound having an asymmetric carbon is represented, the compound includes any of a racemic form, an R form, and an S form unless otherwise specified.

### [Tetrahydronaphthalene derivative]

A tetrahydronaphthalene derivative of the present invention is a compound represented by Formula (I) below or a pharmaceutically acceptable salt thereof. 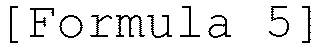 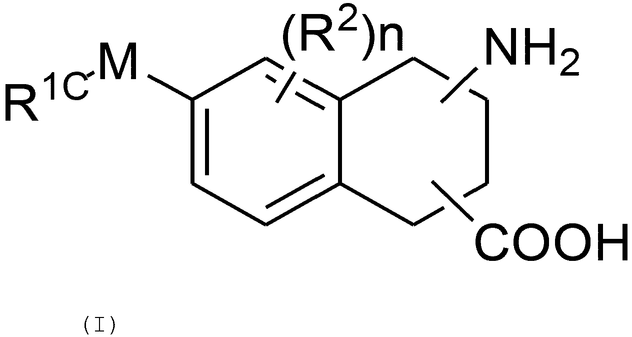
wherein in Formula (I),
M represents S, O, or NH,
R^{1C} represents any structure selected from the group consisting of a 5- to 7-membered aromatic heterocyclic group having a substituent, a C5- to 7-membered aromatic cyclic group having a substituent, and a substituted or unsubstituted 8- to 16-membered polycyclic group,
where, the substituent in the 5- to 7-membered aromatic heterocyclic group having the substituent or the C5- to 7-membered aromatic cyclic group having the substituent is 1 to 4 groups independently selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted biphenyl, substituted or unsubstituted pyridyl, substituted or unsubstituted naphthyl, halogen, hydroxy, cyano, amino, C1-6 alkylamino, di-(C1-6 alkyl)amino, C1-6 alkyl, C2-6 alkenyl, C2-6 alkynyl, C1-6 alkoxyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl,
where, the substituent of the polycyclic group is 1 to 6 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl;
   or,
M is S, O, NH, or absent (simply represents a single bond), R^{1C} represents ¹⁸F, ¹²³I, a substituent including boron, an α radioactive nucleus and/or a β radioactive nucleus;
R² represents a group independently selected from the group consisting of halogen, hydroxy, cyano, amino, C1-6 alkylamino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl; and
n represents any integer of 0 to 5.

In the present specification, the structure selected from the group consisting of a 5- to 7-membered aromatic heterocyclic group having a substituent, a C5- to 7-membered aromatic cyclic group having a substituent, and a substituted or unsubstituted 8- to 16-membered polycyclic group refers to the following structure.

In the present specification, examples of the C5- to 7-membered aromatic cyclic group having a substituent include a phenyl group.

In the present specification, a 5- to 7-membered aromatic heterocycle in the 5- to 7-membered aromatic heterocyclic group having a substituent represents a 5- to 7-membered aromatic ring containing one or more heteroatoms selected from O, N, and S. Examples of the group formed by a 5- to 7-membered aromatic heterocycle include, but are not limited to, thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, and triazinyl.

In the present specification, the substituted or unsubstituted 8- to 16-membered polycyclic group refers to a group formed by a substituted or unsubstituted 8- to 16-membered polycyclic compound, and examples thereof include naphthyl, quinolyl, isoquinolyl, benzothiophenyl, benzofuranyl, benzimidazolyl, benzoxazolyl, benzodioxolyl, benzisoxazolyl, benzothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, phthalazinyl, naphthyridinyl, quinoxalinyl, quinazolinyl, cinnolinyl, carbazolyl, β-carborinyl, phenanthridinyl, acridinyl, phenazinyl, phenothiazinyl, phenoxazinyl, and tetrahydronaphthyl, as well as groups formed by acenaphthene, anthracene, phenanthrene, pyrene, and naphthacene.

In the present specification, the expression "having a substituent" refers to being substituted with any of substituents within a range of 1 to 5 substituents. The substituent described in the "5- to 7-membered aromatic heterocyclic group having the substituent" or the "C5- to 7-membered aromatic cyclic group having the substituent" is 1 to 5 groups independently selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted biphenyl, substituted or unsubstituted pyridyl, substituted or unsubstituted naphthyl, halogen, hydroxy, cyano, amino, C1-6 alkylamino, di-(C1-6 alkyl)amino, C1-6 alkyl, C2-6 alkenyl, C2-6 alkynyl, C1-6 alkoxyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl, and
where the substituent of the polycyclic group may be 1 to 5 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl.

In the present specification, the term "substituted or unsubstituted" means that it may be optionally substituted with any of substituents. Examples of the substituent in the substituted or unsubstituted benzyloxy, the substituted or unsubstituted benzyl, the substituted or unsubstituted phenyl, the substituted or unsubstituted phenyl C1-6 alkyl, the substituted or unsubstituted biphenyl, the substituted or unsubstituted pyridyl, or the substituted or unsubstituted naphthyl include, but are not limited to, groups independently selected from the group consisting of halogen, hydroxy, cyano, amino, C1-6 alkylamino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl.

In the present specification, halogen may be any of F, Cl, Br, and I, and particularly preferably is F, Cl, or Br.

In the present specification, the C1-6 alkylamino refers to an amino having a linear or branched C1-6 saturated hydrocarbon group. Examples thereof include methylamino, ethylamino, propyl (for example, n-propyl and isopropyl) amino, butyl (for example, n-butyl, isobutyl, and t-butyl) amino, and pentyl (for example, n-pentyl, isopentyl, and neopentyl) amino. Preferably, the C1-6 alkylamino is a linear or branched C1-C4 alkylamino group, and examples thereof include, but are not limited to, a methylamino group, an ethylamino group, an isopropylamino group, and a butylamino group.

In the present specification, the di-(C1-6 alkyl)amino refers to an amino having two linear or branched C1-6 saturated hydrocarbon groups. Examples of the di-(C1-6 alkyl)amino group include dimethylamino, diethylamino, methylethylamino, dipropyl (for example, n-propyl and isopropyl) amino, dibutyl (for example, n-butyl, isobutyl, and t-butyl) amino, and dipentyl (for example, n-pentyl, isopentyl, and neopentyl) amino. Preferably, the di-(C1-6 alkyl)amino is an amino group having two linear or branched C1-C4 alkyl groups, and examples thereof include, but are not limited to, a dimethylamino group, a diethylamino group, a methylethylamino group, a methylisopropylamino group, and a methylbutylamino group.

In the present specification, the C1-6 alkyl refers to a linear or branched C1-6 saturated hydrocarbon group. Examples of the C1-6 alkyl group include methyl, ethyl, propyl (for example, n-propyl and isopropyl), butyl (for example, n-butyl, isobutyl, and t-butyl), and pentyl (for example, n-pentyl, isopentyl, and neopentyl). Preferably, the linear or branched C1-C4 alkyl group, and examples thereof include, but are not limited to, a methyl group, an ethyl group, an isopropyl group, and a butyl group.

In the present specification, the C2-6 alkenyl refers to a group including a double bond among linear or branched C2-C6 unsaturated hydrocarbon groups. Examples of the C2-6 alkenyl group include ethenyl, propenyl, and butenyl.

In the present specification, the C2-6 alkynyl refers to a group including a triple bond among linear or branched C2-C6 unsaturated hydrocarbon groups. Examples of the C2-6 alkynyl group include acetylene, methylacetylene, butyne, and pentyne.

In the present specification, the C1-6 haloalkyl refers to a C1-6 alkyl group having one or more halogen substituents. The C1-6 haloalkyl group is preferably represented by C₂X₅, CH₂X, CHX₂, or CX₃ (X represents Cl, F, Br, or I), and examples thereof include, but are not limited to, CF₃, C₂F₅, CHF₂, CH₂F, CC1₃, CHCl₂, and C₂Cl₅. Among them, trifluoromethyl, difluoromethyl, or monofluoromethyl is particularly preferable.

In the present specification, the C1-6 alkoxyl refers to a linear or branched C1-6 alkyl group and a group having an oxygen molecule. The C1-6 alkoxy or alkoxyl preferably has linear or branched C1-C4 alkyl, and examples thereof include, but are not limited to, groups such as methoxy, ethoxy, isopropoxy, and butoxy.

In the present specification, examples of C1-6 alkoxy C1-6 alkyl include, but are not limited to, methoxyethyl and ethoxyethyl.

In the present specification, the C1-6 alkylamino or di- (C1-6 alkyl) amino means a group represented as NR⁴¹R¹², wherein R¹¹R¹² each independently represents H or C1-6 alkyl (provided that, a case where both R¹¹ and R¹² are H is excluded). Examples thereof include, but are not limited to, methylamino, dimethylamino, ethylamino, and i-propylamino.

In the present specification, the C1-6 alkylaminocarbonyl or di-(C1-6 alkyl)aminocarbonyl means a group represented as CONR¹³R¹⁴, wherein R¹³ and R¹⁴ each independently represent H or C1-6 alkyl (provided that, a case where both R¹³ and R¹⁴ are H is excluded) . Examples thereof include, but are not limited to, methylaminocarbonyl, dimethylaminocarbonyl, ethylaminocarbonyl, and i-propylaminocarbonyl.

In the present specification, examples of the C1-6 alkylcarbonyl include, but are not limited to, methylcarbonyl and ethylcarbonyl.

In the present specification, examples of the C1-6 alkoxycarbonyl include, but are not limited to, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

In the present specification, examples of COOR¹⁰ (R¹⁰ is H or C1-6 alkyl, amino, or alkylamino (NR¹¹R¹² (R¹¹R¹² each independently represents H or C1-6 alkyl)) include, but are not limited to, methyloxycarbonyl and aminooxycarbonyl.

In the present specification, examples of each of the C1-6 haloalkylsulfanyl, the C1-6 haloalkylsulfinyl, and the C1-6 haloalkylsulfonyl include, but are not limited to, trifluoromethylsulfanyl, a trifluoromethylsulfinyl group, and trifluoromethylsulfonyl.

In the present specification, examples of the C1-6 alkylthio include, but are not limited to, methylthio, ethylthio, propylthio, isopropylthio, butylthio, sec-butylthio, tert-butylthio, pentylthio, and hexylthio.

In the present specification, examples of the C1-6 alkylsulfinyl include, but are not limited to, methylsulfinyl and ethylsulfinyl.

In the present specification, examples of the C1-6 alkylsulfonyl include, but are not limited to, methylsulfonyl and ethylsulfonyl.

In the present specification, examples of the substituted C1-6 alkyl include, but are not limited to, linear or branched C1-6 alkyl in which one to three hydrogen atoms are substituted with other groups.

In the present specification, examples of the substituted C1-6 alkoxy include, but are not limited to, linear or branched C1-6 alkoxy in which one or two hydrogen atoms are substituted with other groups.

In the present specification, examples of the substituted C1-6 alkoxy C1-6 alkyl include, but are not limited to, linear or branched C1-6 alkoxy C1-6 alkyl in which one or two hydrogen atoms are substituted with other groups.

In the present specification, the C3-8 cycloalkyl refers to, for example, cyclopropyl, cyclobutyl, cyclopentyl, cyclohexyl, cycloheptyl, or cyclooctyl.

In the present specification, a 3- to 8-membered non-aromatic heterocycle is, for example, a saturated or unsaturated ring of a 3- to 8-membered ring containing one or two heteroatoms selected from an oxygen atom, a nitrogen atom, and a sulfur atom in the ring, and means a ring that is not an aromatic ring. Examples thereof include aziridino, azetidino, morpholino, thiomorpholino, 1-pyrrolidinyl, piperidino, 4-piperidinyl, 1-piperazinyl, 1-pyrrolyl, oxazolidino, thiazolidino, tetrahydrofuranyl, and tetrahydropyranyl.

In the present specification, the C3-8 cycloalkylamino refers to, for example, cyclopropylamino, cyclobutylamino, cyclopentylamino, cyclohexylamino, cycloheptylamino, or cyclooctylamino.

In the present specification, examples of the 3- to 8-membered non-aromatic heterocycle-substituted amino include aziridinoamino, azetidinoamino, morpholinoamino, thiomorpholinoamino, 1-pyrrolidinylamino, piperidinoamino, 4-piperidinylamino, 1-piperazinylamino, 1-pyrrolylamino, oxazolidinoamino, and thiazolidinoamino.

In the present specification, examples of the substituent including boron include boronic acid (-B(OH)₂), a boronic acid ester, a boronic acid amide, and a boron cluster. The boron cluster includes, but is not limited to, BSH (sodium borocaptate), dodecaborate, decaborate, or carborane.

In the present specification, examples of the α radioactive nucleus and/or the β radioactive nucleus preferably include, but are not limited to, ²¹¹At as the α radioactive nucleus, and ¹³¹I as the β radioactive nucleus.

In the compound, Formula (I) is not limited, but preferably is 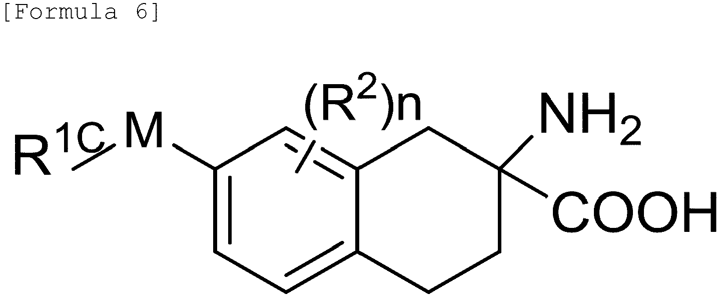 or

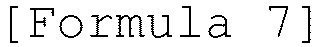

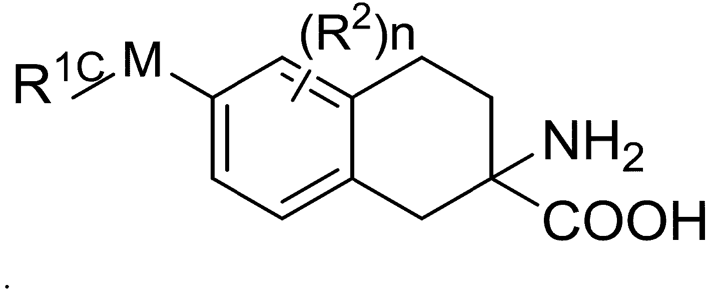

In the compound, the R^{1C} is, but is not limited to, one selected from the group consisting of substituted or unsubstituted pyridylphenyl, substituted or unsubstituted biphenyl, substituted or unsubstituted phenylpyridyl, substituted or unsubstituted biphenyl-substituted phenyl, substituted or unsubstituted pyridyl, substituted or unsubstituted naphthyl-substituted phenyl, substituted or unsubstituted naphthyl-substituted pyridyl, substituted or unsubstituted naphthyl, substituted or unsubstituted benzofuranyl, substituted or unsubstituted benzothiophenyl, substituted or unsubstituted acenaphthene, substituted or unsubstituted quinoline, substituted or unsubstituted quinoline-substituted phenyl, substituted or unsubstituted isoquinoline, substituted or unsubstituted isoquinoline-substituted phenyl, substituted or unsubstituted anthracene, substituted or unsubstituted anthracene-substituted phenyl, substituted or unsubstituted dithiazole ylideneaminonaphthyl, substituted or unsubstituted dithiazole ylideneaminobiphenyl, substituted or unsubstituted dithiazole ylideneaminophenyl, substituted or unsubstituted phenanthrene, substituted or unsubstituted pyrene, substituted or unsubstituted naphthacene, substituted or unsubstituted benzofuran, substituted or unsubstituted benzothiophene, substituted or unsubstituted tetrahydronaphthyl and substituted or unsubstituted indole, substituted or unsubstituted phenyloxazole, and substituted or unsubstituted phenyloxazole.

The substituent in the case of substitution is preferably a group selected from the group consisting of halogen, hydroxy, cyano, amino, C1-6 alkylamino, C1-6 alkyl, C2-6 alkenyl, C2-6 alkynyl, C1-6 alkoxy, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, or C1-6 alkylamino), morpholinocarbonyl, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, C3-8 cycloalkyl, 3- to 8-membered non-aromatic heterocycle, C3-8 cycloalkylamino, 3- to 8-membered non-aromatic heterocyclic amino, dialkylamino, and sulfamoyl, and is more preferably a group selected from the group consisting of halogen, hydroxy, cyano, amino, C1-6 alkylamino, C1-6 alkyl, C2-6 alkenyl, C2-6 alkynyl, C1-6 alkoxy, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, or C1-6 alkylamino), and morpholinocarbonyl.

In the compound, the M is preferably, but is not limited to, an O atom.

In the compound, it is preferable that, although not limited, n is 0 or n is 1 or 2 and the R² is a group selected from the group consisting of halogen, hydroxy, cyano, amino, C1-6 alkylamino, C1-6 alkyl, C2-6 alkenyl, C2-6 alkynyl, C1-6 alkoxy, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, or C1-6 alkylamino), and morpholinocarbonyl.

In the compound, it is more preferable that, although not limited, the M is an O atom, the R^{1C} is one selected from the group consisting of substituted or unsubstituted pyridylphenyl, substituted or unsubstituted biphenyl, substituted or unsubstituted phenylpyridyl, substituted or unsubstituted biphenyl-substituted phenyl, substituted or unsubstituted naphthyl-substituted phenyl, and substituted or unsubstituted naphthyl, and n is 0.

The substituent in the case of substitution is more preferably 1 to 4 groups independently selected from the group consisting of halogen, amino, C1-4 alkylamino, di-(C1-4 alkyl)amino, C1-4 alkyl, C2-4 alkenyl, C2-4 alkynyl, C1-4 alkoxy, benzyloxy, benzyl, phenyl, phenyl C1-4 alkyl, C1-4 alkoxy C1-4 alkyl, nitro, C1-4 haloalkyl, carbamoyl, C1-4 alkylaminocarbonyl, di-(C1-4 alkyl)aminocarbonyl, C1-4 alkoxycarbonyl, C1-4 alkylcarbonyl, COOR¹⁰ (R¹⁰ is H or C1-4 alkyl, amino, or C1-4 alkylamino), and morpholinocarbonyl.

Among the tetrahydronaphthalene derivatives of the present invention, particularly preferred compounds include any of the following compounds or salts thereof:
2-amino-7-(naphthalene-2-yloxy)-1,2,3,4-tetrahydronaphthalene-2-carboxylic acid;
2-amino-7-(naphthalene-1-yloxy)-1,2,3,4-tetrahydronaphthalene-2-carboxylic acid;
2-amino-7-((3'-fluoro-[1,1'-biphenyl]-2-yl)oxy)-1,2,3,4-tetrahydronaphthalene-2-carboxylic acid;
2-amino-7-((3'-chloro-[1,1'-biphenyl]-2-yl)oxy)-1,2,3,4-tetrahydronaphthalene-2-carboxylic acid;
2-amino-7-(2-(naphthalene-1-yl)phenoxy)-1,2,3,4-tetrahydronaphthalene-2-carboxylic acid;
2-amino-7-((3'-nitro-[1,1'-biphenyl]-2-yl)oxy)-1,2,3,4-tetrahydronaphthalene-2-carboxylic acid;
2-amino-7-((3'-methyl-[1,1'-biphenyl]-2-yl)oxy)-1,2,3,4-tetrahydronaphthalene-2-carboxylic acid;
2-amino-7-((2',3'-difluoro-[1,1'-biphenyl]-2-yl)oxy)-1,2,3,4-tetrahydronaphthalene-2-carboxylic acid;
2-amino-7-((8-methylnaphthalene-1-yl)oxy)-1,2,3,4-tetrahydronaphthalene-2-carboxylic acid;
2-amino-7-((3'-chloro-4'-fluoro-[1,1'-biphenyl]-2-yl)oxy)-1,2,3,4-tetrahydronaphthalene-2-carboxylic acid;
2-amino-7-((2'-chloro-3'-fluoro-[1,1'-biphenyl]-2-yl)oxy)-1,2,3,4-tetrahydronaphthalene-2-carboxylic acid;
2-amino-7-((3'-(dimethylamino)-[1,1'-biphenyl]-2-yl)oxy)-1,2,3,4-tetrahydronaphthalene-2-carboxylic acid;
2-amino-7-((3'-(methylthio)-[1,1'-biphenyl]-2-yl)oxy)-1,2,3,4-tetrahydronaphthalene-2-carboxylic acid;
2-amino-7-((2',3'-dichloro-[1,1'-biphenyl]-2-yl)oxy)-1,2,3,4-tetrahydronaphthalene-2-carboxylic acid;
2-amino-7-((3'-chloro-2'-fluoro-[1,1'-biphenyl]-2-yl)oxy)-1,2,3,4-tetrahydronaphthalene-2-carboxylic acid;
2-amino-7-((2'-fluoro-3'-methyl-[1,1'-biphenyl]-2-yl)oxy)-1,2,3,4-tetrahydronaphthalene-2-carboxylic acid;
2-amino-7-((2'-chloro-3'-methyl-[1,1'-biphenyl]-2-yl)oxy)-1,2,3,4-tetrahydronaphthalene-2-carboxylic acid;
2-amino-7-((2',3',4'-trifluoro-[1,1'-biphenyl]-2-yl)oxy)-1,2,3,4-tetrahydronaphthalene-2-carboxylic acid;
2-amino-7-((2',3',5'-trifluoro-[1,1'-biphenyl]-2-yl)oxy)-1,2,3,4-tetrahydronaphthalene-2-carboxylic acid; or
2-amino-7-(2-(naphthalen-1-yl)-4-nitrophenoxy)-1,2,3,4-tetrahydronaphthalene-2-carboxylic acid.

Examples of the "pharmaceutically acceptable salt" in the present invention include a salt with an inorganic base, a salt with an organic base, a salt with an inorganic acid, a salt with an organic acid, and a salt with a basic or acidic amino acid. Suitable examples of the salt with an inorganic base include alkali metal salts such as a sodium salt and a potassium salt; alkaline earth metal salts such as a calcium salt and a magnesium salt; an aluminum salt, and an ammonium salt. Suitable examples of the salt with an organic base include salts with trimethylamine, triethylamine, pyridine, picoline, ethanolamine, diethanolamine, triethanolamine, dicyclohexylamine, N,N'-dibenzylethylenediamine, and the like. Suitable examples of the salt with an inorganic acid include salts with hydrochloric acid, hydrobromic acid, nitric acid, sulfuric acid, phosphoric acid, and the like. Suitable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. Suitable examples of the salt with a basic amino acid include salts with arginine, lysine, ornithine, and the like, and suitable examples of the salt with an acidic amino acid include aspartic acid, glutamic acid, and the like.

In recent years, the structure of LAT1 has been clarified by cryoelectron microscopic analysis, and understanding of the mechanism of dynamic interaction of a site binding to a ligand has been advanced. Among the compounds of the present invention, an LAT1 inhibitory drug is created with the intention of binding to a distal pocket (see Non-Patent Document 2), which is a binding pocket of LAT1 revealed by cryoelectron microscopic analysis. No existing compound is known to bind to this distal pocket.

That is, it is expected that the compound of the present invention has an amino group and a carboxyl group fixed as substituents of a cyclic tetrahydronaphthalene derivative, and has LAT1 selectivity as a common property. Since the linkage between tetrahydronaphthalene and the ring structure (for example, an aromatic ring) at the terminal on the R^{1c} side is -M-, and the ring structure such as a phenyl group or a polycyclic group is directly bonded to -M-, it is considered that bending is reduced as the shape of the entire compound, and it is considered that the compound easily enters a site called a distal pocket in the LAT-1.

The compound of the present invention binds strongly to LAT1 by being well adapted to such a binding pocket, and the effect of continuously suppressing LAT1 can be exhibited even after the end of compound exposure.

Therefore, in the LAT1-expressing cells exposed to the compound of the present invention, inflow and outflow of amino acids from LAT1 can be suppressed continuously even after the end of exposure.

It is expected that the compound of the present invention has an ability to inhibit the uptake of amino acids into cancer cells via LAT1, thereby exhibiting anticancer action, has high LAT1 affinity and high LAT1 selectivity, maintains the LAT1 inhibitory effect, and effectively maintains the blood concentration.

In one aspect of the compound of the present invention, the high affinity and high selectivity for LAT1 and the sustained LAT1 inhibitory effect can inhibit the uptake of substances into cancer cells by LAT1. For example, enhancement of the BNCT effect can be achieved by blocking the extracellular release of substances useful for BNCT, such as L-BPA.

In another aspect of the compound of the present invention, when the compound contains an α radioactive nucleus or a β radioactive nucleus, the compound can act as a compound that is efficiently and selectively transported and can emit radiation by having high affinity and high selectivity for LAT1.

In another aspect of the compound of the present invention, when the compound contains a boron atom, the compound can function as a boron-containing compound for use in boron delivery to cancer cells in BNCT.

Alternatively, in still another aspect of the compound of the present invention, the compound can also be a radioactive compound used for detection and diagnosis of cancer by PET or SPECT.

### [Method for producing tetrahydronaphthalene derivative]

In the present invention, a method for producing a novel tetrahydronaphthalene derivative is not limited, but may be, for example, the following method using a particularly preferable compound as a representative example.

First, for example, a hydantoin compound of General Formula (III) below is prepared from a tetralone type compound represented by General Formula (II) below, and then an amino acid (Formula (IV)) is prepared. The compound of the present invention can be prepared by protecting the amino group and the carboxyl group of the obtained amino acid and modifying or substituting the group that binds to M.

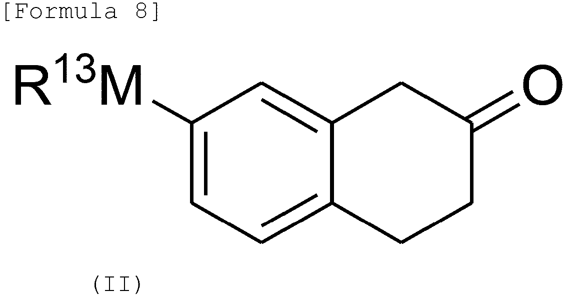

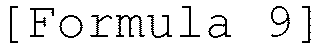

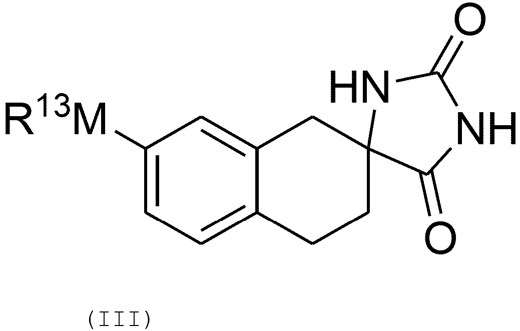

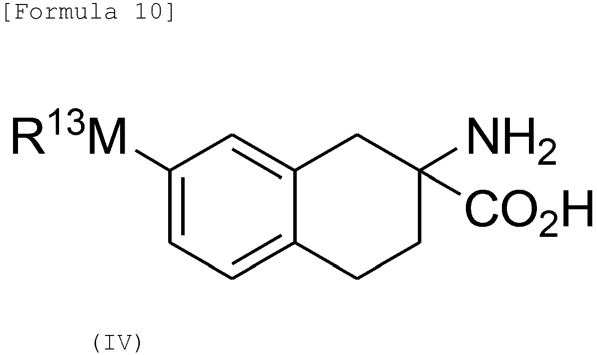

In the formula, MR¹³ represents, for example, OH.

The tetralone type compound of Formula (II) is converted into the hydantoin of Formula (III) by treatment with ammonium carbonate and potassium cyanide. This reaction is performed, for example, in a hydrous ethanol mixture at a temperature between 50°C and 90°C, preferably between 80°C and 90°C. The direct hydrolysis of this hydantoin to an amino acid (Formula (IV)) requires treatment with a strong base, for example, a sodium hydroxide solution or barium hydroxide, at a reflux temperature. The reaction time at this time is preferably a long time such as 10 to 30 hours. In this way, the hydantoin compound is converted into an amino acid.

The amino group and the carboxyl group of the obtained compound are protected by a conventional method. A protecting group is not limited, and for example, a carbamate-based protecting group, an amide-based protecting group, or an alkyl protecting group is preferably used. Examples of such a carbamate-based protecting group include a tert-butoxycarbonyl group (Boc), a benzyloxycarbonyl group, a 9-fluorenylmethyloxycarbonyl group (Fmoc), and a 2,2,2-trichloroethoxycarbonyl group (Troc), examples of the amide-based protecting group include an acetyl group and a benzoyl group, and examples of the alkyl protecting group include a methyl group, a t-butyl group, and a benzyl group.

A group that binds to M can be appropriately reacted in a protected state.

For example, when M in R¹³M is O, an ether form can be obtained by reacting the phenol form obtained above with a halogenated ring structure in the presence of a base.

Examples of the base to be used at this time include potassium carbonate, tripotassium phosphate, cesium carbonate, triethylamine, N,N-diisopropylethylamine, pyridine, sodium ethoxide, potassium tert-butoxide, sodium hydride, lithium hexamethyldisilazide, sodium hexamethyldisilazide, and n-butyllithium.

Examples of the catalyst to be used at this time include palladium catalysts such as palladium(II) acetate, palladium(II) chloride, tris(dibenzylideneacetone)dipalladium(0), [1,1'-bis(diphenylphosphino)ferrocene]palladium(II) dichloride dichloromethane adduct, and tetrakis(triphenylphosphine)palladium(0), and catalysts such as copper iodide, copper bromide, and copper oxide. Examples of a ligand to be used at this time include triphenylphosphine, tricyclohexylphosphine, 1,1'-bis(diphenylphosphino)ferrocene, 2-dicyclohexylphosphino-2',6'-dimethoxybiphenyl, 2-dicyclohexylphosphino-2',4',6'-triisopropylbiphenyl, and 2-dicyclohexylphosphino-2'-(N,N-dimethylamino)biphenyl.

Next, the obtained compound is sequentially deprotected or directly subjected to the next step. Deprotection follows a conventional method, but can be performed by, for example, hydrolysis, catalytic hydrogenation, decarboxylation, or oxidation.

When M is S in the compound represented by Chemical Formula (I), it is also preferable to employ the following production method.

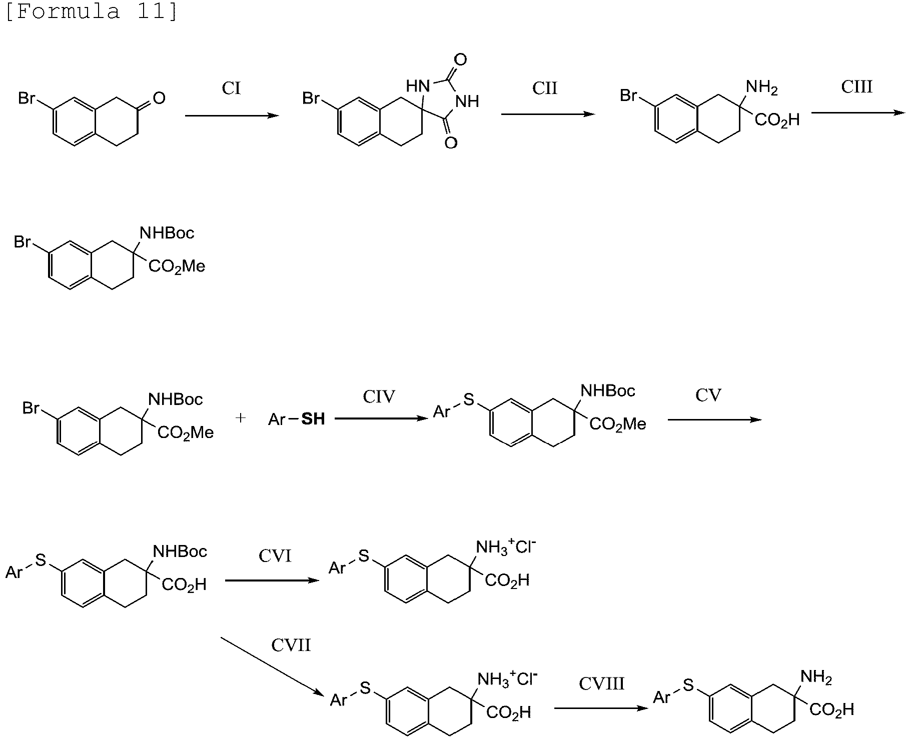

When M is NH in the compound represented by Chemical Formula (I), it is also preferable to employ the following production method.

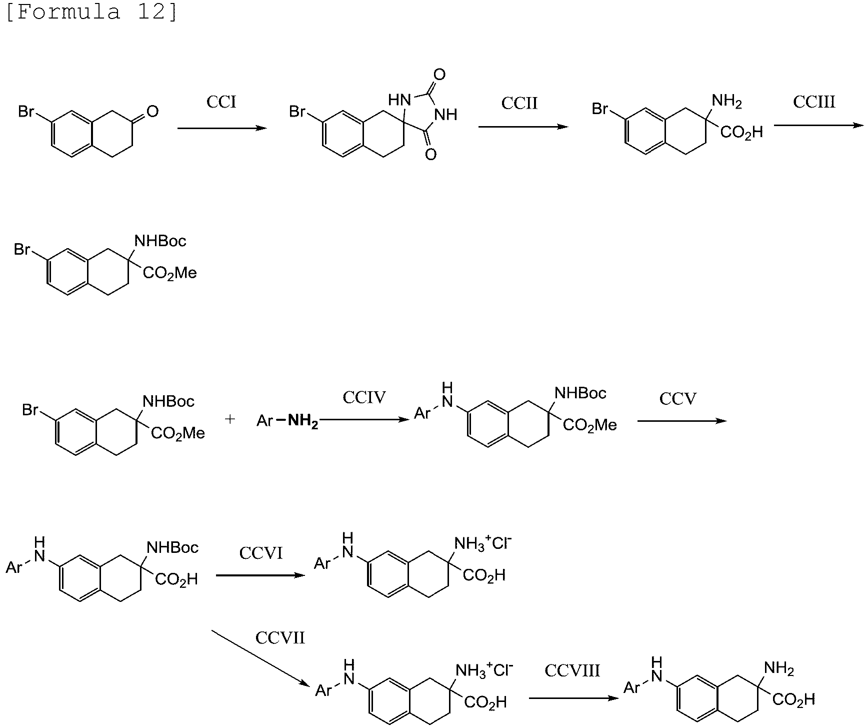

Among the above chemical reactions, the steps of CI and CCI are reactions in which a tetralone type compound is converted into hydantoin by treatment with ammonium carbonate and potassium cyanide. This reaction is performed, for example, in a hydrous ethanol mixture at a temperature between 50°C and 90°C, preferably between 80°C and 90°C. The direct hydrolysis of this hydantoin to an amino acid requires treatment with a strong base, for example, a sodium hydroxide solution or barium hydroxide, at a reflux temperature. The reaction time at this time is preferably a long time such as 10 to 30 hours. Next, such a hydantoin compound is converted into an amino acid.

The amino group and the carboxyl group of the obtained compound are protected by a conventional method.

Next, the reaction represented by CIV is a step of reacting the halogenated ring structure in the presence of a base to obtain a thioether form. Examples of the base to be used at this time include potassium carbonate, tripotassium phosphate, cesium carbonate, triethylamine, N,N-diisopropylethylamine, pyridine, sodium ethoxide, sodium tert-butoxide, potassium tert-butoxide, sodium hydride, lithium hexamethyldisilazide, sodium hexamethyldisilazide, n-butyllithium, zinc chloride, zinc bromide, zinc iodide, and lithium iodide. Examples of the catalyst to be used at this time include palladium such as di-µ-bromobis(tri-t-butylphosphine)dipalladium(I), nickel such as nickel acetate and nickel chloride, copper catalysts such as copper chloride, copper bromide, and copper iodide, and examples of the ligand include heterocyclic carbenes such as 1,3-bis(2,6-diisopropylphenyl)imidazolium chloride, and triphenylphosphine.

Although not limited to the following, the compound can be synthesized by any method at a reaction temperature of 90°C and a reaction time of about 2 hours in a THF solvent with a di-µ-bromobis(tri-t-butylphosphine)dipalladium(I) catalyst in the presence of a base such as sodium t-butoxide, zinc chloride, or lithium iodide, or at a reaction temperature of 70°C and a reaction time of about 18 hours in a DMF solvent in the presence of potassium t-butoxide, a nickel acetate catalyst or 1,3-bis(2,6-diisopropylphenyl)imidazolium chloride as a ligand.

The reaction represented by CCIV is a step of reacting the halogenated ring structure in the presence of a base to obtain an amino form. Examples of the base to be used at this time include potassium carbonate, tripotassium phosphate, cesium carbonate, triethylamine, N,N-diisopropylethylamine, pyridine, sodium ethoxide, sodium tert-butoxide, potassium tert-butoxide, sodium hydride, lithium hexamethyldisilazide, sodium hexamethyldisilazide, and n-butyllithium. Examples of the catalyst to be used at this time include palladium catalysts such as palladium acetate, and examples of the ligand include bicyclic triaminophosphines such as 2,8,9-triisobutyl-2,5,8,9-tetraaza-1-phosphabicyclo[3,3,3]undecane.

Although not limited to the following, the compound can be synthesized at a reaction temperature of 80°C and a reaction time of about 18 hours in a toluene solvent in the presence of sodium t-butoxide or in the presence of a palladium acetate catalyst and 2,8,9-triisobutyl-2,5,8,9-tetraaza-1-phosphabicyclo[3,3,3]undecane as a ligand.

In each step of the production method, purification is performed according to a conventional method, and can be appropriately modified.

In particular, when the compound is a racemic form, the compound can be used as it is, or for example, the optical purity of the R form or the S form can also be increased in order to obtain a preferred compound for use in boron neutron capture therapy.

As the optical resolution, a known method may be appropriately used, and for example, in addition to a method in which the optical resolution (using α-chymotrypsin or the like) is performed through a hydrolysis step and an esterification step, a simplified method including a simplified step in which an acylase is used through a hydrolysis step may be adopted.

### [L-type amino acid transporter 1 (LAT1) selective inhibitor]

The tetrahydronaphthalene derivative of the present invention has an affinity for the amino acid transporter LAT1 expressed on the surface of cells. Preferably, the tetrahydronaphthalene derivative of the present invention has no affinity for the amino acid transporter LAT2. LAT1 is a type of amino acid transporters, which is a membrane protein required for intracellular uptake of neutral branched chain amino acids and aromatic amino acids, and it has been revealed that LAT1 is specifically expressed in cancer. LAT1 is responsible for supplying amino acids as nutrients in cancer tissues. The amino acid taken up in the cancer cell by LAT1 not only serves as a nutrient but is also responsible for regulating protein synthesis, intracellular metabolism, and cell growth via an amino acid signal system. In the present specification, an "LAT1 selective inhibitor" means a substance that inhibits intracellular uptake of amino acids via LAT1, and more preferably, a substance that inhibits intracellular influx via LAT1 more than an amino acid transporter (for example, LAT2) in normal cells.

The tetrahydronaphthalene derivative of the present invention may be used as an LAT1 selective inhibitor in the form of the compound or pharmaceutically acceptable salt as it is, or in the form of a formulation known to those skilled in the art by mixing with a pharmaceutically acceptable carrier or in the form of encapsulation in micro/nanoparticles, or the like. In the tetrahydronaphthalene derivative of the present invention, the ratio of affinity to cells expressing LAT1/affinity to cells expressing LAT2 is preferably 2 times or more, more preferably 5 times or more, and further preferably 10 times or more.

### [Pharmaceutical composition for cancer treatment]

The tetrahydronaphthalene derivative of the present invention can be used as an LAT1 inhibitory drug that inhibits the function of LAT1 specifically expressed in cancer cells to induce cancer-specific nutrient starvation and suppresses protein synthesis, intracellular metabolism, and cell growth through suppression of an amino acid signal system to reduce cancer tissues. As a result of the function inhibition, the supply of nutrients to the cells is blocked, and protein synthesis, intracellular metabolism, and cell growth are suppressed through suppression of the amino acid signal system, leading to the shrinkage of tumor cells.

Alternatively, the tetrahydronaphthalene derivative of the present invention may be, for example, a compound having the structure in which a therapeutic radioisotope is introduced as a substituent or a pharmaceutically acceptable salt thereof. The radioisotope is an α radioactive nucleus and/or a β radioactive nucleus. Preferably, the tetrahydronaphthalene derivative of the present invention may be provided as a pharmaceutical composition for cancer treatment containing a compound having the structure in which ²¹¹At of an α radioactive nucleus is introduced as a substituent or a pharmaceutically acceptable salt thereof.

²¹¹At is a radioisotope of astatine (At) that is an element belonging to halogen. The ²¹¹At emits high-energy α rays that have cell killing properties and disintegrates into stable lead (²⁰⁷Pb). The half-life of ²¹¹At is 7.2 hours. That is, ²¹¹At has a short lifespan and high cell killing property, and thus can efficiently destroy a cancer tissue by labeling the ²¹¹At as a substituent and guiding the ²¹¹At to an affected area. By labeling ²¹¹At as a substituent with an LAT1 affinity compound, it is possible to specifically accumulate ²¹¹At in a cancer cell expressing LAT1 and perform α irradiation, and further, it is possible to provide a therapeutic effect that there is little possibility of side effects since LAT1 itself is hardly expressed in a normal cell.

In another aspect, preferably, the tetrahydronaphthalene derivative of the present invention may be provided as a pharmaceutical composition for cancer treatment containing a compound having the structure in which ¹³¹I of a β radioactive nucleus is introduced as a substituent or a pharmaceutically acceptable salt thereof.

When the formulation of the tetrahydronaphthalene derivative of the present invention is used as an LAT1 selective inhibitor, the formulation can be used for the treatment of tumors. The treatment is performed by administration of the formulation in any suitable route of administration in such a way that the tetrahydronaphthalene derivative accumulates in a target tumor. The treatment using the tetrahydronaphthalene derivative is not limited, and the tetrahydronaphthalene derivative can be administered so that the ratio of tumor : blood is at least 1.5 or more : 1, and preferably 2 or more : 1. The tetrahydronaphthalene derivative can be administered at a time or can be continuously administered. In some cases, the tetrahydronaphthalene derivative can also be administered by being divided. The tetrahydronaphthalene derivative can be used singly as an anticancer agent, but can be used in combination with an existing anticancer agent, an existing molecular target drug, an immune checkpoint inhibitory drug including an anti-PD-1 antibody, a selective serotonin reuptake inhibitory drug having an antitumor effect, an mTOR inhibitory drug, another LAT1 inhibitory drug, or the like, or in combination with radiation therapy including particle therapy, surgical therapy, BNCT, and nuclear medicine therapy. If desired, the tetrahydronaphthalene derivative can be utilized for residual tumor or metastatic tumor after the surgical procedure is performed or for the purpose of metastasis suppression.

### [Composition for enhancing BNCT effect]

The tetrahydronaphthalene derivative of the present invention can also enhance the effect of BNCT by preventing a drug for BNCT exhibiting accumulability in cancer cells, such as sodium borocaptate (BSH) or L-boronophenylalanine (L-BPA), from flowing out of cells from cells, and can also be used as a composition for enhancing the BNCT effect.

That is, when the tetrahydronaphthalene derivative of the present invention is administered together with a substance such as sodium borocaptate (BSH) or L-boronophenylalanine (L-BPA) simultaneously or continuously within a certain period of time, it is possible to prevent the drug from flowing out from cells, and thus the efficacy of BNCT can be enhanced. For example, even when a drug such as L-BPA is not continuously administered during neutron irradiation, the drug can remain in the cell for a long time. As a result, the dose of the drug can be kept low as a whole, and the efficacy of BNCT can be effectively exhibited.

### [Drug for BNCT]

The tetrahydronaphthalene derivative of the present invention can contain a boron-containing group instead of or in addition to any of the groups in the above compounds. The boron-containing group is preferably any of boronic acid (-B(OH)₂), a boronic acid ester, a boronic acid amide, or a boron cluster. The boron cluster is preferably any of sodium borocaptate (BSH), dodecaborate, decaborate, or carborane. Such a compound may be conveniently used for BNCT in the form of the compound as it is or a pharmaceutically acceptable salt, or in the form of a formulation known to those skilled in the art by mixing with a pharmaceutically acceptable carrier or in the form of encapsulation in micro/nanoparticles, or the like.

In the tetrahydronaphthalene derivative of the present invention, examples of the boronic acid ester or boronic acid amide group in this definition represent a group having the chain structure such as -B(NR⁷¹)₂ or - B(OR⁷¹)₂, or a group having the cyclic structure together with the atom B. R⁷¹ represents a linear or branched C1-C10 alkyl group. The expression "linear or branched C1-C10 alkyl group" may be any alkyl group having 1 to 10 carbon atoms. A linear or branched C1-C8 alkyl group is preferred, and a linear or branched C1-6 alkyl group is more preferred. Examples of these groups include, but are not limited to, a methyl group, an ethyl group, an isopropyl group, and a butyl group.

In the group having the cyclic structure together with the boron atom B, only the O atom is not necessarily interposed, but the N atom may be interposed. Although not limited to the following, the group having a cyclic structure together with the boron atom B is, for example, a group including an ester or ester analog composed of an atom B and any one selected from the group consisting of pinacol, 2,2-dimethyl-1,3-propanediol, N-methyldiethanolamine, 1-6-diaminonaphthalene, N-methyliminodiacetic acid, 1,1,1-trishydroxymethylethane, and catechol. These include, but are not limited to, for example, cyclic forms of boron with boronic acid pinacol ester, boronic acid MIDA ester, boronic acid 1,3-propanediol ester, boronic acid neopentyl glycol ester, boronic acid catechol ester, boronic acid pinanediol ester, boronic acid biscyclohexyldiol ester, boronic acid MPM ester, trifluoroborate salt, cyclic triol borate salt, and diaminonaphthalenamide.

The boron atom is not limited, and the proportion of boron 10 may be preferably 75 mass% or more, more preferably 80 mass% or more, further more preferably 90 mass% or more, and particularly preferably 95 mass% or more.

In natural boron (boron), boron 10 and boron 11 are present as isotopes at a ratio of boron 10 of 20% and boron 11 of 80%. Therefore, prior to the production of the tetrahydronaphthalene derivative of the present invention, it is also preferable to concentrate boron (boron 10) having a mass number of 10. In the present invention, for example, a commercially available product may be used as the boron atom source. As a commercially available product, for example, ¹⁰B Enriched Boric Acid (manufactured by STELLA CHEMIFA CORPORATION) can be used.

As a method of measuring boron 10, Agilent 710 (manufactured by Agilent) can be used, and multi-type ICP optical emission spectrometry (ICP-OES) can be performed. ICP-OES used for the measurement is adjusted in accordance with JIS K 0116.

The compound preferably contains, but is not limited to, boronic acid (B(OH)₂) or a boronic acid ester having the chain or cyclic structure together with a B atom, and is more preferably boronic acid (B(OH)₂) or a pinacol ester of boronic acid.

Such a compound containing a boron-containing group is obtained, for example, by reacting a boron compound with a compound represented by Formula (I) in a solvent in the presence of a palladium catalyst, an organophosphorus compound, and a base.

Examples of the palladium catalyst include, but are not limited to, palladium(II) acetate, palladium(II) chloride, tris(dibenzylideneacetone)dipalladium(0), [1,1'-bis(diphenylphosphino)ferrocene]palladium(II) dichloride dichloromethane adduct, and tetrakis(triphenylphosphine)palladium(0).

Examples of the organophosphorus compound include, but are not limited to, triphenylphosphine, tricyclohexylphosphine, 1,1'-bis(diphenylphosphino)ferrocene, 2-dicyclohexylphosphino-2',6'-dimethoxybiphenyl, 2-dicyclohexylphosphino-2',4',6'-triisopropylbiphenyl, and 2-dicyclohexylphosphino-2'-(N,N-dimethylamino)biphenyl.

Examples of the base include, but are not limited to, potassium acetate, sodium acetate, sodium carbonate, cesium carbonate, potassium carbonate, and sodium hydrogen carbonate.

Examples of the boron compound include a boric acid ester and a boric acid amide, and preferably refer to a compound represented by B(OR)₃, B(NR)₃, B(OR)₂(NR), (RO)₂B- or B(OR)(NR)₂ (R is a linear or branched C1-C10 alkyl group, a phenyl group, or a benzyl group), or the like. Among these, a compound represented by (RO)₂B-B(OR)₂ is particularly preferably used. The expression "linear or branched C1-C10 alkyl group" may be any alkyl group having 1 to 10 carbon atoms, and is preferably a linear or branched C1-C8 alkyl group, and more preferably a linear or branched C1-6 alkyl group. Examples of these groups include, but are not limited to, a methyl group, an ethyl group, an isopropyl group, and a butyl group. Typical examples of the boron compound include, but are not limited to, bis(pinacolato)diboron.

Examples of the solvent include, but are not limited to, ether-based solvents such as 1,4-dioxane, tetrahydrofuran, and 1,2-dimethoxyethane; hydrocarbon-based solvents such as toluene; and polar solvents such as N,N-dimethylformamide and dimethyl sulfoxide. Preferred solvents include dimethyl sulfoxide. The reaction temperature is, for example, 20°C to 160°C and preferably 60°C to 120°C.

When the tetrahydronaphthalene derivative of the present invention contains boron, treatment using formulation is performed by administration in any suitable route of administration in such a way that the tetrahydronaphthalene derivative accumulates in a target tumor. The tetrahydronaphthalene derivative is preferably concentrated in a tumor before irradiation with radiation, and the ratio of tumor : blood before irradiation with radiation is at least 1.5 or more : 1, and preferably 2 or more : 1. The tetrahydronaphthalene derivative can be administered at a time or can be continuously administered. In some cases, the tetrahydronaphthalene derivative can also be administered by being divided. After the compound desirably accumulates in the tumor, the site is irradiated with an effective amount of low-energy neutron radiation (for example, an epithermal neutron ray). The site can be irradiated through the skin, or the site can be fully or partially exposed before irradiation. The administration of the tetrahydronaphthalene derivative and irradiation with radiation subsequent to the administration can be repeated as necessary. If desired, the tetrahydronaphthalene derivative of the present invention as an anticancer agent can be used for residual tumor or metastatic tumor after treatment with BNCT.

### [Diagnostic agent containing radioisotope]

The tetrahydronaphthalene derivative of the present invention can also be prepared as a drug containing a radioisotope for diagnosis. In the case of preparation as a drug having radioisotope irradiating ability, although not limited to the following, typically, ¹⁸F can be used as the F atom contained in the compound, ¹³¹I or ¹²³I can be used as the I atom contained in the compound, or ¹¹C can be used as the C atom contained in the compound. The compound thus obtained can be used for, for example, RI inspection or nuclear medicine inspection. These include, but are not limited to, drugs for scintigraphy tomography, single photon emission computed tomography (SPECT), and positron emission tomography (PET). That is, the tetrahydronaphthalene derivative of the present invention having radioactivity is administered to a subject as a drug for PET or a drug for SPECT, and an image is acquired before treatment, so that information on the bioaccumulation distribution of the derivative, the tumor tissue/normal tissue abundance ratio (T/N ratio), and the like can be obtained. Based on these pieces of information, it is also possible to presume the therapeutic effect of BNCT and formulate a study or treatment plan. The administration mode and the like are in accordance with the contents described in the section of [Drug for BNCT].

In the present specification, "cancer" in the term of "cancer therapeutic drug" has the same meaning as "malignant tumor".

"Cancer" or "malignant tumor" described herein includes both epithelial ("cancer") and non-epithelial ("sarcoma" (for example, osteosarcoma or leukemia)). Although not particularly limited, brain tumors including glioblastoma, malignant glioma, and the like, other head and neck cancers, malignant melanoma, skin cancer, breast cancer, lung cancer, uterine cancer, ovarian cancer, kidney cancer, pancreatic cancer, biliary tract cancer, pancreatic cancer, liver cancer, colon cancer, esophageal cancer, stomach cancer, tongue cancer, prostate cancer, osteosarcoma, multiple myeloma, leukemia, and the like may be particularly suitable targets.

Diseases other than cancer in which LAT1 is involved in the formation of pathological conditions, such as autoimmune diseases such as multiple sclerosis and rheumatoid arthritis, and allergic diseases such as atopic dermatitis, may also be targets.

The tetrahydronaphthalene derivative of the present invention may be administered orally and parenterally. In the case of parenteral administration, the tetrahydronaphthalene derivative may be administered intravenously, intraarterially (for example, via carotid artery), intramuscularly, subcutaneously, intramedullary, intrathecally, intracerebroventricularly, intraperitoneally, intrathoracicly, intrapelvicly, or intranasally.

The formulation may be in any form of powder, granules, subtle granules, dry syrup, tablet, capsule, injection, liquid, and the like. Depending on its dosage form, it may also be administered to a patient by a pharmaceutically known procedure in admixture of suitable additives and/or pharmaceutically acceptable carriers, singly, or in combination with other drugs. Examples of the additive include an excipient; a disintegrant; a binder; a lubricant; a diluent; buffering agents such as phosphoric acid, citric acid, succinic acid, acetic acid, and other organic acids or salts thereof; an isotonic agent; a preservative; a wetting agent; an emulsifier; a dispersant; a stabilizer; a solubilizing agent; antioxidants such as ascorbic acid; low-molecular-weight (less than about 10 residues) polypeptides (for example, polyarginine or tripeptide); proteins (for example, serum albumin, gelatin, or immunoglobulins); hydrophilic polymers (for example, polyvinylpyrrolidone); amino acids (for example, glycine, glutamic acid, aspartic acid, or arginine); monosaccharides, disaccharides and other carbohydrates (including cellulose or derivatives thereof, glucose, mannose, or dextrin); chelate agents (for example, EDTA); sugar alcohols (for example, mannitol or sorbitol); counter ions (for example, sodium); and/or nonionic surfactants (for example, polysorbate or poloxamer). The formulation can be prepared by appropriately mixing or diluting/dissolving these pharmaceutical additives. A carrier to be preferably used is not limited, and is a pharmaceutically inert aqueous carrier. Examples of such a carrier include saline, buffered saline, dextrose, and water. In one embodiment of the present invention, the pharmaceutically acceptable carrier is pharmaceutically inactive. Suitable additives and/or pharmaceutically acceptable carriers are non-toxic to recipients at the dosages and concentrations employed. Particularly preferred in the formulation are injections prepared with an aqueous carrier.

Techniques for formulation and administration are described, for example, in the latest edition of the Japanese Pharmacopoeia and its latest supplement, and in the final version of "REMINGTON'S PHARMACEUTICAL SCIENCES" (Maack Publishing Co. Easton, PA).

The formulations of the tetrahydronaphthalene derivative of the present invention are drugs contained in amounts effective to achieve the intended purpose of the drug of interest, and "therapeutically effective amount" or "pharmacologically effective amount" refers to an amount of drug that is sufficiently appreciated by those skilled in the art to produce pharmacological results. The determination of a therapeutically effective dose is well known to those skilled in the art.

The therapeutically effective amount herein refers to the amount of the drug that alleviates the condition of the disease after administration of the drug. Such a therapeutic effect and toxicity of the compound may be determined by standard pharmacological procedures in cell cultures or experimental animals. The dose is preferably within a range of circulating concentrations including ED₅₀ with little or no toxicity. This dose varies within this range depending on the form of administration used, the sensitivity of the patient, and the route of administration. As an example, the dose is appropriately selected according to the age and other conditions of the patient, the type of disease, the type of preparation, and the like.

### Examples

The present invention will be described in more detail with reference to the following Examples, however, this invention is not limited thereto.

In the following Examples, the compounds were analyzed and separated and purified using the following models and reagents.

. NMR spectrum: (JEOL RESONANCE/JNM-ECZ500R/500MHz

### (Example 1-1)

### Production of 2-carboxy-7-(naphthalene-2-yloxy)-1,2,3,4-tetrahydronaphthalene-2-aminium chloride (Compound 1-5)

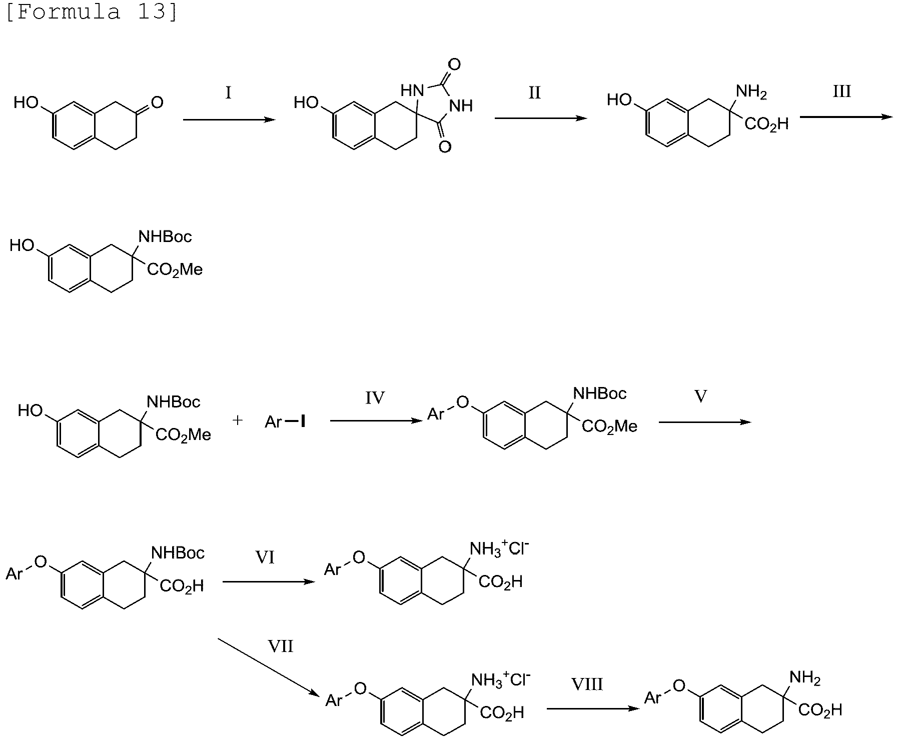

### Step 1 (Step I)

### Production of 7'-hydroxy-3',4'-dihydro-1'H-spiro[imidazolidine-4,2'-naphthalene]-2,5-dione

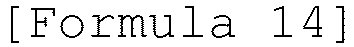

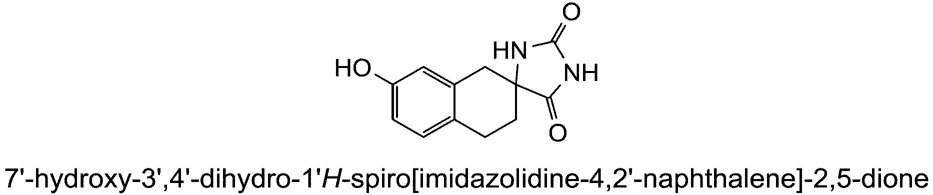

7-hydroxy-2-tetralone (2.90 g, 17.9 mmol), ammonium carbonate (13.7 g, 143 mmol), and sodium cyanide (1.32 g, 26.9 mmol) were dissolved in 50% ethanol water (73 mL), and the mixture was stirred for 18 hours. Thereafter, the mixture was cooled, and the obtained solid was collected by filtration and further washed with 50% ethanol water. The obtained solid was dried under reduced pressure to obtain a target compound (3.42 g). This dried hydantoin compound was used as it was for the next step without purification.

### Step 2 (Step II)

### Production of 2-amino-7-hydroxy-1,2,3,4-tetrahydronaphthalene-2-carboxylic acid

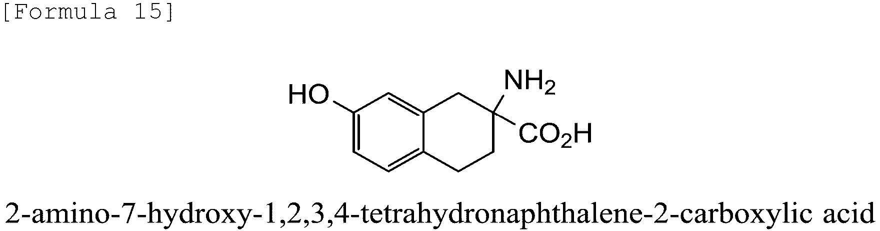

The hydantoin compound (3.42 g, 14.7 mmol) obtained in Step 1 was suspended in an aqueous sodium hydroxide solution in which sodium hydroxide (11.8 g, 295 mmol) was dissolved in pure water (59 mL). This was heated and refluxed. The mixture was refluxed for 18 hours, then cooled, and neutralized with 10% hydrochloric acid. The obtained precipitate was collected by filtration and further washed with cold water. The obtained solid was dried under reduced pressure to obtain a target compound (2.67 g). This dried amino acid compound was used as it was for the next step without purification.

### Step 3 (Step III)

### Production of methyl 2-((tert-butoxycarbonyl)amino)-7-hydroxy-1,2,3,4-tetrahydronaphthalene-2-carboxylate

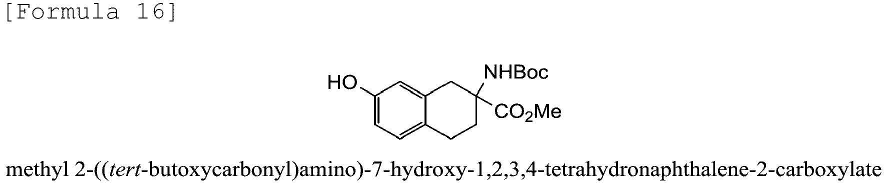

The hydrolyzed compound (2.67 g, 12.9 mmol) obtained above was suspended in methanol (54 mL) and then cooled, and thionyl chloride (1.40 mL, 19.4 mmol) was then added dropwise thereto. After completion of the dropwise addition, the temperature was raised, and the mixture was refluxed for 3 hours. Thereafter, methanol as a solvent was distilled off under reduced pressure. This reaction solution was suspended again in methanol (54 mL), and triethylamine (7.2 mL, 51.6 mmol) and Boc₂O (41.5 mL, 18.1 mmol) were added thereto, and the mixture was stirred at room temperature for 18 hours. Thereafter, distillation under reduced pressure was performed, and this residue was dissolved in ethyl acetate (54 mL). The obtained organic phase was washed with 1 N HCl, pure water, and saturated saline. The organic phase was dried over anhydrous magnesium sulfate and then removed by filtration, the solvent of the solution obtained by filtration was distilled off, and the residue was roughly purified by silica gel column chromatography to obtain a target compound (2.78 g, total yield 48%).

¹H-NMR (CDCl₃); 1.42 (s, 9H), 2.05-2.12 (m, 1H), 2.38-2.45 (m, 1H), 2.75-2.77 (m, 2H), 2.90 (d, J = 16.0 Hz, 1H), 3.25 (d, J = 15.5 Hz, 1H), 3.76 (s, 3H), 4.89 (s, 1H), 5.89 (brs, 1H), 6.54 (s, 1H), 6.66 (dd, J = 2.5, 8.0 Hz, 1H), 6.96 (d, J = 8.0 Hz, 1H).

### Step 4 (Step IV)

### Production of methyl 2-((tert-butoxycarbonyl)amino)-7-(naphthalene-2-yloxy)-1,2,3,4-tetrahydronaphthalene-2-carboxylate

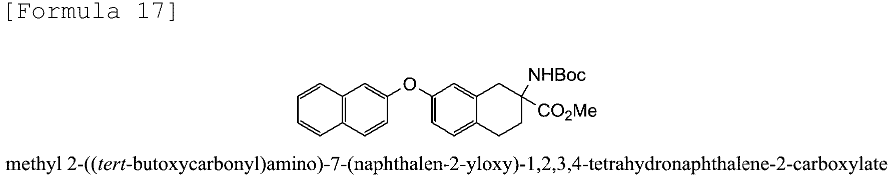

The phenol form (280 mg, 0.871 mmol) obtained in Step 3, 2-iodonaphthalene (266 mg, 1.05 mmol), cesium carbonate (851 mg, 2.61 mmol), copper(I) iodide (50 mg, 0.261 mmol), N,N-dimethylglycine hydrochloride (36 mg, 0.261 mmol), and dioxane (5 mL) were added to a pressure-resistant tube. After sealing, the mixture was reacted at 90°C for about 18 hours. Thereafter, the reaction solution was cooled in an ice bath, and ethyl acetate (50 mL) and distilled water (25 mL) were added thereto. After stirring for about 5 minutes, celite filtration was performed and the obtained filtrate was transferred to a separatory funnel. The aqueous phase was further extracted with ethyl acetate (25 mL), and the obtained organic phase was combined and washed with saturated saline (49 mL). The organic phase was dried over anhydrous magnesium sulfate and then removed by filtration, and the residue obtained by filtration was purified by silica gel column chromatography to obtain a target product (250 mg, yield 64%).

¹H-NMR (CDCl₃); 1.43 (s, 9H), 2.11-2.17 (m, 1H), 2.82-2.85 (m, 2H), 2.91 (d, J = 16.5 Hz, 1H), 3.28 (d, J = 17.0 Hz, 1H), 3.76 (s, 3H), 4.89 (brs, 1H), 6.77 (d, J = 3.0 Hz, 1H), 6.88 (dd, J = 2.5, 8.5 Hz, 1H), 7.10 (d, J = 8.0 Hz, 1H), 7.24 (dd, J = 2.5, 9.5 Hz, 1H), 7.29 (d, J = 2.5 Hz, 1H), 7.38-7.46 (m, 2H), 7.70 (d, J = 8.0 Hz, 1H), 7.82 (dd, J = 2.5, 8.0 Hz, 1H).

### Step 5 (Step V)

### Production of 2-((tert-butoxycarbonyl)amino)-7-(naphthalene-2-yloxy)-1,2,3,4-tetrahydronaphthalene-2-carboxylic acid

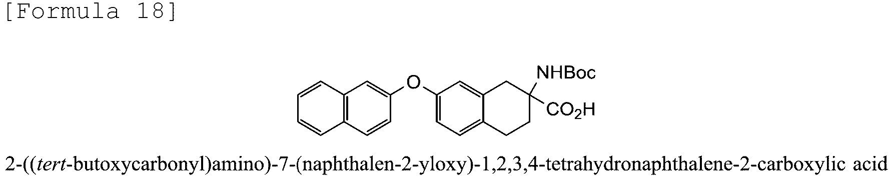

The diaryl ether compound (250 mg, 0.559 mmol) obtained in Step 2 was dissolved in methanol (10 mL), and an aqueous sodium hydroxide solution in which sodium hydroxide (0.22 g, 5.59 mmol) was dissolved in pure water (2.2 mL) was added thereto. The mixture was reacted at 55°C for 1.5 hours, and then the solvent was distilled off under reduced pressure. The pH of the mixture was adjusted to 7 or less with 1 N aqueous hydrochloric acid, and the mixture was extracted with ethyl acetate (50 mL). The aqueous phase was further extracted with ethyl acetate (25 mL), and the obtained organic phase was combined and washed with pure water and saturated saline. The organic phase was dried over anhydrous magnesium sulfate and then removed by filtration to obtain a target product (180 mg, yield 74%). This carboxylic acid compound was used as it was for the next step without purification.

### Step 6 (Step VI)

### Production of 2-carboxy-7-(naphthalene-2-yloxy)-1,2,3,4-tetrahydronaphthalene-2-aminium chloride

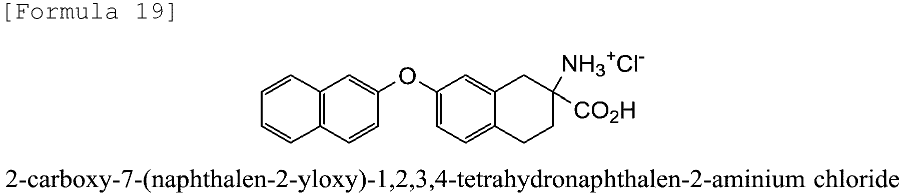

The carboxylic acid compound (180 mg, 0.415 mmol) obtained in Step 5 was dissolved in CPME (10 mL), and 3M HCl-CPME (10 mL) was added thereto. This was reacted at 50°C for 2 hours. After completion of the reaction, the reaction solution was cooled in an ice bath, and the precipitated individual was collected by filtration. The precipitated individual was washed with CPME and diethyl ether and then dried under reduced pressure to obtain a target product (91 mg, yield 59%).

¹H-NMR (CD₃OD); 2.16-2.21 (m, 1H), 2.41-2.47 (m, 1H), 2.88-2.95 (m, 1H), 3.00 (d, J = 16.5 Hz, 1H), 3.02-3.08 (m, 1H), 3.49 (d, J = 17.0 Hz, 1H), 6.77 (d, J = 3.0 Hz, 1H), 6.88 (d, J = 2.0 Hz, 1H), 6.94 (dd, J = 2.5, 8.0 Hz, 1H), 7.21-7.24 (m, 1H), 7.27 (d, J = 2.5 Hz, 1H), 7.38-7.46 (m, 2H), 7.68 (d, J = 8.0 Hz, 1H), 7.82-7.87 (m, 1H).

### (Example 1-2)

### Production of 7-([1,1'-biphenyl]-3-yloxy)-2-carboxy-1,2,3,4-tetrahydronaphthalene-2-aminium chloride (Compound 1-9)

### Step 1 (Step I-IV)

### Production of methyl 7-([1,1'-biphenyl]-3-yloxy)-2-( (tert-butoxycarbonyl)amino)-1,2,3,4-tetrahydronaphthalene-2-carboxylate

The following compound was obtained in the same manner as in Steps 1 to 4 in Example 1-1, except that 2-iodonaphthalene in Step 4 was changed to 3-iodo-1,1'-biphenyl.

¹H-NMR (CDCl₃); 1.42 (s, 9H), 2.09-2.15 (m, 1H), 2.48 (brs, 1H), 2.81-2.83 (m, 2H), 2.91 (d, J = 16.5 Hz, 1H), 3.27 (d, J = 17.0 Hz, 1H), 3.76 (s, 3H), 4.81 (brs, 1H), 6.75 (d, J = 2.0 Hz, 1H), 6.87 (dd, J = 3.0, 8.5 Hz, 1H), 6.96-6.98 (m, 1H), 7.09 (d, J = 8.0 Hz, 1H), 7.24 (t, J = 2.0 Hz, 1H), 7.32-7.44 (m, 5H), 7.55-7.57 (m, 2H).

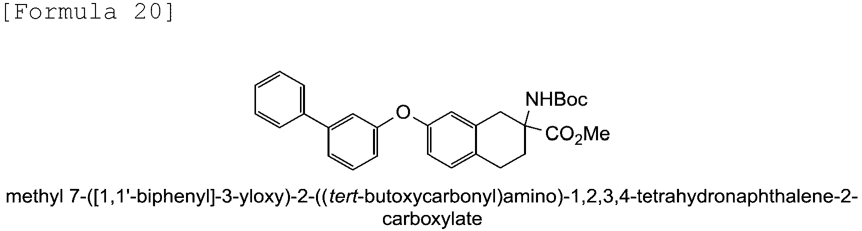

### Step 2 (Step V-VI)

### Production of 7-([1,1'-biphenyl]-3-yloxy)-2-carboxy-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.15-2.2 0 (m, 1H), 2.38-2.45 (m, 1H), 2.86-2.93 (m, 1H), 3.00 (d, J = 17.0 Hz, 1H), 3.00-3.06 (m, 1H), 3.49 (d, J = 17.0 Hz, 1H), 6.87 (d, J = 2.5 Hz, 1H), 6.93 (dd, J = 2.5, 8.5 Hz, 1H), 6.94-6.96 (m, 1H), 7.18 (t, J = 2.0, 1H), 7.21 (d, J = 8.0, 1H), 7.31-7.37 (m, 2H), 7.39-7.43 (m, 2H), 7.53-7.55 (m, 1H).

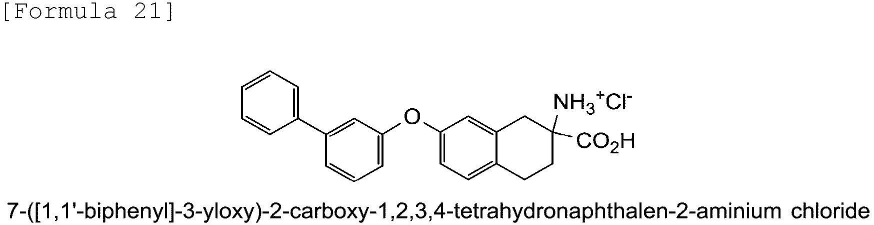

### (Example 1-3)

### Production of 7-(benzofuran-5-yloxy)-2-carboxy-1,2,3,4-tetrahydronaphthalene-2-aminium chloride (Compound 1-16)

### Step 1 (Step I-IV)

### Production of methyl 7-(benzofuran-5-yloxy)-2-((tert-butoxycarbonyl)amino)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 5-iodobenzofuran.

¹H-NMR (CDCl₃); 1.42 (s, 9H), 2.08-2.14 (m, 1H), 2.47 (brs, 1H), 2.79-2.82 (m, 2H), 2.88 (d, J = 17.0 Hz, 1H), 3.25 (d, J = 17.5 Hz, 1H), 3.76 (s, 3H), 4.81 (brs, 1H), 6.71 (d, J = 2.5 Hz, 1H), 6.80 (dd, J = 3.0, 8.5 Hz, 1H), 7.00 (dd, J = 2.5, 8.5 Hz, 1H), 7.06 (d, J = 9.0 Hz, 1H), 7.20 (d, J = 3.0 Hz, 1H), 7.46 (d, J = 8.5 Hz, 1H), 7.64 (d, J = 2.0 Hz, 1H).

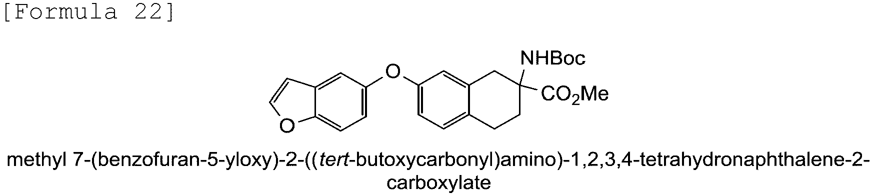

### Step 2 (Step V-VI)

### Production of 7-(benzofuran-5-yloxy)-2-carboxy-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.14-2.19 (m, 1H), 2.37-2.43 (m, 1H), 2.84-2.90 (m, 1H), 2.96 (d, J = 17.0 Hz, 1H), 2.99-3.04 (m, 1H), 3.45 (d, J = 17.5 Hz, 1H), 6.75 (d, J = 2.5 Hz, 1H), 6.79 (dd, J = 1.0, 2.0 Hz, 1H), 6.83 (dd, J = 3.0, 8.5 Hz, 1H), 6.97 (dd, J = 2.5, 8.5 Hz, 1H), 7.16 (d, J = 8.5, 1H), 7.21 (d, 3.0 Hz, 1H), 7.48 (d, J = 9.0 Hz, 1H), 7.77 (d, J = 2.5 Hz, 1H).

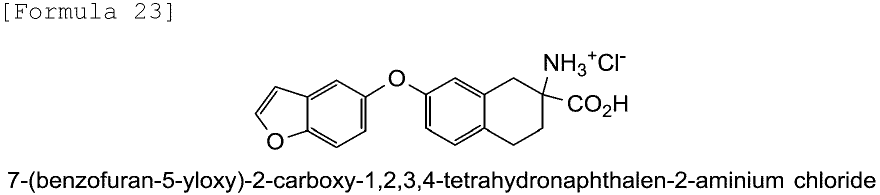

### (Example 1-4)

### Production of 2-carboxy-7-(naphthalene-1-yloxy)-1,2,3,4-tetrahydronaphthalene-2-aminium chloride (Compound 1-10)

### Step 1 (Step I-IV)

### Production of methyl 2-((tert-butoxycarbonyl)amino)-7-(naphthalene-1-yloxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 1-iodonaphthalene.

¹H-NMR (CDCl₃); 1.42 (s, 9H), 2.09-2.15 (m, 1H), 2.48 (brs, 1H), 2.81-2.84 (m, 2H), 2.88 (d, J = 16.5 Hz, 1H), 3.25 (d, J = 17.0 Hz, 1H), 3.76 (s, 3H), 4.80 (brs, 1H),

6.74 (d, J = 3.0 Hz, 1H), 6.86 (dd, J = 3.0, 8.5 Hz, 1H), 6.93 (dd, J = 1.0, 8.5 Hz, 1H), 7.08 (d, J = 8.5 Hz, 1H), 7.38 (t, J = 8.0 Hz, 1H), 7.47-7.54 (m, 2H), 7.61 (d, J = 8.0 Hz, 1H), 7.87 (dd, J = 1.0, 8.5 Hz, 1H), 8.19 (d, J = 8.0 Hz, 1H).

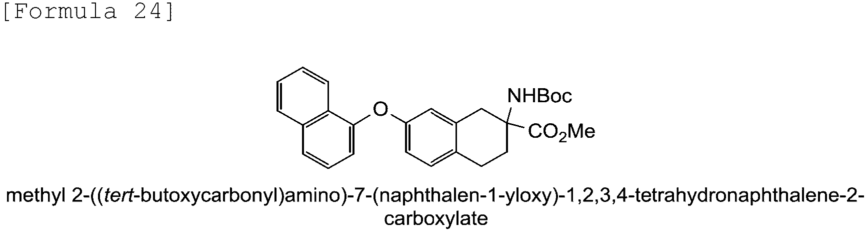

### Step 2 (Step V-VI)

### Production of 2-carboxy-7-(naphthalene-1-yloxy)-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.16-2.21 (m, 1H), 2.38-2.44 (m, 1H), 2.86-2.92 (m, 1H), 2.97 (d, J = 18.0 Hz, 1H), 3.00-3.05 (m, 1H), 3.45 (d, J = 17.5 Hz, 1H), 6.82 (d, J = 2.0 Hz, 1H), 6.89 (dd, J = 3.0, 8.5 Hz, 1H), 6.93 (dd, J = 1.0, 8.5 Hz, 1H), 7.19 (d, J = 9.0, 1H), 7.39 (t, J = 8.0, 1H), 7.45-7.48 (m, 1H), 7.50-7.53 (m, 1H), 7.65 (d, J = 8.0 Hz, 1H), 7.90 (d, J = 8.5 Hz, 1H), 8.09 (d, J = 8.5 Hz, 1H).

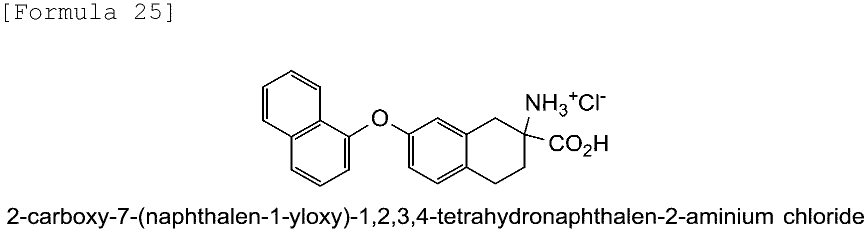

### (Example 1-5)

### Production of 2-carboxy-7-((7-methoxynaphthalene-2-yl)oxy)-1,2,3,4-tetrahydronaphthalene-2-aminium chloride (Compound 1-25)

### Step 1 (Step I-IV)

### Production of methyl 2-((tert-butoxycarbonyl)amino)-7-((7-methoxynaphthalene-2-yl)oxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 2-iodo-7-methoxynaphthalene.

¹H-NMR (CDCl₃); 1.43 (s, 9H), 2.10-2.16 (m, 1H), 2.50 (brs, 1H), 2.83-2.85 (m, 2H), 2.91 (d, J = 16.5 Hz, 1H), 3.28 (d, J = 17.0 Hz, 1H), 3.77 (s, 3H), 3.89 (s, 3H), 4.83 (brs, 1H), 6.80 (d, J = 2.5 Hz, 1H), 6.89 (dd, J = 2.5, 8.5 Hz, 1H), 7.00 (d, J = 2.5 Hz, 1H), 7.04-7.11 (m, 3H), 7.18 (d, J = 1.5 Hz, 1H), 7.70 (d, J = 9.0 Hz, 1H), 7.73 (d, J = 9.0 Hz, 1H).

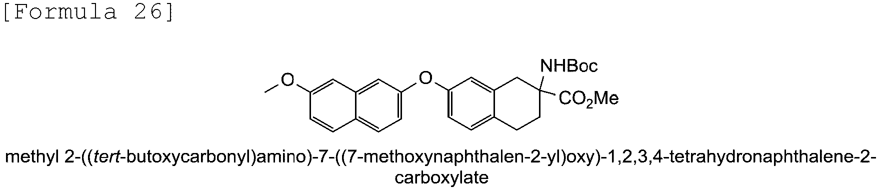

### Step 2 (Step V-VI)

### Production of 2-carboxy-7-((7-methoxynaphthalene-2-yl)oxy)-1,2,3,4-tetrahydronaphthalene-2-aminium chloride

The above compound was treated in the same manner as in Steps 5 and 6 in Example 1-1 to synthesize the following compound.

¹H-NMR (CD₃OD); 2.17-2.20 (m, 1H), 2.39-2.45 (m, 1H), 2.89-2.92 (m, 1H), 3.00 (d, J = 17.0 Hz, 1H), 3.02-3.05 (m, 1H), 3.48 (d, J = 17.5 Hz, 1H), 3.85 (s, 3H), 6.86 (s, 1H), 6.93 (d, J = 8.0 Hz, 1H), 7.02-7.07 (m, 3H), 7.20-7.22 (m, 2H), 7.71 (dd, J = 2.0, 8.5 Hz, 1H), 7.75 (dd, J = 2.5, 9.0, 1H).

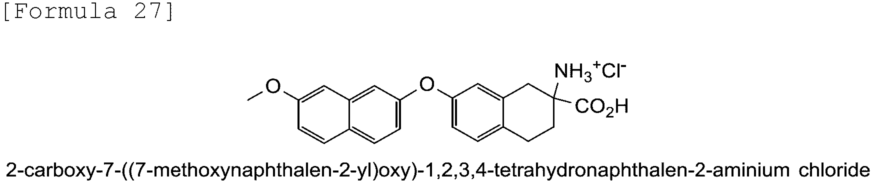

### (Example 1-6)

### Production of 2-amino-7-((6-(dimethylamino)naphthalene-2-yl)oxy)-1,2,3,4-tetrahydronaphthalene-2-carboxylic acid (Compound 1-2)

### Step 1 (Step I-IV)

### Production of methyl 2-((tert-butoxycarbonyl)amino)-7-((6-(dimethylamino)naphthalene-2-yl)oxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 6-iodo-N,N-dimethylnaphthalene-2-amine.

¹H-NMR (CDCl₃); 1.42 (s, 9H), 2.09-2.15 (m, 1H), 2.49 (brs, 1H), 2.80-2.83 (m, 2H), 2.87 (d, J = 16.5 Hz, 1H), 3.04 (s, 6H), 3.24 (d, J = 16.5 Hz, 1H), 3.76 (s, 3H), 4.81 (brs, 1H), 6.72 (d, J = 2.5 Hz, 1H), 6.83 (dd, J = 3.0, 8.5 Hz, 1H), 6.94 (d, J = 2.5 Hz, 1H), 7.06 (d, J = 9.0 Hz, 1H), 7.14 (dd, J = 2.5, 9.0 Hz, 1H), 7.18 (dd, J = 2.5, 9.5 Hz, 1H), 7.24 (d, J = 2.5 Hz, 1H), 7.60 (d, J = 9.5 Hz, 1H), 7.65 (d, J = 8.5 Hz, 1H).

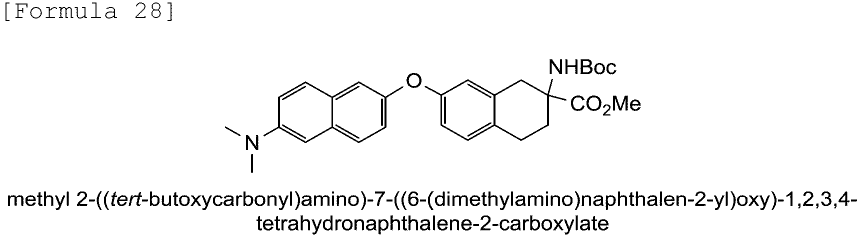

### Step 2 (Step V)

### Production of 2-((tert-butoxycarbonyl)amino)-7-((6-(dimethylamino)naphthalene-2-yl)oxy)-1,2,3,4-tetrahydronaphthalene-2-carboxylic acid

The above compound was treated in the same manner as in Step 5 in Example 1-1 to synthesize the following compound. This carboxylic acid compound was used as it was for the next step without purification.

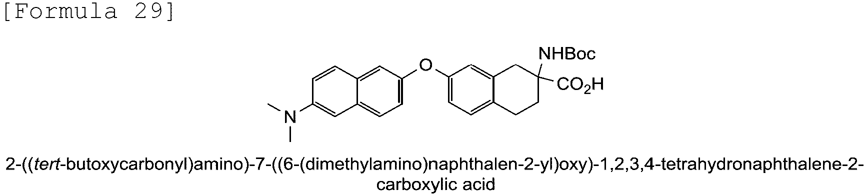

### Step 3 (Step VII-VIII)

### Production of 2-amino-7-((6-(dimethylamino)naphthalene-2-yl)oxy)-1,2,3,4-tetrahydronaphthalene-2-carboxylic acid

To the above compound (70 mg, 0.147 mmol), 10% hydrochloric acid was added, and the mixture was stirred under reflux for 1 hour. After cooling, the precipitate was observed when the pH was increased to 7 with a 1 H aqueous sodium hydroxide solution. This precipitate was collected by filtration, and further this precipitate was purified by silica gel column chromatography to obtain a target compound (41 mg, yield 74%) described below.

¹H-NMR (DMSO-d6); 1.85-1.87 (m, 1H), 2.06-2.08 (m, 1H), 2.73 (d, J = 18.0 Hz, 1H), 2.74-2.82 (m, 2H), 2.98 (s, 6H), 3.26 (d, J = 17.5 Hz, 1H), 6.73 (d, J = 2.0 Hz, 1H), 6.77 (dd, J = 3.0, 9.0 Hz, 1H), 6.97 (d, J = 2.0, 1H), 7.09-7.12 (m, 2H), 7.21-7.24 (m, 2H), 7.65 (d, J = 9.5 Hz, 1H), 7.71 (d, J = 8.5, 1H).

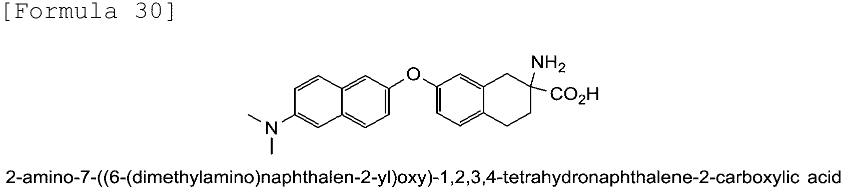

### (Example 1-7)

### Production of 2-amino-7-((7-(dimethylamino)naphthalene-2-yl)oxy)-1,2,3,4-tetrahydronaphthalene-2-carboxylic acid (Compound 1-1)

### Step 1 (Step I-IV)

### Production of methyl 2-((tert-butoxycarbonyl)amino)-7-((7-(dimethylamino)naphthalene-2-yl)oxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 7-iodo-N,N-dimethylnaphthalene-2-amine.

¹H-NMR (CDCl₃); 1.43 (s, 9H), 2.10-2.16 (m, 1H), 2.50 (brs, 1H), 2.82-2.84 (m, 2H), 2.89 (d, J = 16.0 Hz, 1H), 3.03 (s, 6H), 3.26 (d, J = 16.5 Hz, 1H), 3.76 (s, 3H), 4.81 (brs, 1H), 6.76-6.78 (m, 2H), 6.88 (dd, J = 3.0, 9.0 Hz, 1H), 6.96 (dd, J = 2.5, 9.0 Hz, 1H), 7.06-7.11 (m, 2H), 7.65-7.67 (m, 2H).

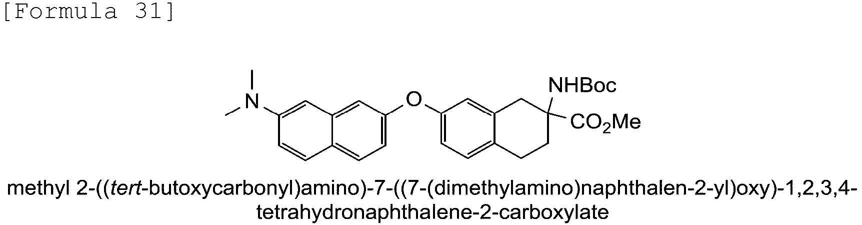

### Step 2 (Step V)

### Production of 2-((tert-butoxycarbonyl)amino)-7-((7-(dimethylamino)naphthalene-2-yl)oxy)-1,2,3,4-tetrahydronaphthalene-2-carboxylic acid

The above compound was treated in the same manner as in Step 5 in Example 1-1 to synthesize the following compound. This carboxylic acid compound was used as it was for the next step without purification.

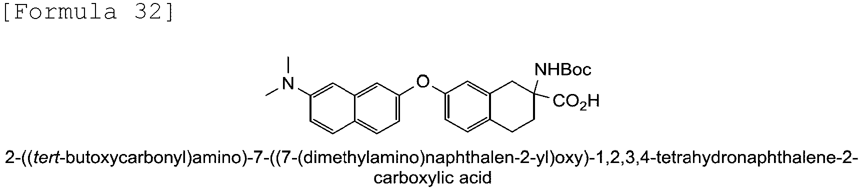

### Step 3 (Step VII-VIII)

### Production of 2-amino-7-((7-(dimethylamino)naphthalene-2-yl)oxy)-1,2,3,4-tetrahydronaphthalene-2-carboxylic acid

The above compound was treated in the same manner as in Step 3 in Example 1-6 to synthesize the following compound.

¹H-NMR (DMSO-d6); 1.84-1.87 (m, 1H), 2.06-2.12 (m, 1H), 2.71 (d, J = 17.0 Hz, 1H), 2.72-2.83 (m, 2H), 2.97 (s, 6H), 3.30 (d, J = 17.0 Hz, 1H), 6.79-6.82 (m, 3H), 6.89 (dd, J = 2.0, 8.5 Hz, 1H), 7.06 (d, J = 2.5 Hz, 1H), 7.10-7.12 (m, 2H), 7.68-7.74 (m, 2H).

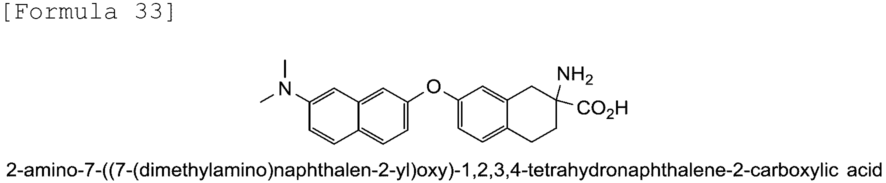

### (Example 1-8)

### Production of 2-amino-7-(quinoline-6-yloxy)-1,2,3,4-tetrahydronaphthalene-2-carboxylic acid (Compound 1-18)

### Step 1 (Step I-IV)

### Production of methyl 2-((tert-butoxycarbonyl)amino)-7-(quinoline-6-yloxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 3-(3-iodophenyl)pyridine.

¹H-NMR (CDCl₃); 1.43 (s, 9H), 2.12-2.18 (m, 1H), 2.49 (brs, 1H), 2.85-2.87 (m, 2H), 2.94 (d, J = 16.5 Hz, 1H), 3.31 (d, J = 16.5 Hz, 1H), 3.77 (s, 3H), 4.85 (brs, 1H), 6.80 (d, J = 3.0 Hz, 1H), 6.90 (dd, J = 2.5, 8.0 Hz, 1H), 7.14 (d, J = 8.5 Hz, 1H), 7.21 (d, J = 3.0 Hz, 1H), 7.37 (dd, J = 4.5, 8.5 Hz, 1H), 7.48 (dd, J = 2.0, 9.0 Hz, 1H), 8.01 (dd, J = 1.0, 8.5 Hz, 1H), 8.08 (d, J = 9.5 Hz, 1H), 8.83 (dd, J = 1.5, 4.0 Hz, 1H).

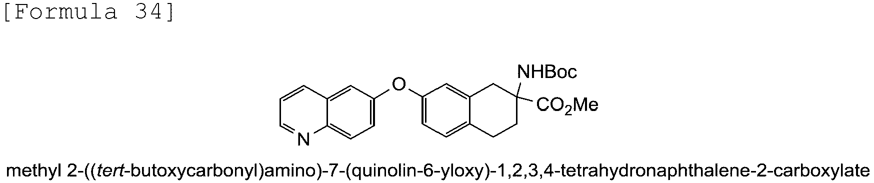

### Step 2 (Step V)

### Production of 2-((tert-butoxycarbonyl)amino)-7-(quinoline-6-yloxy)-1,2,3,4-tetrahydronaphthalene-2-carboxylic acid

The above compound was treated in the same manner as in Step 2 in Example 1-6 to synthesize the following compound. This carboxylic acid compound was used as it was for the next step without purification.

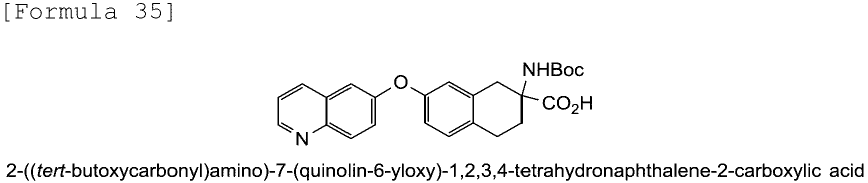

### Step 3

### Production of 2-amino-7-(quinoline-6-yloxy)-1,2,3,4-tetrahydronaphthalene-2-carboxylic acid

The above compound was treated in the same manner as in Step 3 in Example 1-6 to synthesize the following compound.

¹H-NMR (DMSO-d6); 1.84-1.87 (m, 1H), 2.07-2.13 (m, 1H), 2.74 (d, J = 17.5 Hz, 1H), 2.78-2.84 (m, 2H), 3.32 (d, J = 17.0 Hz, 1H), 6.88-6.90 (m, 2H), 7.16 (d, J = 9.0 Hz, 1H), 7.35 (d, J = 2.5 Hz, 1H), 7.48-7.52 (m, 2H), 8.04 (d, J = 9.0 Hz, 1H), 8.25 (dd, J = 1.5, 8.5 Hz, 1H), 8.81 (dd, J = 1.5, 4.5 Hz, 1H).

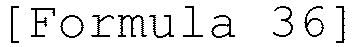

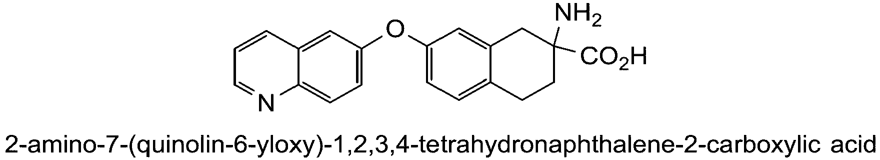

### (Example 1-9)

### Production of 2-amino-7-(3-(pyridine-2-yl)phenoxy)-1,2,3,4-tetrahydronaphthalene-2-carboxylic acid (Compound 1-21)

### Step 1 (Step I-IV)

### Production of methyl 2-((tert-butoxycarbonyl)amino)-7-(3-(pyridine-2-yl)phenoxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 2-(3-iodophenyl)pyridine.

¹H-NMR (CDCl₃); 1.42 (s, 9H), 2.09-2.15 (m, 1H), 2.49 (brs, 1H), 2.81-2.83 (m, 2H), 2.90 (d, J = 17.0 Hz, 1H), 3.26 (d, J = 17.5 Hz, 1H), 3.76 (s, 3H), 4.83 (brs, 1H), 7.03-7.05 (m, 1H), 7.08 (d, J = 8.5 Hz, 1H), 7.23-7.25 (m, 1H), 7.43 (t, J = 8.0 Hz, 1H), 7.64-7.65 (m, 1H), 7.69 (d, J = 8.0 Hz, 1H), 7.72-7.77 (m, 1H), 8.67-8.68 (m, 1H).

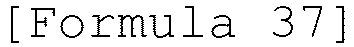

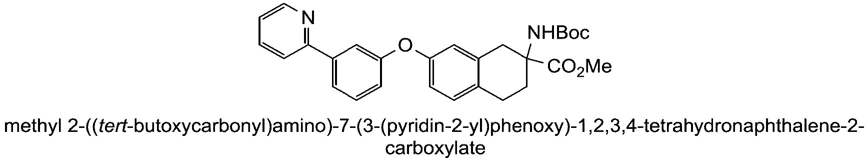

### Step 2 (Step V)

### Production of 2-((tert-butoxycarbonyl)amino)-7-(3-(pyridine-2-yl)phenoxy)-1,2,3,4-tetrahydronaphthalene-2-carboxylic acid

The above compound was treated in the same manner as in Step 2 in Example 1-6 to synthesize the following compound. This carboxylic acid compound was used as it was for the next step without purification.

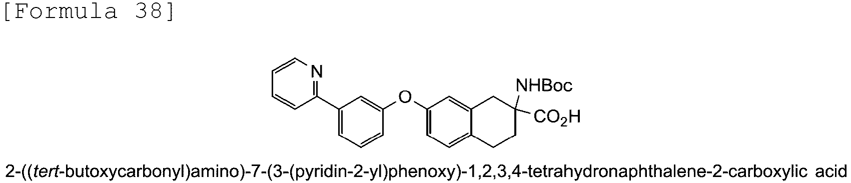

### Step 3

### Production of 2-amino-7-(3-(pyridine-2-yl)phenoxy)-1,2,3,4-tetrahydronaphthalene-2-carboxylic acid

The above compound was treated in the same manner as in Step 3 in Example 1-6 to synthesize the following compound.

¹H-NMR (DMSO-d6); 1.81-1.86 (m, 1H), 2.04-2.10 (m, 1H), 2.67-2.80 (m, 2H), 2.71 (d, J = 17.0 Hz, 1H), 3.29 (d, J = 17.5 Hz, 1H), 6.81-6.92 (m, 2H), 7.02-7.04 (m, 1H), 7.10 (d, J = 9.0 Hz, 1H), 7.33-7.36 (m, 1H), 7.47 (t, J = 8.0 Hz, 1H), 7.70 (s, 1H), 7.80 (d, J = 7.0 Hz, 1H), 7.86 (t, J = 7.5 Hz, 1H), 7.94 (d, J = 7.5 Hz, 1H), 8.62 (d, J = 4.5 Hz, 1H).

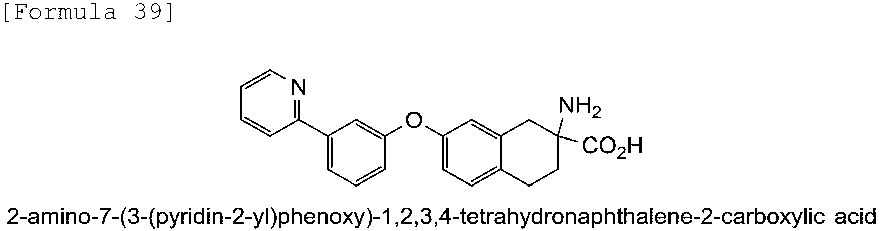

### (Example 1-10)

### Production of 2-carboxy-7-((4'-chloro-[1,1'-biphenyl]-3-yl)oxy)-1,2,3,4-tetrahydronaphthalene-2-aminium chloride (Compound 1-31)

### Step 1 (Step I-IV)

### Production of methyl 2-((tert-butoxycarbonyl)amino)-7-((4'-chloro-[1,1'-biphenyl]-3-yl)oxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 3-bromo-4'-chloro-1,1'-biphenyl.

¹H-NMR (CDCl₃); 1.42 (s, 9H), 2.09-2.15 (m, 1H), 2.47 (brs, 1H), 2.81-2.84 (m, 2H), 2.92 (d, J = 17.5 Hz, 1H), 3.28 (d, J = 17.0 Hz, 1H), 3.76 (s, 3H), 4.81 (brs, 1H), 6.75 (d, J = 2.5 Hz, 1H), 6.87 (dd, J = 2.5, 8.0 Hz, 1H), 6.97 (dd, J = 2.0, 7.5 Hz, 1H), 7.09 (d, J = 8.0 Hz, 1H), 7.19 (t, J = 2.0 Hz, 1H), 7.27-7.29 (m, 1H), 7.37-7.40 (m, 3H), 7.47 7.50 (m, 2H).

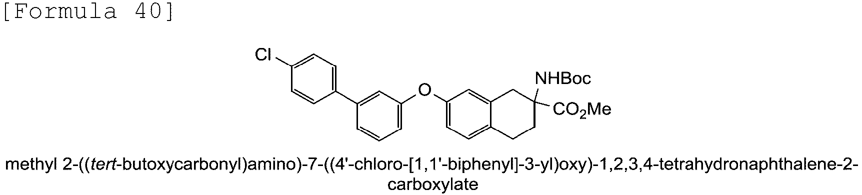

### Step 2 (Step V-VI)

### Production of 2-carboxy-7-((4'-chloro-[1,1'-biphenyl]-3-yl)oxy)-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.16-2.21 (m, 1H), 2.38-2.45 (m, 1H), 2.86-2.93 (m, 1H), 3.00 (d, J = 16.5 Hz, 1H), 3.00-3.06 (m, 1H), 3.49 (d, J = 17.0 Hz, 1H), 6.86 (d, J = 2.0 Hz, 1H), 6.92 (dd, J = 2.0, 8.5 Hz, 1H), 6.96 (dd, J = 2.5, 8.5 Hz, 1H), 7.19 (brs, 1H), 7.21 (t, J = 8.0, 1H), 7.35 (d, J =, 7.5 Hz, 1H), 7.40-7.43 (m, 3H), 7.54 (d, J = 8.0 Hz, 1H).

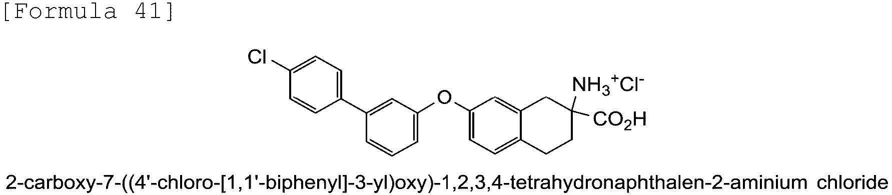

### (Example 1-11)

### Production of 2-carboxy-7-((3'-chloro-[1,1'-biphenyl]-3-yl)oxy)-1,2,3,4-tetrahydronaphthalene-2-aminium chloride (Compound 1-30)

### Step 1 (Step I-IV)

### Production of methyl 2-((tert-butoxycarbonyl)amino)-7-((3'-chloro-[1,1'-biphenyl]-3-yl)oxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 3-bromo-3'-chloro-1,1'-biphenyl.

¹H-NMR (CDCl₃); 1.42 (s, 9H), 2.09-2.16 (m, 1H), 2.48 (brs, 1H), 2.81-2.84 (m, 2H), 2.92 (d, J = 17.0 Hz, 1H), 3.28 (d, J = 16.5 Hz, 1H), 3.76 (s, 3H), 4.82 (brs, 1H), 6.74 (d, J = 2.5 Hz, 1H), 6.86 (dd, J = 3.0, 8.5 Hz, 1H), 6.97-7.00 (m, 1H), 7.09 (d, J = 8.5 Hz, 1H), 7.20 (t, J = 2.5 Hz, 1H), 7.28-7.41 (m, 4H), 7.43 (dt, J = 1.5, 8.0 Hz, 1H), 7.54 (t, J = 2.0 Hz, 1H).

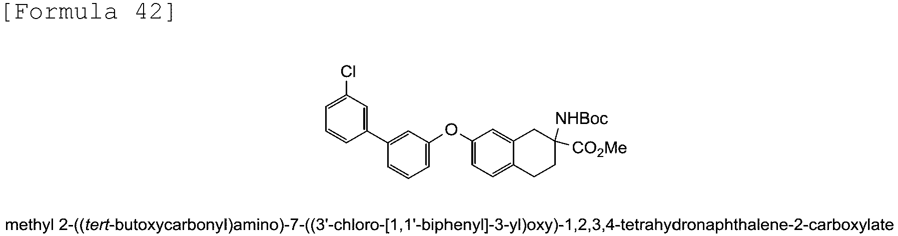

### Step 2 (Step V-VI)

### Production of 2-carboxy-7-((3'-chloro-[1,1'-biphenyl]-3-yl)oxy)-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.15-2 .20 (m, 1H), 2.39-2.45 (m, 1H), 2.86-2.93 (m, 1H), 3.00 (d, J = 18.0 Hz, 1H), 3.01-3.07 (m, 1H), 3.50 (d, J = 17.5 Hz, 1H), 6.87 (d, J = 2.5 Hz, 1H), 6.93 (dd, J = 2.5, 8.5 Hz, 1H), 6.99 (dd, J = 2.5, 8.5 Hz, 1H), 7.18 (t, J = 2.0, 1H), 7.22 (d, J = 8.5, 1H), 7.34-7.36 (m, 2H), 7.39-7.45 (m, 2H), 7.49 (dt, J = 1.5, 7.5 Hz, 1H), 7.55 (t, J = 2.5 Hz, 1H).

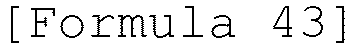

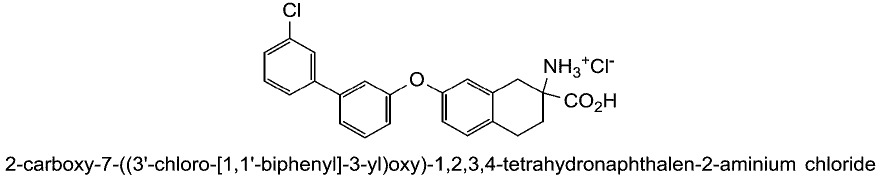

### (Example 1-12)

### Production of 7-([1,1'-biphenyl]-4-yloxy)-2-carboxy-1,2,3,4-tetrahydronaphthalene-2-aminium chloride (Compound 1-19)

### Step 1 (Step I-IV)

### Production of methyl 7-([1,1'-biphenyl]-4-yloxy-2-((tert-butoxycarbonyl)amino)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 4-iodo-1,1'-biphenyl.

¹H-NMR (CDCl₃); 1.43 (s, 9H), 2.10-2.16 (m, 1H), 2.49 (brs, 1H), 2.82-2.84 (m, 2H), 2.92 (d, J = 17.0 Hz, 1H), 3.28 (d, J = 17.0 Hz, 1H), 3.77 (s, 3H), 4.83 (brs, 1H), 6.76 (d, J = 2.0 Hz, 1H), 6.87 (dd, J = 2.0, 8.0 Hz, 1H), 7.04-7.07 (m, 2H), 7.10 (d, J = 8.5 Hz, 1H), 7.31-7.35 (m, 1H), 7.42-7.45 (m, 2H), 7.53-7.58 (m, 4H).

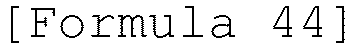

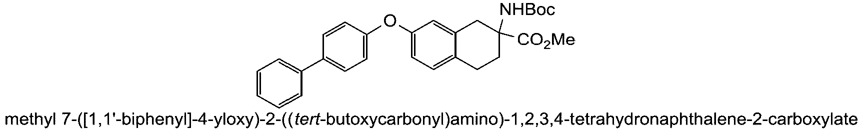

### Step 2 (Step V-VI)

### Production of 7-([1,1'-biphenyl]-4-yloxy)-2-carboxy-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.16-2.21 (m, 1H), 2.39-2.45 (m, 1H), 2.86-2.93 (m, 1H), 3.00 (d, J = 17.0 Hz, 1H), 3.00-3.06 (m, 1H), 3.49 (d, J = 17.0 Hz, 1H), 6.85 (d, J = 2.0 Hz, 1H), 6.91 (dd, J = 2.0, 8.0 Hz, 1H), 7.03-7.06 (m, 2H), 7.21 (d, J = 8.0, 1H), 7.31 (t, J = 7.5, 1H), 7.40-7.43 (m, 2H), 7.57-7.61 (m, 4H) .

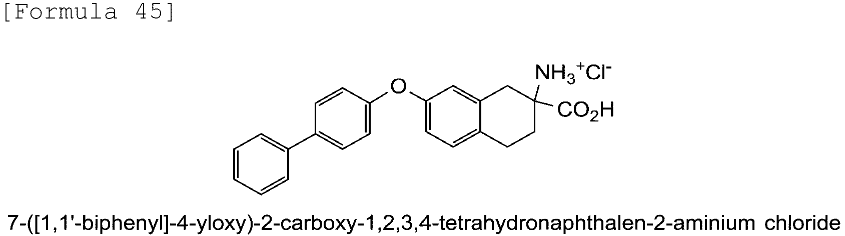

### (Example 1-13)

### Production of 2-carboxy-7-(3-(naphthalene-2-yl)phenoxy)-1,2,3,4-tetrahydronaphthalene-2-aminium chloride (Compound 1-32)

### Step 1 (Step I-IV)

### Production of methyl 2-((tert-butoxycarbonyl)amino)-7-(3-(naphthalene-2-yl)phenoxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 2-(3-iodophenyl) naphthalene.

¹H-NMR (CDCl₃); 1.42 (s, 9H), 2.11-2.16 (m, 1H), 2.48 (brs, 1H), 2.81-2.84 (m, 2H), 2.92 (d, J = 16.5 Hz, 1H), 3.28 (d, J = 16.5 Hz, 1H), 3.76 (s, 3H), 4.81 (brs, 1H), 6.78 (d, J = 2.5 Hz, 1H), 6.89 (dd, J = 2.5, 8.5 Hz, 1H), 6.98-7.01 (m, 1H), 7.10 (d, J = 8.0 Hz, 1H), 7.38 (t, J = 1.5 Hz, 1H), 7.41-7.51 (m, 4H), 7.71 (dd, J = 1.5, 8.5 Hz, 1H), 7.85-7.91 (m, 3H), 8.02 (s, 1H).

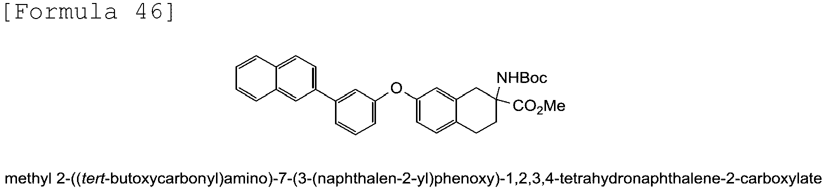

### Step 2 (Step V-VI)

### Production of 2-carboxy-7-(3-(naphthalene-2-yl)phenoxy)-1,2,3,4-tetrahydronaphthalene-2-aminium chloride

The compound obtained in Step 1 was treated in the same manner as in Steps 5 and 6 in Example 1-1 to synthesize the following compound.

¹H-NMR (CD₃OD); 2.15-2.20 (m, 1H), 2.38-2.44 (m, 1H), 2.86-2.93 (m, 1H), 3.00 (d, J = 16.5 Hz, 1H), 3.00-3.06 (m, 1H), 3.49 (d, J = 17.0 Hz, 1H), 6.89 (d, J = 2.0 Hz, 1H), 6.95 (dd, J = 2.5, 8.0 Hz, 1H), 6.98-7.00 (m, 1H), 7.22 (d, J = 8.0, 1H), 7.33 (m, 1H), 7.44-7.51 (m, 4H), 7.70 (dd, J = 1.5, 8.5 Hz, 1H), 7.85-7.92 (m, 3H), 8.03 (s, 1H).

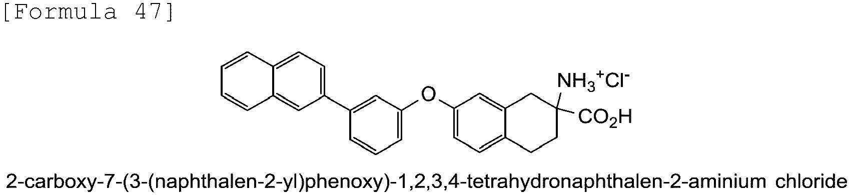

### (Example 1-14.)

### 2- Preparation of carboxy-7-((4'-fluoro-[1,1'-biphenyl]-3-yl)oxy)-1,2,3,4-tetrahydronaphthalene-2-aminium chloride (Compound 1-40)

### Step 1 (Steps I-IV)

### Preparation of methyl 2-((tert-butoxycarbonyl)amino)-7-((4'-fluoro-[1,1'-biphenyl]-3-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 4'-fluoro-3-iodo-1,1'-biphenyl.

1H-NMR (CDCl₃); 1.42 (s, 9H), 2.09-2.15 (m, 1H), 2.48 (brs, 1H), 2.81-2.84 (m, 2H), 2.92 (d, J = 17.0 Hz, 1H), 3.28 (d, J = 17.5 Hz, 1H), 3.76 (s, 3H), 4.81 (brs, 1H), 6.75 (d, J = 2.5 Hz, 1H), 6.87 (dd, J = 2.0, 8.0 Hz, 1H), 6.96 (dd, J = 2.0, 8.0 Hz, 1H), 7.08-7.14 (m, 3H), 7.18 (t, J = 1.5 Hz, 1H), 7.28 (t, J = 1.5 Hz, 1H), 7.38 (t, J = 8.0 Hz, 1H), 7.49-7.53 (m, 2H).

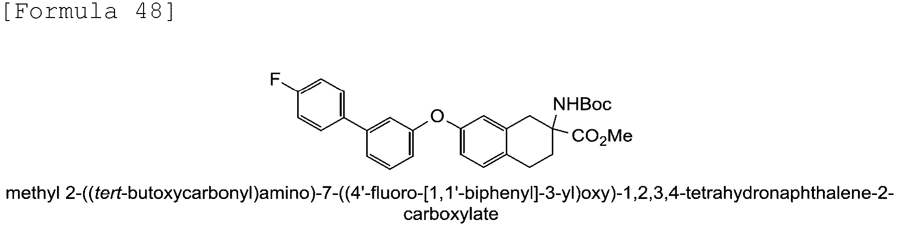

### Step 2 (Steps V-VI)

2- Production of carboxy-7-((4'-fluoro-[1,1'-biphenyl]-3-yl)oxy)-1,2,3,4-tetrahydronaphthalene-2-aminium chloride The following compound was synthesized from the compound obtained in Step 1 in the same manner as in Steps 5 and 6 of Example 1-1.

1H-NMR (CD3OD); 2.16-2.19 (m, 1H), 2.39-2.45 (m, 1H), 2.86-2.93 (m, 1H), 3.00 (d, J = 16.5 Hz, 1H), 3.02-3.05 (m, 1H), 3.49 (d, J = 17.0 Hz, 1H), 6.86 (d, J = 2.0 Hz, 1H), 6.91-6.95 (m, 2H), 7.13-7.22 (m, 4H), 7.32-7.34 (m, 1H), 7.41 (t, J = 7.5 Hz, 1H), 7.55-7.58 (m, 2H).

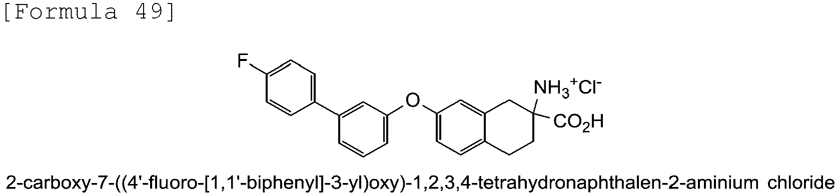

### (Example 1-15)

2- Preparation of Carboxy-7-((3'-(trifluoromethyl)-[1,1'-biphenyl]-3-yl)oxy)-1,2,3,4-tetrahydronaphthalene-2-aminium chloride (Compound 1-37).

### Step 1 (Steps I-IV)

### Preparation of methyl 2-((tert-butoxycarbonyl)amino)-7-((3'-(trifluoromethyl)-[1,1'-biphenyl]-3-yl)oxy)-1,2,3,4-tetrahydronaphthalene-2-carboxylate

The following compound was obtained in the same manner as in Step 1 to Step 4 of Example 1-1, except that 2-iodonaphthalene in Step 4 of Example was changed to 3-iodo-3'-(trifluoromethyl)-1,1'-biphenyl.

1H-NMR (CDCl₃); 1.42 (s, 9H), 2.09-2.16 (m, 1H), 2.47 (brs, 1H), 2.82-2.84 (m, 2H), 2.92 (d, J = 16.5 Hz, 1H), 3.29 (d, J = 16.5 Hz, 1H), 3.76 (s, 3H), 4.82 (brs, 1H), 6.75 (d, J = 2.0 Hz, 1H), 6.87 (dd, J = 2.5, 9.0 Hz, 1H), 7.01 (dd, J = 2.0, 8.0 Hz, 1H), 7.10 (d, J = 8.5 Hz, 1H), 7.24 (t, J = 1.5 Hz, 1H), 7.32-7.34 (m, 1H), 7.42 (t, J = 8.5 Hz, 1H), 7.55 (t, J = 7.5 Hz, 1H), 7.61 (d, J = 7.5 Hz, 1H), 7.73 (d, J = 8.0 Hz, 1H), 7.80 (s, 1H).

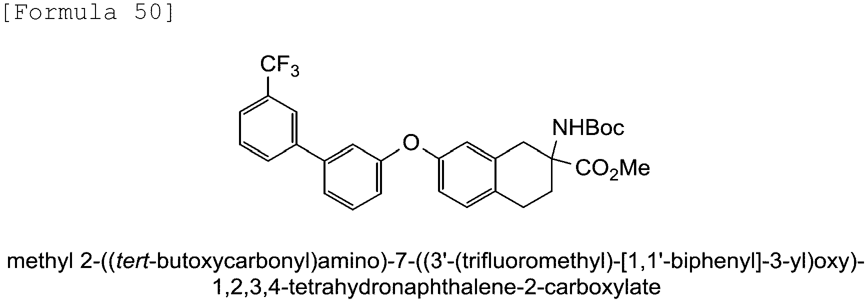

### Step 2 (Steps V-VI)

### 2- Production of carboxy-7-((3'-(trifluoromethyl)-[1,1'-biphenyl]-3-yl)oxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.15-2.20 (m, 1H), 2.39-2.45 (m, 1H), 2.86-2.93 (m, 1H), 3.00 (d, J = 18.5 Hz, 1H), 3.03-3.07 (m, 1H), 3.50 (d, J = 18.0 Hz, 1H), 6.88 (d, J = 2.5 Hz, 1H), 6.94 (dd, J = 3.0, 9.0 Hz, 1H), 7.01 (dd, J = 1.5, 7. 0 Hz, 1H), 7.22-7.24 (m, 2H), 7.40 (dd, J = 2.0, 7.0 Hz, 1H), 7.46 (t, J = 7.5 Hz, 1H), 7.62-7.67 (m, 2H), 7.82-7.84 (m, 2H).

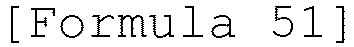

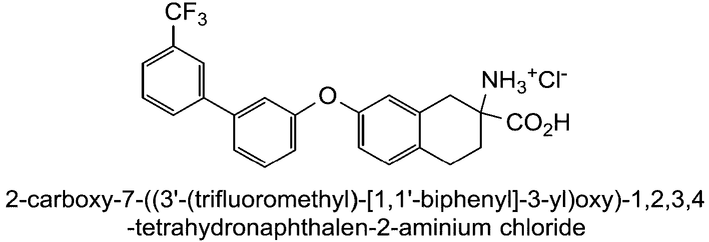

### (Example 1-16)

### Preparation of 7-([1,1'-biphenyl]-2-yloxy)-2-carboxy-1,2,3,4-tetrahydronaphthalene-2-aminium chloride (Compound 1-34)

### Step 1 (Steps I-IV)

### Preparation of methyl 7-([1,1'-biphenyl]-2-yloxy)-2-((tert-butoxycarbonyl)amino)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-iodo-1,1'-biphenyl, and the reaction temperature was further changed from 90°C. to 120°C.

1H-NMR (CDCl3); 1.42 (s, 9H), 2.05-2.11 (m, 1H), 2.46 (brs, 1H), 2.76-2.78 (m, 2H), 2.84 (d, J = 17.0 Hz, 1H), 3.21 (d, J = 17.0 Hz, 1H), 3.75 (s, 3H), 4.76 (brs, 1H), 6.62 (d, J = 2.5 Hz, 1H), 6.75 (dd, J = 2.5, 8.0 Hz, 1H), 6.98-7.02 (m, 2H), 7.18-7.22 (m, 1H), 7.27-7.31 (m, 2H), 7.35-7.38 (m, 2H), 7.45 (dd, J = 1.5, 7.5 Hz, 1H), 7.52-7.54 (m, 2H).

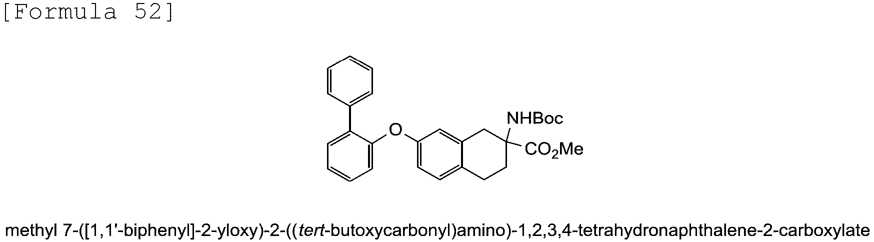

### Step 2 (Steps V-VI)

Production of 7-([1,1'-biphenyl]-2-yloxy)-2-carboxy-1,2,3,4-tetrahydronaphthalene-2-aminium chloride The following compound was synthesized from the compound obtained in Step 1 in the same manner as in Steps 5 and 6 of Example 1-1.

1H-NMR (CD3OD); 2.10-2.15 (m, 1H), 2.35-2.39 (m, 1H), 2.78-2.84 (m, 1H), 2.91 (d, J = 17.2 Hz, 1H), 2.93-2.99 (m, 1H), 3.41 (d, J = 17.2 Hz, 1H), 6.65 (d, J = 2.4 Hz, 1H), 6.72 (dd, J = 2.4, 8.2 Hz, 1H), 7.00 (d, J = 7.9 Hz, 1H), 7.09 (d, J = 8.3 Hz, 1H), 7.24-7.27 (m, 2H), 7.30-7.35 (m, 3H), 7.45-7.50 (m, 3H).

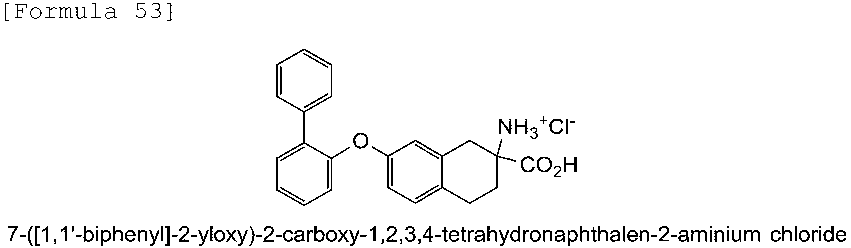

### (Example 1-17.)

2- Preparation of carboxy-7-(phenanthren-3-yloxy)-1,2,3,4-tetrahydronaphthalene-2-aminium chloride (Compound 1-35) .

### Step 1 (Steps I-IV)

### Preparation of methyl 2-((tert-butoxycarbonyl)amino)-7-(phenanthren-3-yloxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 3-iodophenanthrene.

1H-NMR (CDCl3); 1.43 (s, 9H), 2.11-2.18 (m, 1H), 2.50 (brs, 1H), 2.84-2.86 (m, 2H), 2.91 (d, J = 16.5 Hz, 1H), 3.28 (d, J = 17.0 Hz, 1H), 3.76 (s, 3H), 4.83 (brs, 1H), 6.80 (d, J = 3.0 Hz, 1H), 6.91 (dd, J = 2.5, 8.5 Hz, 1H), 7.11 (d, J = 8.0 Hz, 1H), 7.30 (dd, J = 2.5, 9.0 Hz, 1H), 7.58-7.63 (m, 2H), 7.68 (d, J = 9.0 Hz, 1H), 7.72 (d, J = 9.0 Hz, 1H), 7.86-7.89 (m, 2H), 8.25 (d, J = 2.5 Hz, 1H), 8.49-8.51 (m, 1H) .

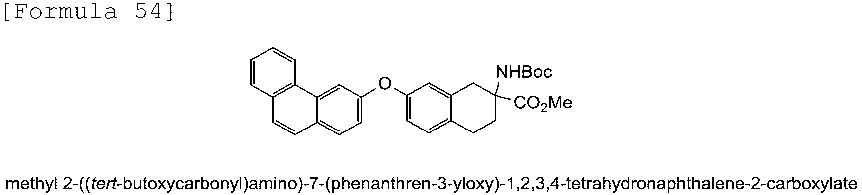

### Step 2 (Steps V-VI)

### 2- Production of carboxy-7-(phenanthren-3-yloxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.16-2.22 (m, 1H), 2.39-2.46 (m, 1H), 2.89-2.95 (m, 1H), 3.00 (d, J = 18.0 Hz, 1H), 3.01-3.07 (m, 1H), 3.50 (d, J = 17.2 Hz, 1H), 6.92 (d, J = 2.6 Hz, 1H), 6.97-6.99 (m, 1H), 7.25 (d, J = 8.4 Hz, 1H), 7.29 (dd, J = 2.4, 8.4 Hz, 1H), 7.58-7.60 (m, 2H), 7.69-7.75 (m, 2H), 7.88-7.92 (m, 2H), 8.24 (m, 1H), 8.47-8.49 (m, 1H).

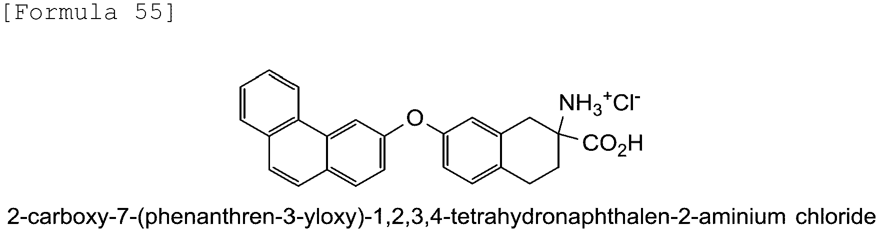

### (Example 1-18)

### 2- Preparation of amino-7-((7-dimethylamino)naphthalen-1-yl)oxy)-1,2,3,4-tetrahydronaphthalene-2-carboxylic acid (Compound 1-36).

### Step 1 (Steps I-IV)

### Preparation of methyl 2-((tert-butoxycarbonyl)amino)-7-((7-dimethylamino)naphthalen-1-yl)oxy)-1,2,3,4-tetrahydronaphthalene-2-carboxylate

The following compound was obtained in the same manner as in Step 1 to Step 4 of Example 1-1, except that 2-iodonaphthalene in Step 4 of Example was changed to 8-iodo-N,N-dimethylnaphthalen-2-amine.

1H-NMR (CDCl3); 1.43 (s, 9H), 2.09-2.15 (m, 1H), 2.49 (brs, 1H), 2.81-2.83 (m, 2H), 2.87 (d, J = 18.0 Hz, 1H), 3.03 (s, 6H), 3.24 (d, J = 16.5 Hz, 1H), 3.76 (s, 3H), 4.80 (brs, 1H), 6.75 (d, J = 2.0 Hz, 1H), 6.85-6.89 (m, 2H), 7.07 (d, J = 8.5 Hz, 1H), 7.10 (t, J = 8.0 Hz, 1H), 7.19-7.21 (m, 2H), 7.48 (d, J = 8.5 Hz, 1H), 7.74 (d, J = 10.0 Hz, 1H).

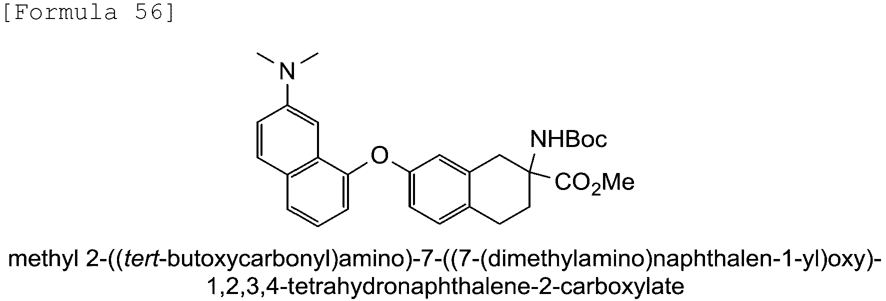

### Step 2 (Process V)

### Preparation of 2-((tert-butoxycarbonyl)amino)-7-((7-dimethylamino)naphthalen-1-yl)oxy)-1,2,3,4-tetrahydronaphthalene-2-carboxylic acid

The following compound was synthesized from the above compound in the same manner as in Step 2 of Example 1-6. In addition, this carboxylic acid compound was directly used in the next step without purification.

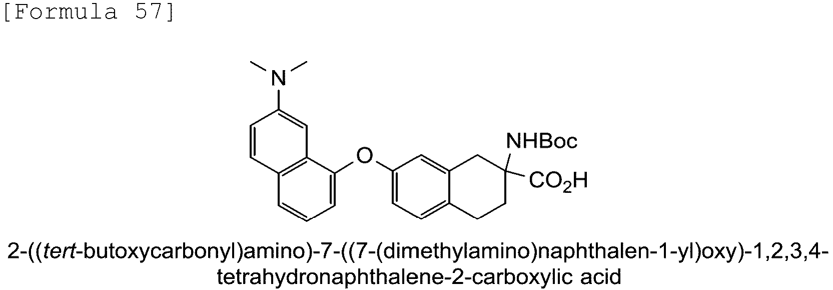

### Step 3

2- Preparation of amino-7-((7-dimethylamino)naphthalen-1-yl)oxy)-1,2,3,4-tetrahydronaphthalene-2-carboxylic acid The following compound was synthesized from the above compound in the same manner as in Step 3 of Example 1-6. 1H-NMR (DMSO-d6); 1.82-1.86 (m, 1H), 2.04-2.10 (m, 1H), 2.70 (d, J = 18.0 Hz, 1H), 2.70-2.80 (m, 2H), 2.97 (s, 6H), 3.27 (d, J = 17.0 Hz, 1H), 6.76-6.78 (m, 2H), 6.81 (d, J = 7.5 Hz, 1H), 7.03 (d, J = 2.5 Hz, 1H), 7.07 (d, J = 8.0 Hz, 1H), 7.10 (t, J = 8.0 Hz, 1H), 7.28 (dd, J = 3.0, 9.5 Hz, 1H), 7.51 (d, J = 8.5 Hz, 1H), 7.79 (d, J = 9.0 Hz, 1H).

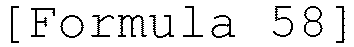

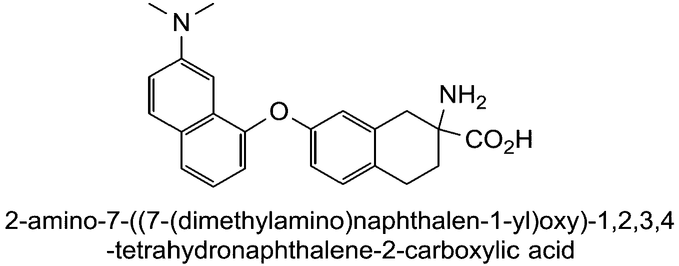

### (Example 1-19)

### Preparation of 7-(anthracen-2-yloxy)-2-carboxy-1,2,3,4-tetrahydronaphthalene-2-aminium chloride (Compound 1-42) Step 1 (Steps I-IV)

### Preparation of methyl 7-(anthracen-2-yloxy)-2-((tert-butoxycarbonyl)amino)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-iodoanthracene.

1H-NMR (CDCl3); 1.44 (s, 9H), 2.12-2.18 (m, 1H), 2.51 (brs, 1H), 2.85-2.87 (m, 2H), 2.93 (d, J = 16.5 Hz, 1H), 3.30 (d, J = 16.5 Hz, 1H), 3.77 (s, 3H), 4.84 (brs, 1H), 6.83 (d, J = 1.5 Hz, 1H), 6.94 (dd, J = 2.5, 8.0 Hz, 1H), 7.14 (d, J = 8.5 Hz, 1H), 7.27 (dd, J = 2.5, 9.5 Hz, 1H), 7.34 (s, 1H), 7.41-7.46 (m, 2H), 7.92 (d, J = 7.5 Hz, 1H), 7.97-8.00 (m, 2H), 8.25 (s, 1H), 8.40 (s, 1H).

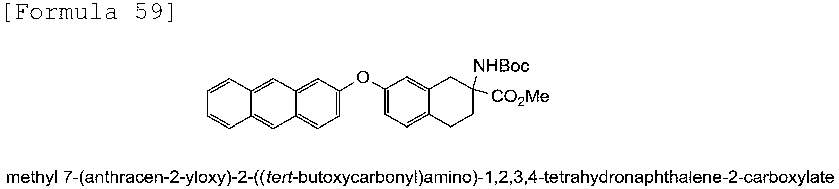

### Step 2 (Steps V-VI)

### Preparation of 7-(anthracen-2-yloxy)-2-carboxy-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.17-2.22 (m, 1H), 2.41-2.47 (m, 1H), 2.89-2.96 (m, 1H), 3.02 (d, J = 17.0 Hz, 1H), 3.04-3.09 (m, 1H), 3.51 (d, J = 17.0 Hz, 1H), 6.94 (d, J = 2.5 Hz, 1H), 7.00 (dd, J = 2.0, 8.5 Hz, 1H), 7.24-7.26 (m, 2H), 7.35 (d, J = 1.5 Hz, 1H), 7.41-7.46 (m, 2H), 7.93 (d, J = 8.0 Hz, 1H), 8.00 (d, J = 7.5 Hz, 1H), 8.05 (d, J = 9.0 Hz, 1H), 8.23 (s, 1H), 8.44 (s, 1H).

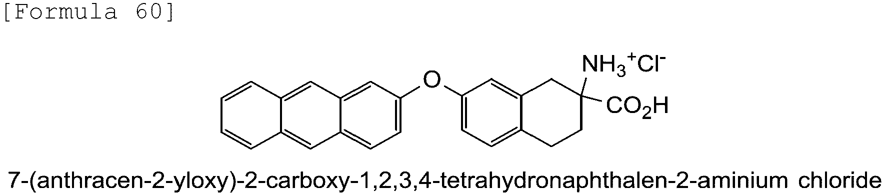

### (Example 1-20)

### 2- Preparation of amino-7-((4'-(dimethylamino)-[1,1'-biphenyl]-3-yl)oxy)-1,2,3,4-tetrahydronaphthalene-2-carboxylic acid (Compound 1-41)

### Step 1 (Steps I-IV)

### Preparation of methyl 2-((tert-butoxycarbonyl)amino)-7-((4'-(dimethylamino)-[1,1'-biphenyl]-3-yl)oxy)-1,2,3,4-tetrahydronaphthalene-2-carboxylate

The following compound was obtained in the same manner as in Step 1 to Step 4 of Example 1-1, except that 2-iodonaphthalene in Step 4 of Example was replaced with 3'-iodo-N,N-dimethyl-[1,1'-biphenyl]-4-amine.

1H-NMR (CDCl3); 1.42 (s, 9H), 2.09-2.15 (m, 1H), 2.49 (brs, 1H), 2.80-2.83 (m, 2H), 2.89 (d, J = 16.5 Hz, 1H), 2.99 (s, 6H), 3.25 (d, J = 17.0 Hz, 1H), 3.76 (s, 3H), 4.81 (brs, 1H), 6.75 (d, J = 2.5 Hz, 1H), 6.77-6.79 (m, 2H), 6.85-6.87 (m, 2H), 7.07 (d, J = 8.0 Hz, 1H), 7.20 (t, J = 2.5 Hz, 1H), 7.29-7.31 (m, 1H), 7.34 (t, J = 7.5 Hz, 1H), 7.46-7.49 (m, 2H).

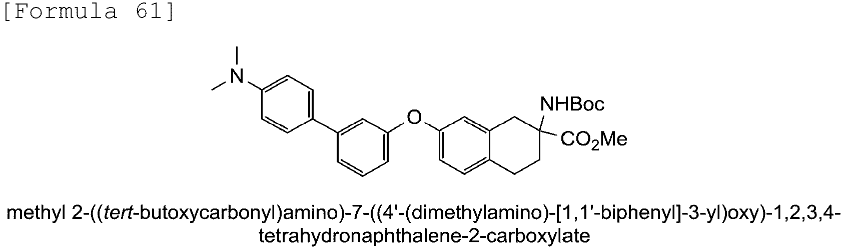

### Step 2 (Process V)

### Preparation of 2-((tert-butoxycarbonyl)amino)-7-((4'-(dimethylamino)-[1,1'-biphenyl]-3-yl)oxy)-1,2,3,4-tetrahydronaphthalene-2-carboxylic acid

The following compound was synthesized from the above compound in the same manner as in Step 2 of Example 1-6. In addition, this carboxylic acid compound was directly used in the next step without purification.

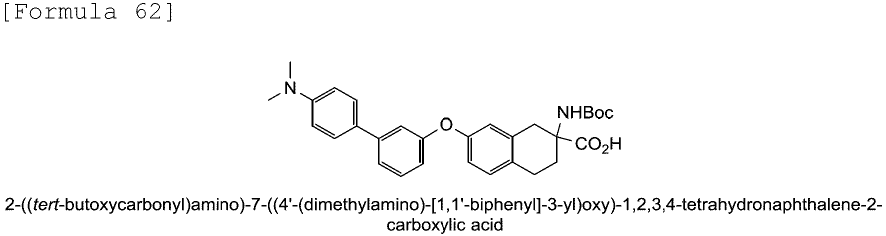

### Step 3

### 2- Preparation of amino-7-((4'-(dimethylamino)-[1,1'-biphenyl]-3-yl)oxy)-1,2,3,4-tetrahydronaphthalene-2-carboxylic acid

The following compound was synthesized from the above compound in the same manner as in Step 3 of Example 1-6. 1H-NMR (DMSO-d6); 1.79-1.82 (m, 1H), 2.02-2.08 (m, 1H), 2.68 (d, J = 18.0 Hz, 1H), 2.72-2.78 (m, 2H), 2.91 (s, 6H), 3.26 (d, J = 17.0 Hz, 1H), 6.75-6.79 (m, 5H), 7.08 (d, J = 8.0 Hz, 1H), 7.16 (s, 1H), 7.30-7.36 (m, 2H), 7.46 (d, J = 8.5 Hz, 2H).

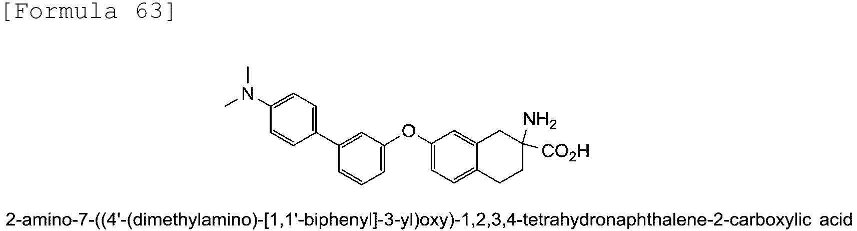

### (Example 1-21)

### 2- Preparation of carboxy-7-((6-fluoronaphthalen-2-yl)oxy)-1,2,3,4-tetrahydronaphthalene-2-aminium chloride (Compound 1-7).

### Step 1 (Steps I-IV)

### Preparation of methyl 2-((tert-butoxycarbonyl)amino)-7-((6-fluoronaphthalen-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2-bromo-6-fluoronaphthalene.

1H-NMR (CDCl3); 1.43 (s, 9H), 2.10-2.17 (m, 1H), 2.49 (brs, 1H), 2.83-2.85 (m, 2H), 2.91 (d, J = 17.0 Hz, 1H), 3.29 (d, J = 17.0 Hz, 1H), 3.76 (s, 3H), 4.82 (brs, 1H), 6.76 (d, J = 2.0 Hz, 1H), 6.87 (dd, J = 2.5, 8.5 Hz, 1H), 7.11 (d, J = 8.5 Hz, 1H), 7.23 (dd, J = 2.0, 8.5 Hz, 1H), 7.26-7.29 (m, 2H), 7.44 (dd, J = 3.0, 10.0 Hz, 1H), 7.68 (dd, J = 6.0, 9.5 Hz, 1H), 7.76 (d, J = 8.5 Hz, 1H).

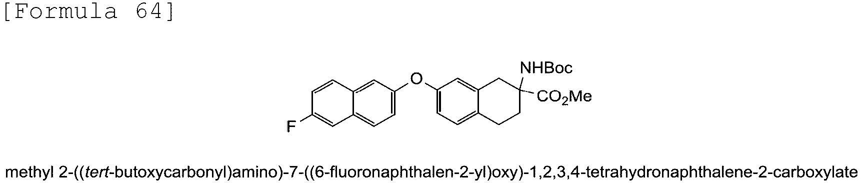

### Step 2 (Steps V-VI)

### 2- Production of carboxy-7-((6-fluoronaphthalen-2-yl)oxy)-1,2,3,4-tetrahydronaphthalene-2-aminium chloride The following compound was synthesized from the compound obtained in Step 1 in the same manner as in Steps 5 and 6 of Example 1-1.

1H-NMR (CD3OD); 2.16-2.21 (m, 1H), 2.40-2.46 (m, 1H), 2.88-2.94 (m, 1H), 3.00 (d, J = 17.0 Hz, 1H), 3.03-3.07 (m, 1H), 3.49 (d, J = 17.0 Hz, 1H), 6.87 (d, J = 2.5 Hz, 1H), 6.93 (dd, J = 2.0, 8.0 Hz, 1H), 7.23 (d, J = 9.0 Hz, 1H), 7.26-7.31 (m, 3H), 7.52 (dd, J = 3.0, 10.5 Hz, 1H), 7.74 (dd, J = 6.0, 9.5 Hz, 1H), 7.85 (d, J = 9.5 Hz, 1H).

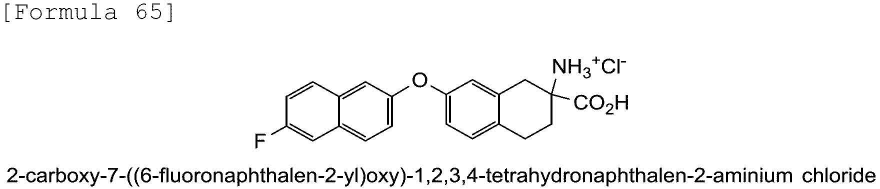

### (Example 1-22)

### 2- Preparation of carboxy-7-((8-chloronaphthalen-2-yl)oxy)-1,2,3,4-tetrahydronaphthalene-2-aminium chloride (Compound 1-44).

### Step 1 (Steps I-IV)

### Preparation of methyl 2-((tert-butoxycarbonyl)amino)-7-((8-chloronaphthalen-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was replaced with 7-bromo-1-chloronaphthalene.

1H-NMR (CDCl3); 1.43 (s, 9H), 2.11-2.18 (m, 1H), 2.48 (brs, 1H), 2.84-2.86 (m, 2H), 2.93 (d, J = 16.5 Hz, 1H), 3.29 (d, J = 17.0 Hz, 1H), 3.77 (s, 3H), 4.83 (brs, 1H), 6.79 (d, J = 2.5 Hz, 1H), 6.89 (dd, J = 2.0, 8.0 Hz, 1H), 7.12 (d, J = 8.5 Hz, 1H), 7.28 (dd, J = 2.0, 8.5 Hz, 1H), 7.31 (t, J = 7.5 Hz, 1H), 7.55-7.56 (m, 1H), 7.73-7.75 (m, 2H), 7.84 (d, J = 9.5 Hz, 1H).

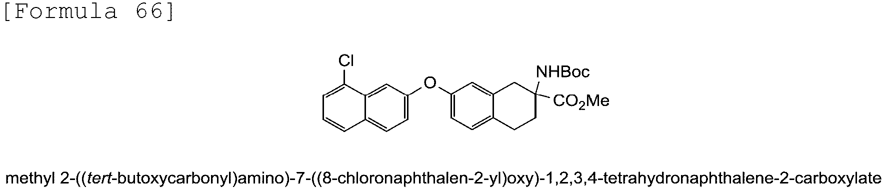

### Step 2 (Steps V-VI)

### 2- Production of carboxy-7-((8-chloronaphthalen-2-yl)oxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.18-2.23 (m, 1H), 2.41-2.47 (m, 1H), 2.90-2.96 (m, 1H), 3.03 (d, J = 18.0 Hz, 1H), 3.04-3.10 (m, 1H), 3.52 (d, J = 18.0 Hz, 1H), 6.94 (d, J = 1.5 Hz, 1H), 6.99 (dd, J = 1.5, 8.0 Hz, 1H), 7.27 (d, J = 9.0 Hz, 1H), 7.32 (dd, J = 3.0, 9.5 Hz, 1H), 7.35 (d, J = 8.0 Hz, 1H), 7.57 (d, J = 7.5 Hz, 1H), 7.61 (d, J = 2.5 Hz, 1H), 7.82 (d, J = 8.0 Hz, 1H), 7.93 (d, J = 9.0 Hz, 1H).

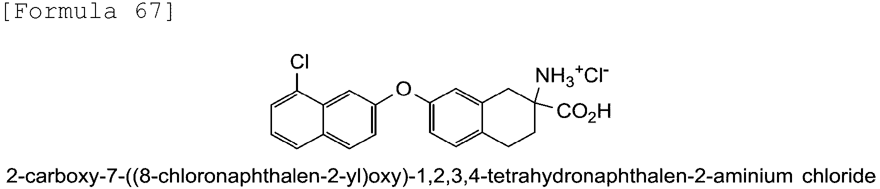

### (Example 1-23)

### 2- Preparation of carboxy-7-((4'-methyl-[1,1'-biphenyl]-3-yl)oxy)-1,2,3,4-tetrahydronaphthalene-2-aminium chloride (Compound 1-45)

### Step 1 (Steps I-IV)

### Preparation of methyl 2-((tert-butoxycarbonyl)amino)-7-((4'-methyl-[1,1'-biphenyl]-3-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 3-iodo-4'-methyl-1,1'-biphenyl.

1H-NMR (CDCl3); 1.42 (s, 9H), 2.09-2.15 (m, 1H), 2.39 (s, 3H), 2.50 (brs, 1H), 2.82-2.83 (m, 2H), 2.90 (d, J = 16.0 Hz, 1H), 3.27 (d, J = 17.0 Hz, 1H), 3.76 (s, 3H), 4.81 (brs, 1H), 6.75 (d, J = 2.5 Hz, 1H), 6.86 (dd, J = 2.0, 8.0 Hz, 1H), 6.93-6.95 (m, 1H), 7.08 (d, J = 8.5 Hz, 1H), 7.22-7.24 (m, 3H), 7.30-7.32 (m, 1H), 7.37 (t, J = 8.0 Hz, 1H), 7.45-7.47 (m, 2H).

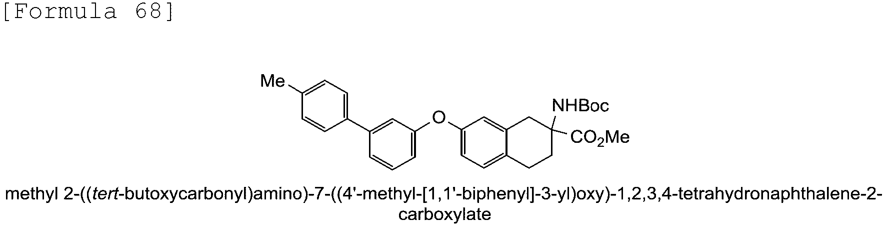

### Step 2 (Steps V-VI)

### 2- Production of carboxy-7-((4'-methyl-[1,1'-biphenyl]-3-yl)oxy)-1,2,3,4-tetrahydronaphthalene-2-aminium chloride The following compound was synthesized from the compound obtained in Step 1 in the same manner as in Steps 5 and 6 of Example 1-1.

1H-NMR (CD3OD); 2.15-2.20 (m, 1H), 2.35 (s, 3H), 2.38-2.44 (m, 1H), 2.86-2.92 (m, 1H), 2.99 (d, J = 18.0 Hz, 1H), 3.00-3.06 (m, 1H), 3.49 (d, J = 17.0 Hz, 1H), 6.85 (d, J = 2.0 Hz, 1H), 6.91-6.93 (m, 2H), 7.16 (m, 1H), 7.20-7.23 (m, 3H), 7.33 (d, J = 8.0 Hz, 1H), 7.39 (t, J = 8.0 Hz, 1H), 7.43 (d, J = 8.0 Hz, 2H).

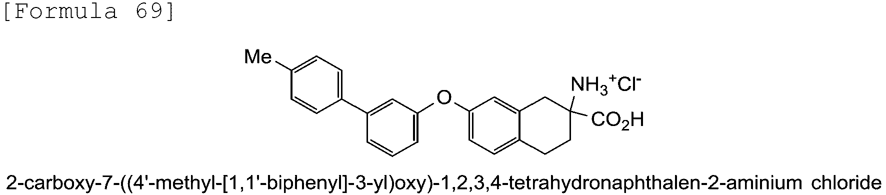

### (Example 1-24)

### 2- Preparation of carboxy-7-((1,2-dihydroacenaphthylen-5-yl)oxy)-1,2,3,4-tetrahydronaphthalene-2-aminium chloride (Compound 1-47).

### Step 1 (Steps I-IV)

### Preparation of methyl 2-((tert-butoxycarbonyl)amino)-7-((1,2-dihydroacenaphthylen-5-yl)oxy)-1,2,3,4-tetrahydronaphthalene-2-carboxylate

The following compound was obtained in the same manner as in Step 1 to Step 4 of Example 1-1, except that 2-iodonaphthalene in Step 4 of Example was changed to 5-iodo-1,2-dihydroacenaphthylene.

1H-NMR (CDCl3); 1.42 (s, 9H), 2.07-2.13 (m, 1H), 2.48 (brs, 1H), 2.79-2.81 (m, 2H), 2.84 (d, J = 17.5 Hz, 1H), 3.22 (d, J = 17.0 Hz, 1H), 3.37-3.39 (m, 2H), 3.44-3.47 (m, 2H), 3.75 (s, 3H), 4.79 (brs, 1H), 6.68 (d, J = 2.0 Hz, 1H), 6.81 (dd, J = 2.5, 8.0 Hz, 1H), 6.98 (d, J = 7.5 Hz, 1H), 7.04 (d, J = 8.5 Hz, 1H), 7.18 (d, J = 7.5 Hz, 1H), 7.30 (d, J = , 7.0 Hz, 1H), 7.41 (dd, J = 7.0, 8.0 Hz, 1H), 7.67 (m, J = 8.0 Hz, 1H).

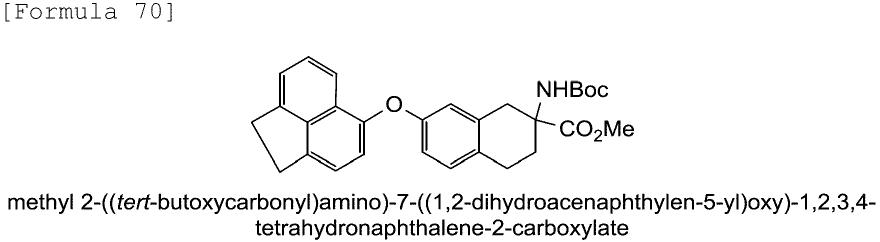

### Step 2 (Steps V-VI)

### 2- Production of carboxy-7-((1,2-dihydroacenaphthylen-5-yl)oxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.13-2.18 (m, 1H), 2.36-2.42 (m, 1H), 2.83-3.03 (m, 3H), 3.35-3.37 (m, 2H), 3.41-3.44 (m, 2H), 6.74 (d, J = 2.6 Hz, 1H), 6.84 (dd, J = 2.7, 8.5 Hz, 1H), 6.97 (d, J = 7.4 Hz, 1H), 7.14 (d, J = 8.5 Hz, 1H), 7.20 (d, J = 7.6 Hz, 1H), 7.29 (d, J = 6.8 Hz, 1H), 7.35-7.39 (m, 1H), 7.53 (d, J = 8.4 Hz, 1H).

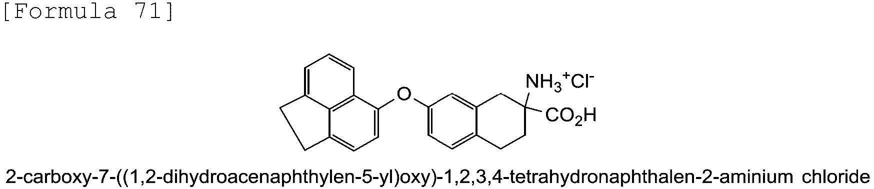

### (Example 1-25)

### 2- Preparation of carboxy-7-((5,6,7,8-tetrahydronaphthalen-2-yl)oxy)-1,2,3,4-tetrahydronaphthalene-2-aminium chloride (Compound 1-48) Step 1 (Steps I-IV)

### Preparation of methyl 2-((tert-butoxycarbonyl)amino)-7-((5,6,7,8-tetrahydronaphthalen-2-yl)oxy)-1,2,3,4-tetrahydronaphthalene-2-carboxylate

The following compound was obtained in the same manner as in Step 1 to Step 4 of Example 1-1, except that 2-iodonaphthalene in Step 4 of Example was changed to 5-iodo-1,2,3,4-tetrahydronaphthalene.

1H-NMR (CDCl3); 1.42 (s, 9H), 1.76-1.82 (m, 4H), 2.08-2.14 (m, 1H), 2.48 (brs, 1H), 2.72-2.75 (m, 4H), 2.79-2.82 (m, 2H), 2.88 (d, J = 17.0 Hz, 1H), 3.24 (d, J = 16.5 Hz, 1H), 3.76 (s, 3H), 4.81 (brs, 1H), 6.67 (d, J = 2.0 Hz, 1H), 6.70 (d, J = 2.5 Hz, 1H), 6.74 (dd, J = 3.0, 8.5 Hz, 1H), 6.80 (dd, J = 3.0, 8.5 Hz, 1H), 7.01 (d, J = 8.5 Hz, 1H), 7.05 (d, J = 8.0 Hz, 1H).

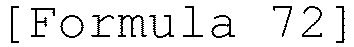

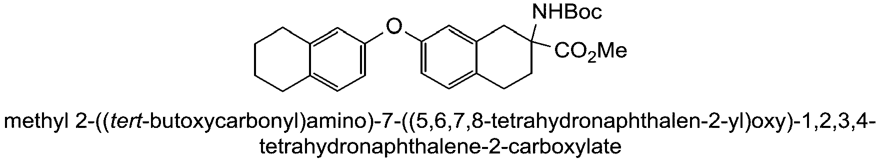

### Step 2 (Steps V-VI)

### 2- Production of carboxy-7-((5,6,7,8-tetrahydronaphthalen-2-yl)oxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 1.76-1.81 (m, 4H), 2.14-2.19 (m, 1H), 2.37-2.43 (m, 1H), 2.70-2.73 (m, 4H), 2.83-2.90 (m, 1H), 2.97 (d, J = 17.5 Hz, 1H), 3.00-3.03 (m, 1H), 3.46 (d, J = 17.0 Hz, 1H), 6.64 (d, J = 2.5 Hz, 1H), 6.68 (dd, J = 2.0, 8.0 Hz, 1H), 6.74 (d, J = 2.0 Hz, 1H), 6.81 (dd, J = 2.5, 8.5 Hz, 1H), 7.00 (d, J = 8.5 Hz, 1H), 7.15 (d, J = 8.5 Hz, 1H).

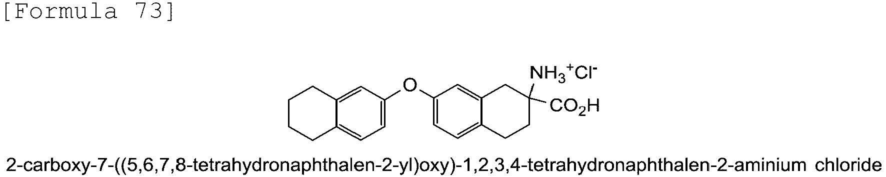

### (Example 1-26)

### 2- Preparation of Carboxy-7-(2-(naphthalen-2-yl)phenoxy)-1,2,3,4-tetrahydronaphthalene-2-aminium chloride (Compound 1-49)

### Step 1 (Steps I-IV)

### Preparation of methyl 2-((tert-butoxycarbonyl)amino)-7-(2-(naphthalen-2-yl)phenoxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-(2-iodophenyl)naphthalene, and the reaction temperature was further changed from 90°C. to 120°C.

1H-NMR (CDCl3); 1.41 (s, 9H), 2.04-2.10 (m, 1H), 2.44 (brs, 1H), 2.74-2.77 (m, 2H), 2.83 (d, J = 16.5 Hz, 1H), 3.19 (d, J = 16.5 Hz, 1H), 3.74 (s, 3H), 4.75 (brs, 1H), 6.64 (d, J = 2.0 Hz, 1H), 6.77 (dd, J = 2.5, 8.0 Hz, 1H), 6.99-7.04 (m, 2H), 7.24 (dd, J = 1.5, 7.5 Hz, 1H), 7.33 (td, J = 1.5, 8.0 Hz, 1H), 7.44-7.48 (m, 2H), 7.56 (dd, J = 1.5, 7.5 Hz, 1H), 7.70 (dd, J = 1.5, 8.0 Hz, 1H), 7.81-7.86 (m, 3H), 7.99 (d, J = 1.5 Hz, 1H).

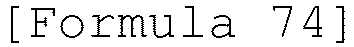

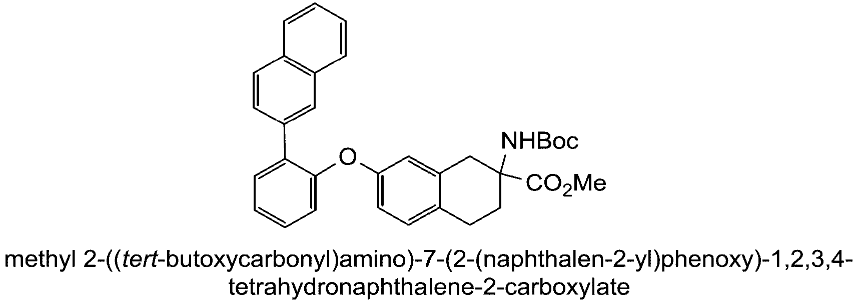

### Step 2 (Process V)

### Preparation of 2-((tert-butoxycarbonyl)amino)-7-(2-(naphthalen-2-yl)phenoxy)-1,2,3,4-tetrahydronaphthalene-2-carboxylic acid

The following compound was synthesized from the above compound in the same manner as in Step 5 of Example 1-1. In addition, this carboxylic acid compound was directly used in the next step without purification.

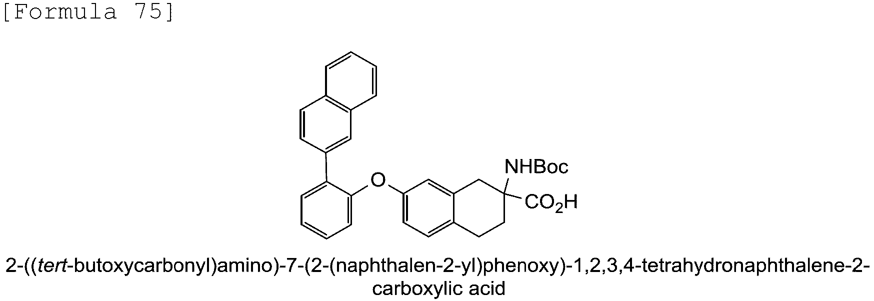

### Step 3 (Process VI)

To the carboxylic acid compound (180 mg, 0.353 mmol) obtained above was added 3M HCl-CPME (5 mL). This was reacted at 60°C. for 2 hours. After completion of the reaction, the solvent was distilled off, and the obtained residue was purified by silica gel column chromatography to obtain the target product (110 mg, 70%).

1H-NMR (CD3OD); 1.97-2.01 (m, 1H), 2.24-2.31 (m, 1H), 2.67-2.73 (m, 1H), 2.76 (d, J = 17.5 Hz, 1H), 2.84-2.90 (m, 1H), 3.49 (d, J = 17.5 Hz, 1H), 6.63 (d, J = 2.0 Hz, 1H), 6.69 (dd, J = 2.0, 8.5 Hz, 1H), 7.00-7.06 (m, 2H), 7.30 (td, J = 1.5, 7.5 Hz, 1H), 7.37 (td, J = 2.0, 8.0 Hz, 1H), 7.43-7.46 (m, 2H), 7.59 (dd, J = 1.0, 7.5 Hz, 1H), 7.67 (dd, J = 2.0, 8.5 Hz, 1H), 7.77-7.83 (m, 3H), 7.97 (s, 1H).

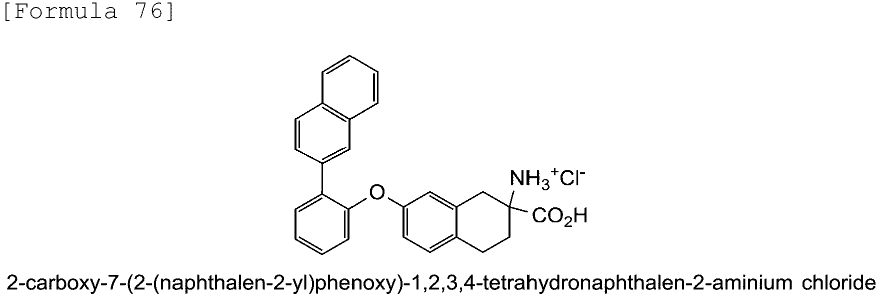

### (Example 1-27)

### 2- Preparation of carboxy-7-((4'-fluoro-[1,1'-biphenyl]-2-yl)oxy)-1,2,3,4-tetrahydronaphthalene-2-aminium chloride (Compound 1-52)

### Step 1 (Steps I-IV)

### Preparation of methyl 2-((tert-butoxycarbonyl)amino)-7-((4'-fluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-4'-fluoro-1,1'-biphenyl, and the reaction temperature was further changed from 90°C. to 120°C.

1H-NMR (CDCl3); 1.42 (s, 9H), 2.05-2.12 (m, 1H), 2.44 (brs, 1H), 2.76-2.79 (m, 2H), 2.85 (d, J = 16.5 Hz, 1H), 3.22 (d, J = 17.0 Hz, 1H), 3.75 (s, 3H), 4.77 (brs, 1H), 6.61 (d, J = 2.5 Hz, 1H), 6.73 (dd, J = 3.0, 8.5 Hz, 1H), 6.97-7.07 (m, 4H), 7.19 (td, J = 1.0, 7.5 Hz, 1H), 7.29 (td, J = 1.5, 8.0 Hz, 1H), 7.41 (dd, J = 1.0, 7.5 Hz, 1H), 7.48-7.52 (m, 2H).

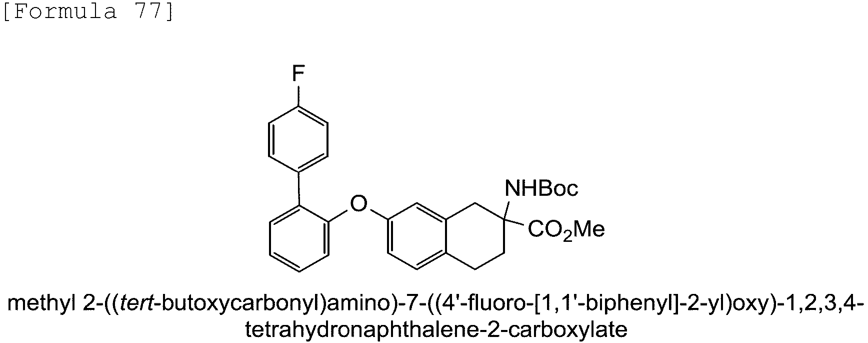

### Step 2 (Steps V-VI)

### 2- Production of carboxy-7-((4'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.02-2.05 (m, 1H), 2.29-2.35 (m, 1H), 2.73-2.79 (m, 1H), 2.82 (d, J = 17.0 Hz, 1H), 2.89-2.95 (m, 1H), 3.40 (d, J = 17.5 Hz, 1H), 6.62 (d, J = 2.5 Hz, 1H), 6.68 (dd, J = 3.0, 8.5 Hz, 1H), 6.98 (dd, J = 1.5, 8.0 Hz, 1H), 7.02-7.06 (m, 3H), 7.24 (td, J = 1.0, 7.5 Hz, 1H), 7.33 (td, J = 1.5, 8.0 Hz, 1H), 7.44 (dd, J = 2.0, 8.0 Hz, 1H), 7.50-7.54 (m, 2H).

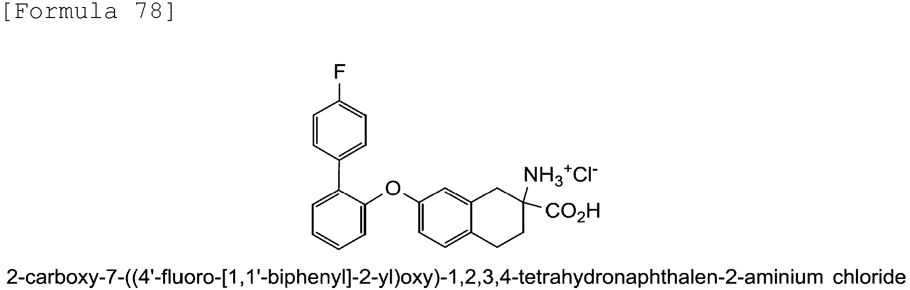

### (Example 1-28)

### 2- Preparation of carboxy-7-((4'-chloro-[1,1'-biphenyl]-2-yl)oxy)-1,2,3,4-tetrahydronaphthalene-2-aminium chloride (Compound 1-53)

### Step 1 (Steps I-IV)

### Preparation of methyl 2-((tert-butoxycarbonyl)amino)-7-((4'-chloro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-4'-chloro-1,1'-biphenyl, and the reaction temperature was further changed from 90°C. to 120°C.

1H-NMR (CDCl3); 1.43 (s, 9H), 2.06-2.12 (m, 1H), 2.45 (brs, 1H), 2.77-2.79 (m, 2H), 2.86 (d, J = 17.5 Hz, 1H), 3.23 (d, J = 17.0 Hz, 1H), 3.76 (s, 3H), 4.78 (brs, 1H), 6.61 (d, J = 2.0 Hz, 1H), 6.73 (dd, J = 3.0, 8.5 Hz, 1H), 6.98 (dd, J = 1.0, 8.0 Hz, 1H), 7.02 (d, J = 8.5 Hz, 1H), 7.20 (td, J = 2.0, 8.0 Hz, 1H), 7.30 (td, J = 1.5, 8.0 Hz, 1H), 7.31-7.35 (m, 2H), 7.41 (dd, J = 2.0, 7.5 Hz, 1H), 7.46-7.49 (m, 2H).

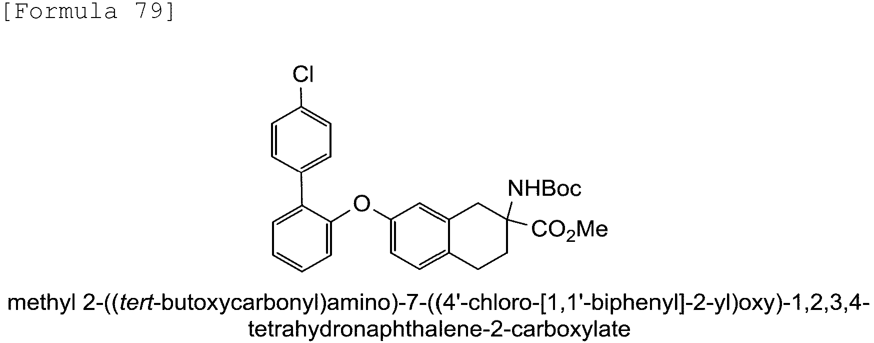

### Step 2 (Steps V-VI)

### 2- Production of carboxy-7-((4'-chloro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.02-2.07 (m, 1H), 2.29-2.35 (m, 1H), 2.74-2.79 (m, 1H), 2.83 (d, J = 18.1 Hz, 1H), 2.89-2.95 (m, 1H), 3.39 (d, J = 17.3 Hz, 1H), 6.62 (d, J = 2.6 Hz, 1H), 6.68 (dd, J = 2.5, 8.5 Hz, 1H), 6.99 (d, J = 8.1 Hz, 1H), 7.06 (d, J = 8.4 Hz, 1H), 7.25 (t, J = 7.5 Hz, 1H), 7.30-7.36 (m, 3H), 7.46 (dd, J = 1.7, 8.4 Hz, 1H), 7.50 (d, J = 8.4 Hz, 2H).

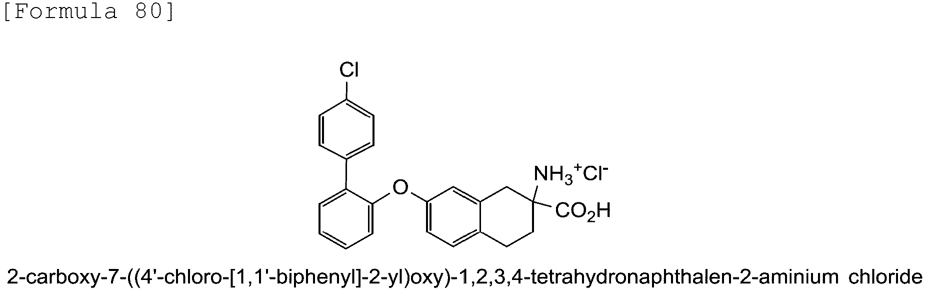

### (Example 1-29)

### 2- Preparation of Carboxy-7-((4'-trifluoromethyl-[1,1'-biphenyl]-2-yl)oxy)-1,2,3,4-tetrahydronaphthalene-2-aminium chloride (Compound 1-63)

### Step 1 (Steps I-IV)

### Preparation of methyl 2-((tert-butoxycarbonyl)amino)-7-((4'-trifluoromethyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-4'-(trifluoromethyl)-1,1'-biphenyl, and the reaction temperature was further changed from 90°C. to 120°C.

1H-NMR (CDCl3); 1.42 (s, 9H), 2.06-2.12 (m, 1H), 2.44 (brs, 1H), 2.77-2.80 (m, 2H), 2.87 (d, J = 16.5 Hz, 1H), 3.24 (d, J = 17.0 Hz, 1H), 3.75 (s, 3H), 4.78 (brs, 1H), 6.64 (d, J = 3.0 Hz, 1H), 6.75 (dd, J = 2.0, 8.0 Hz, 1H), 7.00 (dd, J = 1.0, 8.0 Hz, 1H), 7.03 (d, J = 8.5 Hz, 1H), 7.22 (td, J = 1.0, 7.5 Hz, 1H), 7.33 (td, J = 2.0, 8.0 Hz, 1H), 7.44 (dd, J = 1.5, 7.5 Hz, 1H), 7.62-7.67 (m, 4H).

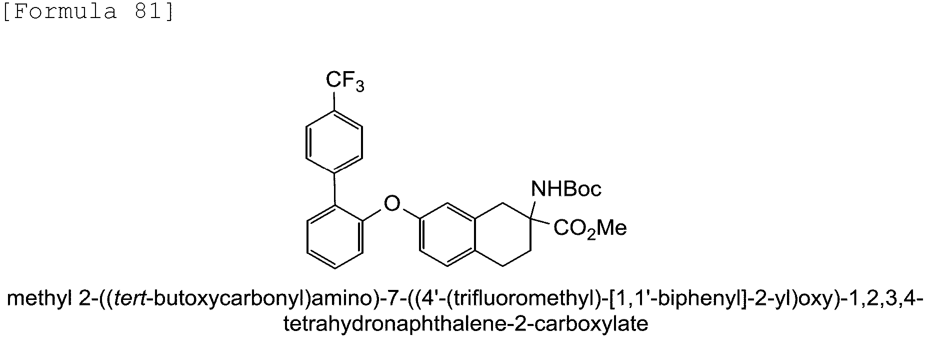

### Step 2 (Steps V-VI)

### 2- Production of carboxy-7-((4'-trifluoromethyl-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.04-2.07 (m, 1H), 2.30-2.36 (m, 1H), 2.75-2.82 (m, 1H), 2.84 (d, J = 17.5 Hz, 1H), 2.91-2.96 (m, 1H), 3.40 (d, J = 17.5 Hz, 1H), 6.66 (d, J = 2.0 Hz, 1H), 6.71 (dd, J = 2.0, 8.5 Hz, 1H), 7.01 (dd, J = 1.0, 8.0 Hz, 1H), 7.08 (d, J = 8.5 Hz, 1H), 7.28 (td, J = 1.0, 7.5 Hz, 1H), 7.39 (td, J = 1.5, 8.5 Hz, 1H), 7.50 (dd, J = 1.5, 8.0 Hz, 1H), 7.63 (d, J = 8.0 Hz, 2H), 7.71 (d, J = 8.5 Hz, 2H).

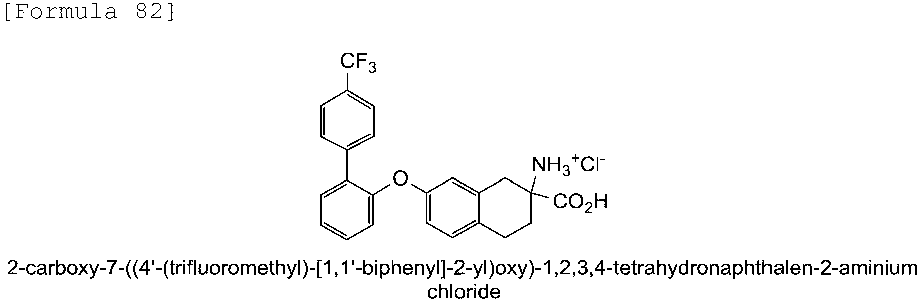

### (Example 1-30)

### 2- Preparation of carboxy-7-((3'-fluoro-[1,1'-biphenyl]-2-yl)oxy)-1,2,3,4-tetrahydronaphthalene-2-aminium chloride (Compound 1-54).

### Step 1 (Steps I-IV)

### Preparation of methyl 2-((tert-butoxycarbonyl)amino)-7-((3'-fluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-fluoro-1,1'-biphenyl, and the reaction temperature was further changed from 90°C. to 120°C.

1H-NMR (CDCl3); 1.42 (s, 9H), 2.05-2.12 (m, 1H), 2.45 (brs, 1H), 2.77-2.79 (m, 2H), 2.86 (d, J = 17.0 Hz, 1H), 3.22 (d, J = 16.5 Hz, 1H), 3.76 (s, 3H), 4.77 (brs, 1H), 6.62 (d, J = 2.5 Hz, 1H), 6.75 (dd, J = 2.5, 8.5 Hz, 1H), 6.98-7.03 (m, 3H), 7.20 (td, J = 1.0, 7.5 Hz, 1H), 7.28-7.35 (m, 4H), 7.43 (dd, J = 1.5, 8.0 Hz, 1H).

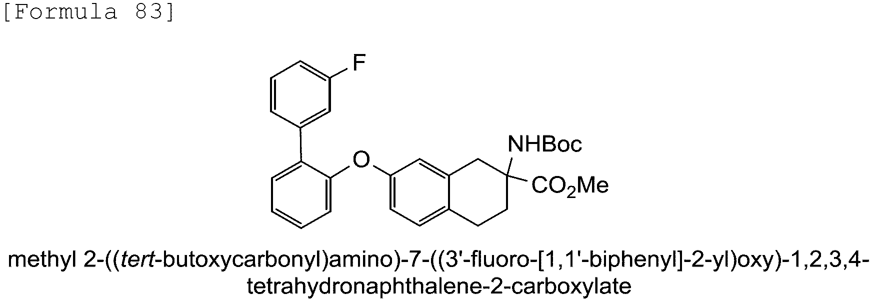

### Step 2 (Steps V-VI)

### 2- Production of carboxy-7-((3'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.04-2.08 (m, 1H), 2.30-2.36 (m, 1H), 2.75-2.82 (m, 1H), 2.85 (d, J = 17.5 Hz, 1H), 2.91-2.96 (m, 1H), 3.41 (d, J = 17.0 Hz, 1H), 6.65 (d, J = 2.5 Hz, 1H), 6.71 (dd, J = 2.5, 8.5 Hz, 1H), 6.98-7.02 (m, 2H), 7.08 (d, J = 8.5 Hz, 1H), 7.24-7.27 (m, 2H), 7.31-7.37 (m, 3H), 7.47 (dd, J = 2.0, 7.5 Hz, 1H).

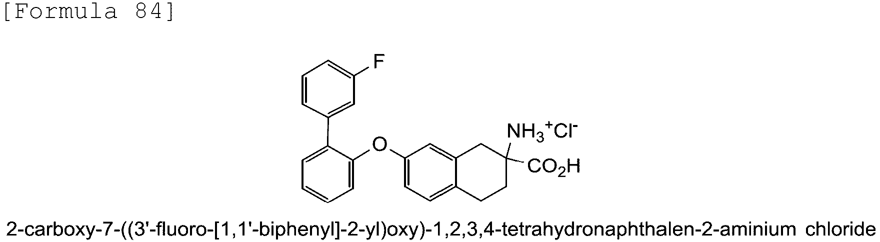

### (Example 1-31)

### Preparation of 3-(2-((7-ammonio-7-carboxy-5,6,7,8-tetrahydronaphthalen-2-yl)oxy)phenyl)pyridin-1-ium chloride (Compound 1-65)

### Step 1 (Steps I-IV)

### Preparation of methyl 2-((tert-butoxycarbonyl)amino)-7-(2-(pyridin-3-yl)phenoxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 3-(2-bromophenyl)pyridine, and the reaction temperature was further changed from 90°C. to 120°C.

1H-NMR (CDCl3); 1.42 (s, 9H), 2.05-2.12 (m, 1H), 2.44 (brs, 1H), 2.76-2.79 (m, 2H), 2.86 (d, J = 16.5 Hz, 1H), 3.23 (d, J = 16.5 Hz, 1H), 3.75 (s, 3H), 4.79 (brs, 1H), 6.62 (d, J = 3.0 Hz, 1H), 6.74 (dd, J = 2.0, 8.0 Hz, 1H), 7.01-7.03 (m, 2H), 7.23 (td, J = 1.0, 7.5 Hz, 1H), 7.30 (dd, J = 5.0, 8.0 Hz, 1H), 7.34 (td, J = 1.5, 8.0 Hz, 1H), 7.45 (dd, J = 1.5, 7.5 Hz, 1H), 7.88 (dt, J = 2.5, 4.0 Hz, 1H), 8.54 (brs, 1H), 8.79 (brs, 1H).

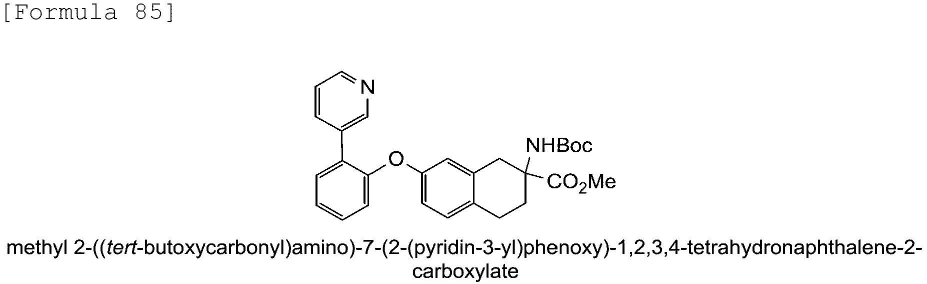

### Step 2 (Steps V-VI)

### Preparation of 3-(2-((7-ammonio-7-carboxy-5,6,7,8-tetrahydronaphthalen-2-yl)oxy)phenyl)pyridin-1-ium chloride

The following compound was synthesized from the compound obtained in Step 1 in the same manner as in Steps 2 and 3 of Example 1-26.

1H-NMR (CD3OD); 2.12-2.15 (m, 1H), 2.32-2.38 (m, 1H), 2.80-2.86 (m, 1H), 2.93 (d, J = 16.5 Hz, 1H), 2.98-2.99 (m, 1H), 3.42 (d, J = 17.2 Hz, 1H), 6.71 (d, J = 2.4 Hz, 1H), 6.75 (dd, J = 2.2, 8.1 Hz, 1H), 7.04 (d, J = 8.1 Hz, 1H), 7.12 (d, J = 8.3 Hz, 1H), 7.32 (t, J = 7.4 Hz, 1H), 7.43 (t, J = 7.8 Hz, 1H), 7.55 (d, J = 7.5 Hz, 1H), 7.70 (brs, 1H), 8.21 (d, J = 7.6 Hz, 1H).

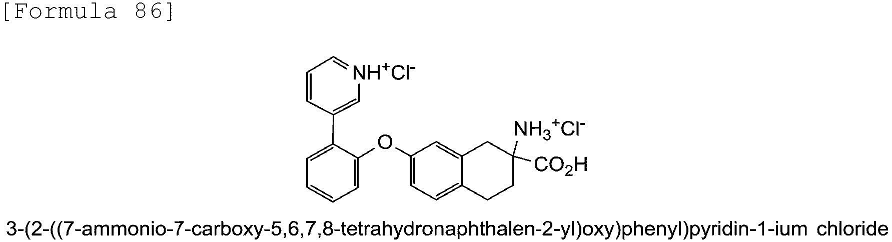

### (Example 1-32)

### Preparation of 2-(2-((7-ammonio-7-carboxy-5,6,7,8-tetrahydronaphthalen-2-yl)oxy)phenyl)pyridin-1-ium chloride (Compound 1-66)

### Step 1 (Steps I-IV)

### Preparation of methyl 2-((tert-butoxycarbonyl)amino)-7-(2-(pyridin-2-yl)phenoxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-(2-bromophenyl)pyridine, and the reaction temperature was further changed from 90°C. to 120°C.

1H-NMR (CDCl3); 1.42 (s, 9H), 2.05-2.12 (m, 1H), 2.46 (brs, 1H), 2.77-2.79 (m, 2H), 2.85 (d, J = 17.5 Hz, 1H), 3.21 (d, J = 17.5 Hz, 1H), 3.75 (s, 3H), 4.77 (brs, 1H), 6.66 (d, J = 2.5 Hz, 1H), 6.78 (dd, J = 2.0, 8.0 Hz, 1H), 6.99 (dd, J = 1.5, 8.5 Hz, 1H), 7.02 (d, J = 8.0 Hz, 1H), 7.18-7.27 (m, 3H), 7.65 (td, J = 1.5, 8.0 Hz, 1H), 7.83 (d, J = 8.0 Hz, 1H), 7.93 (dd, J = 1.5, 8.0 Hz, 1H), 8.69 (td, J = 1.0, 2.0 Hz, 1H).

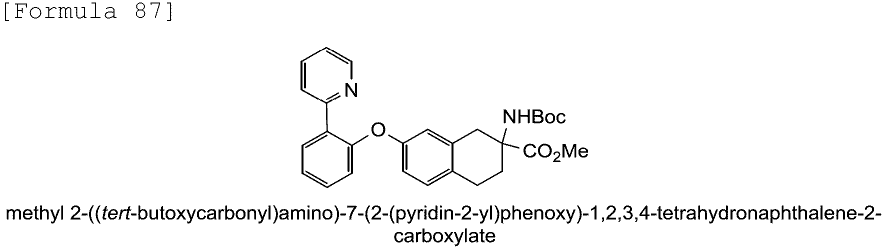

### Step 2 (Steps V-VI)

### Preparation of 2-(2-((7-ammonio-7-carboxy-5,6,7,8-tetrahydronaphthalen-2-yl)oxy)phenyl)pyridin-1-ium chloride

The following compound was synthesized from the compound obtained in Step 1 in the same manner as in Steps 2 and 3 of Example 1-26.

1H-NMR (CD3OD); 2.15-2.20 (m, 1H), 2.36-2.42 (m, 1H), 2.86-2.93 (m, 1H), 2.97-3.02 (m, 1H), 3.03 (d, J = 17.5 Hz, 1H), 3.46 (d, J = 17.5 Hz, 1H), 6.90-6.93 (m, 2H), 7.08 (d, J = 8.5 Hz, 1H), 7.22 (d, J = 8.0 Hz, 1H), 7.40 (t, J = 9.0 Hz, 1H), 7.61-7.65 (m, 1H), 7.79 (dd, J = 1.5, 8.0 Hz, 1H), 8.04 (t, J = 6.0 Hz, 1H), 8.34 (d, J = 8.0 Hz, 1H), 8.65 (td, J = 2.0, 8.0 Hz, 1H), 8.89 (d, J = 5.5 Hz, 1H).

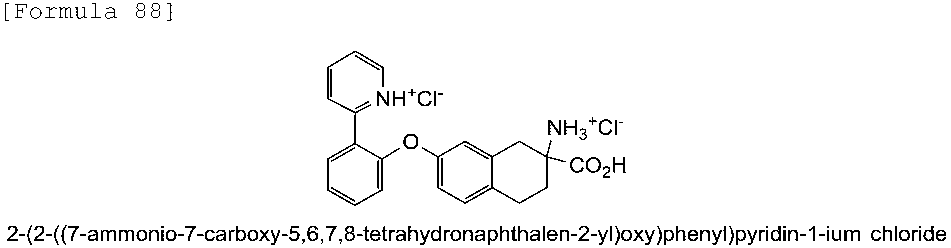

### (Example 1-33)

### 2- Preparation of carboxy-7-((3'-chloro-[1,1'-biphenyl]-2-yl)oxy)-1,2,3,4-tetrahydronaphthalene-2-aminium chloride (Compound 1-55)

### Step 1 (Steps I-IV)

### Preparation of methyl 2-((tert-butoxycarbonyl)amino)-7-((3'-chloro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-chloro-1,1'-biphenyl, and the reaction temperature was further changed from 90°C. to 120°C.

1H-NMR (CDCl3); 1.42 (s, 9H), 2.06-2.12 (m, 1H), 2.46 (brs, 1H), 2.77-2.83 (m, 2H), 2.86 (d, J = 17.0 Hz, 1H), 3.22 (d, J = 17.5 Hz, 1H), 3.75 (s, 3H), 4.77 (brs, 1H), 6.62 (d, J = 2.0 Hz, 1H), 6.74 (dd, J = 2.0, 8.5 Hz, 1H), 6.97-7.01 (m, 2H), 7.20 (td, J = 1.0, 7.5 Hz, 1H), 7.26-7.32 (m, 3H), 7.40-7.43 (m, 2H), 7.53 (d, J = 2.0 Hz, 1H).

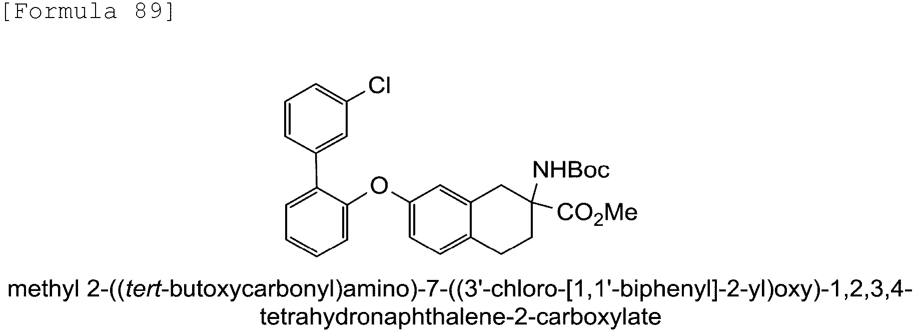

### Step 2 (Steps V-VI)

### 2- Production of carboxy-7-((3'-chloro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.01-2.03 (m, 1H), 2.28-2.34 (m, 1H), 2.73-2.77 (m, 1H), 2.81 (d, J = 17.0 Hz, 1H), 2.89-2.94 (m, 1H), 3.40 (d, J = 17.0 Hz, 1H), 6.63 (d, J = 2.0 Hz, 1H), 6.69 (dd, J = 3.0, 8.5 Hz, 1H), 6.99 (dd, J = 1.5, 8.0 Hz, 1H), 7.06 (d, J = 8.5 Hz, 1H), 7.23-7.36 (m, 4H), 7.42-7.46 (m, 2H), 7.52 (t, J = 1.5 Hz, 1H).

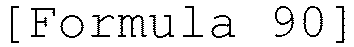

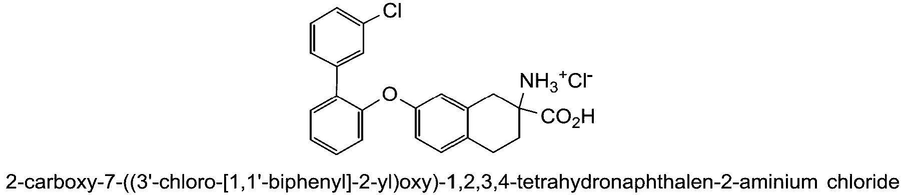

### (Example 1-34)

### 2- Preparation of carboxy-7-((3'-isopropyl-[1,1'-biphenyl]-2-yl)oxy)-1,2,3,4-tetrahydronaphthalene-2-aminium chloride (Compound 1-60)

### Step 1 (Steps I-IV)

### Preparation of methyl 2-((tert-butoxycarbonyl)amino)-7-((3'-isopropyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-isopropyl-1,1'-biphenyl, and the reaction temperature was further changed from 90°C. to 120°C.

1H-NMR (CDCl3); 1.22 (d, J = 6.5 Hz, 6H), 1.42 (s, 9H), 2.05-2.11 (m, 1H), 2.46 (brs, 1H), 2.76-2.78 (m, 2H), 2.83 (d, J = 18.0 Hz, 1H), 2.87-2.92 (m, 1H), 3.19 (d, J = 17.0 Hz, 1H), 3.75 (s, 3H), 4.75 (brs, 1H), 6.60 (d, J = 2.0 Hz, 1H), 6.73 (dd, J = 3.0, 8.5 Hz, 1H), 6.99-7.09 (m, 2H), 7.15 (d, J = 7.5 Hz, 1H), 7.20 (td, J = 1.0, 7.5 Hz, 1H), 7.26-7.34 (m, 3H), 7.38 (s, 1H), 7.46 (dd J = 1.5, 7.5 Hz, 1H).

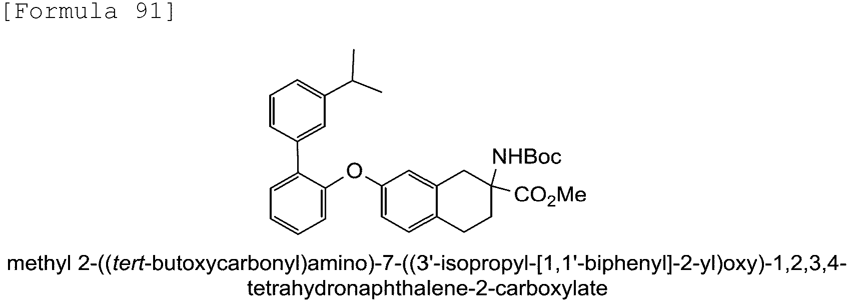

### Step 2 (Steps V-VI)

### 2- Production of carboxy-7-((3'-isopropyl-[1,1'-biphenyl]-2-yl)oxy)-1,2,3,4-tetrahydronaphthalene-2-aminium chloride The following compound was synthesized from the compound obtained in Step 1 in the same manner as in Steps 2 and 3 of Example 1-26.

1H-NMR (CD3OD); 1.21 (d, J = 7.0 Hz, 6H), 2.02-2.05 (m, 1H), 2.28-2.34 (m, 1H), 2.73-2.77 (m, 1H), 2.81 (d, J = 17.0 Hz, 1H), 2.85-2.93 (m, 2H), 3.39 (d, J = 17.0 Hz, 1H), 6.61 (d, J = 2.5 Hz, 1H), 6.68 (dd, J = 2.5, 8.0 Hz, 1H), 6.99 (d, J = 8.0 Hz, 1H), 7.05 (d, J = 8.5 Hz, 1H), 7.12 (d, J = 7.5 Hz, 1H), 7.20-7.28 (m, 3H), 7.32 (td, J = 1.5, 7.0 Hz, 1H), 7.37 (s, 1H), 7.44 (dd, J = 1.5, 8.0 Hz, 1H).

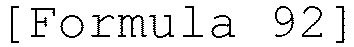

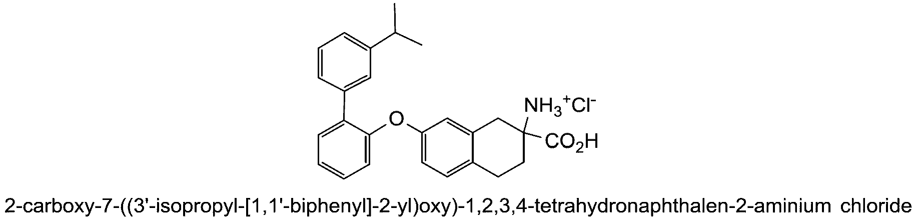

### (Example 1-35)

### 2- Preparation of Carboxy-7-(2-(naphthalen-1-yl)phenoxy)-1,2,3,4-tetrahydronaphthalene-2-aminium chloride (Compound 1-68)

### Step 1 (Steps I-IV)

### Preparation of methyl 2-((tert-butoxycarbonyl)amino)-7-(2-(naphthalen-1-yl)phenoxy)-1,2,3,4-tetrahydronaphthalene-2-carboxylate

A mixture of the following compounds and isomers thereof was obtained in the same manner as in Steps 1 to 4 of Example 1-1, except that 2-iodonaphthalene in Step 4 of Example was changed to 1-(2-bromophenyl)naphthalene, and the reaction temperature was further changed from 90°C. to 120°C.

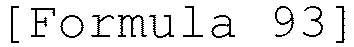

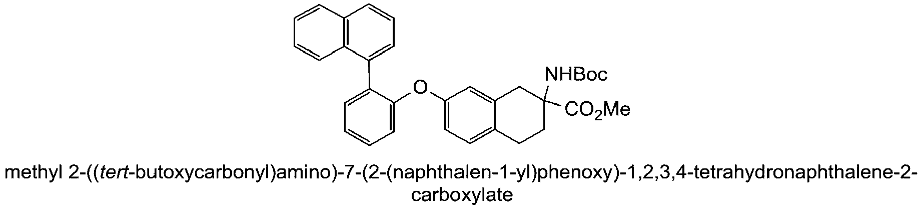

### Step 2 (Steps V-VI)

### 2- Production of carboxy-7-(2-(naphthalen-1-yl)phenoxy)-1,2,3,4-tetrahydronaphthalene-2-aminium chloride

The compound obtained in Step 1 was used in the same manner as in Steps 2 and 3 of Example 1-26 to obtain a mixture of the following compounds and isomers thereof.

HRMS (ESI) m/z calcd for C27H24NO3 [M H] 410.1756, found 410.1821.

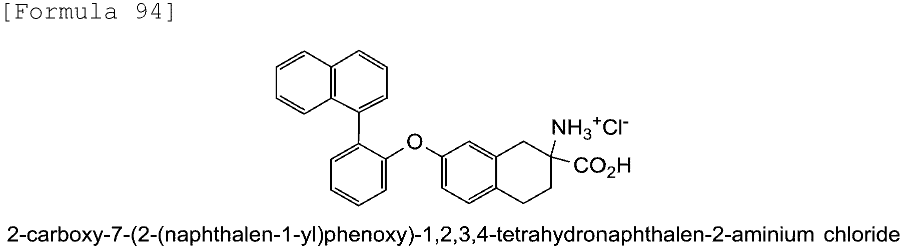

### (Example 1-36)

### 2- Preparation of carboxy-7-((2'-fluoro-[1,1'-biphenyl]-2-yl)oxy)-1,2,3,4-tetrahydronaphthalene-2-aminium chloride (Compound 1-56)

### Step 1 (Steps I-IV)

### Preparation of methyl 2-((tert-butoxycarbonyl)amino)-7-((2'-fluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-2'-fluoro-1,1'-biphenyl, and the reaction temperature was further changed from 90°C. to 120°C.

1H-NMR (CDCl3); 1.42 (s, 9H), 2.05-2.12 (m, 1H), 2.47 (brs, 1H), 2.75-2.78 (m, 2H), 2.84 (d, J = 17.0 Hz, 1H), 3.20 (d, J = 17.5 Hz, 1H), 3.76 (s, 3H), 4.76 (brs, 1H), 6.65 (d, J = 2.5 Hz, 1H), 6.77 (dd, J = 2.0, 8.0 Hz, 1H), 6.97 (dd, J = 1.0, 8.0 Hz, 1H), 7.00 (d, J = 8.0 Hz, 1H), 7.06-7.10 (m, 1H), 7.14 (dd, J = 1.5, 7.5 Hz, 1H), 7.18 (td, J = 1.0, 7.5 Hz, 1H), 7.28-7.39 (m, 4H).

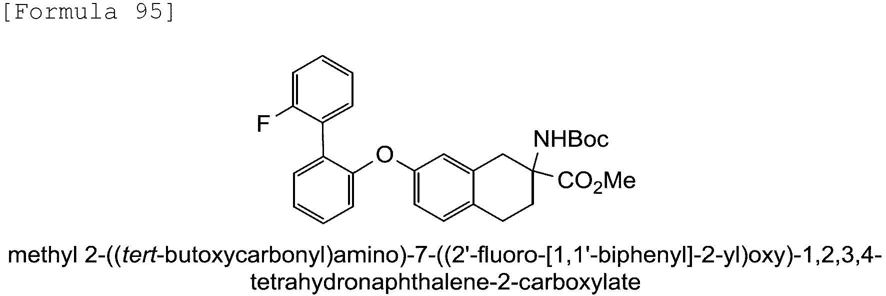

### Step 2 (Steps V-VI)

### 2- Production of carboxy-7-((2'-fluoro-[1,1'-biphenyl]-2-yl)oxy)-1,2,3,4-tetrahydronaphthalene-2-aminium chloride 2-carboxy-7-((2'-fluoro-[1,1'-biphenyl]-2-yl)oxy)-1,2,3,4-tetrahydronaphthalen-2-aminium chloride

The following compound was synthesized from the compound obtained in Step 1 in the same manner as in Steps 2 and 3 of Example 1-26.

1H-NMR (CD3OD); 2.15-2.16 (m, 1H), 2.34-2.40 (m, 1H), 2.79-2.86 (m, 1H), 2.92 (d, J = 17.5 Hz, 1H), 2.95-3.00 (m, 1H), 3.42 (d, J = 17.5 Hz, 1H), 6.69 (d, J = 2.5 Hz, 1H), 6.75 (dd, J = 3.0, 8.5 Hz, 1H), 6.97 (d, J = 8.5 Hz, 1H), 7.05-7.07 (m, 1H), 7.10 (d, J = 8.0 Hz, 1H), 7.15 (td, J = 1.5, 7.5 Hz, 1H), 7.23 (td, J = 1.0, 7.5 Hz, 1H), 7.29-7.38 (m, 4H).

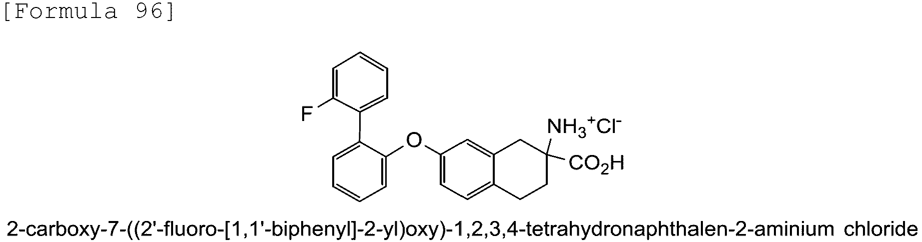

### (Example 1-37)

### 2- Preparation of carboxy-7-((2'-chloro-[1,1'-biphenyl]-2-yl)oxy)-1,2,3,4-tetrahydronaphthalene-2-aminium chloride (Compound 1-57)

### Step 1 (Steps I-IV)

### Preparation of methyl 2-((tert-butoxycarbonyl)amino)-7-((2'-chloro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-2'-chloro-1,1'-biphenyl, and the reaction temperature was further changed from 90°C. to 120°C.

1H-NMR (CDCl3); 1.42 (s, 9H), 2.04-2.10 (m, 1H), 2.46 (brs, 1H), 2.73-2.78 (m, 2H), 2.82 (d, J = 16.3 Hz, 1H), 3.18 (d, J = 16.9 Hz, 1H), 3.75 (s, 3H), 4.74 (brs, 1H), 6.60 (d, J = 2.6 Hz, 1H), 6.74 (dd, J = 2.6, 8.4 Hz, 1H), 6.96-6.99 (m, 2H), 7.16-7.25 (m, 3H), 7.29-7.35 (m, 3H), 7.39-7.41 (m, 1H).

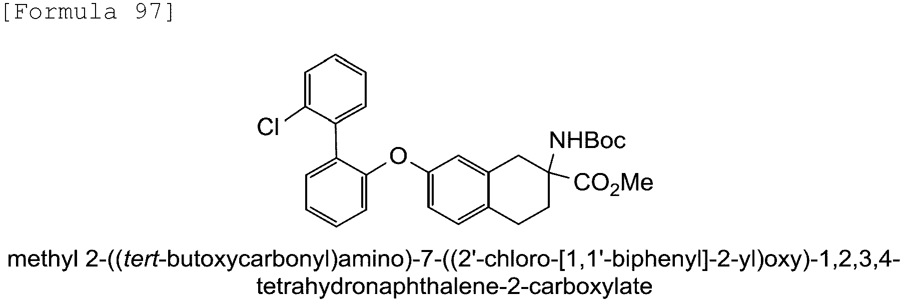

### Step 2 (Steps V-VI)

### 2- Production of carboxy-7-((2'-chloro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.10-2.15 (m, 1H), 2.33-2.39 (m, 1H), 2.78-2.85 (m, 1H), 2.91 (d, J = 17.5 Hz, 1H), 2.94-2.99 (m, 1H), 3.41 (d, J = 17.0 Hz, 1H), 6.66 (d, J = 2.0 Hz, 1H), 6.74 (dd, J = 2.5, 8.5 Hz, 1H), 6.97 (dd, J = 1.0, 8.0 Hz, 1H), 7.07 (d, J = 8.5 Hz, 1H), 7.21 (td, J = 1.0, 7.5 Hz, 1H), 7.24-7.32 (m, 4H), 7.35-7.41 (m, 2H).

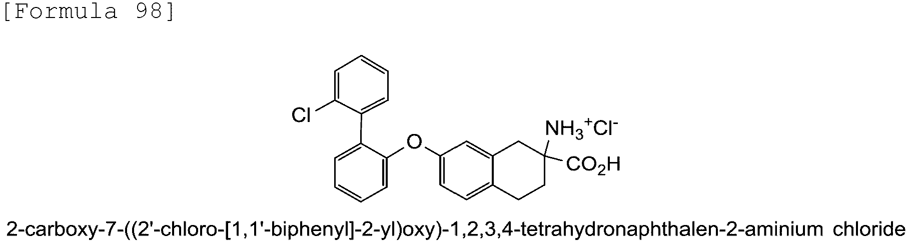

### (Example 1-38)

### 2- Preparation of carboxy-7-((8-chloronaphthalen-1-yl)oxy)-1,2,3,4-tetrahydronaphthalene-2-aminium chloride (Compound 1-62)

### Step 1 (Steps I-IV)

### Preparation of methyl 2-((tert-butoxycarbonyl)amino)-7-((8-chloronaphthalen-1-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 1-bromo-8-chloronaphthalene, and the reaction temperature was further changed from 90°C. to 120°C.

1H-NMR (CDCl3); 1.42 (s, 9H), 2.07-2.14 (m, 1H), 2.48 (brs, 1H), 2.79-2.82 (m, 2H), 2.86 (d, J = 17.5 Hz, 1H), 3.23 (d, J = 17.5 Hz, 1H), 3.75 (s, 3H), 4.81 (brs, 1H), 6.61 (d, J = 2.5 Hz, 1H), 6.75 (dd, J = 2.0, 8.0 Hz, 1H), 7.06 (d, J = 9.0 Hz, 1H), 7.11 (dd, J = 1.0, 8.0 Hz, 1H), 7.38 (t, J = 8.0 Hz, 1H), 7.44 (t, J = 8.0 Hz, 1H), 7.53 (dd, J = 1.0, 7.0 Hz, 1H), 7.68 (d, J = 8.0 Hz, 1H), 7.78 (d, J = 8.0 Hz, 1H).

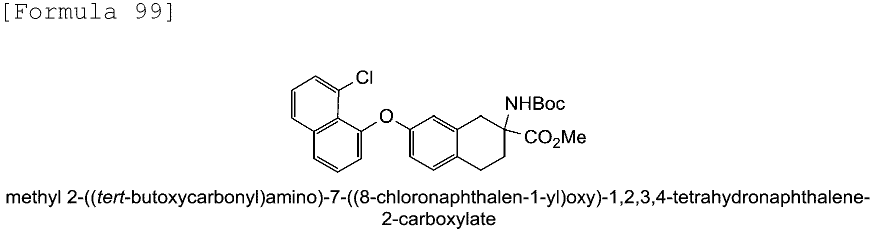

### Step 2 (Steps V-VI)

### 2- Production of carboxy-7-((8-chloronaphthalen-1-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.14-2.19 (m, 1H), 2.37-2.43 (m, 1H), 2.83-2.90 (m, 1H), 2.94 (d, J = 17.0 Hz, 1H), 2.98-3.04 (m, 1H), 3.44 (d, J = 17.5 Hz, 1H), 6.65 (d, J = 2.5 Hz, 1H), 6.75 (dd, J = 2.5, 8.0 Hz, 1H), 7.12 (dd, J = 1.0, 7.5 Hz, 1H), 7.15 (d, J = 8.0 Hz, 1H), 7.43 (t, J = 7.5 Hz, 1H), 7.49-7.54 (m, 2H), 7.78-7.80 (m, 1H), 7.89 (dd, J = 1.0, 8.0 Hz, 1H).

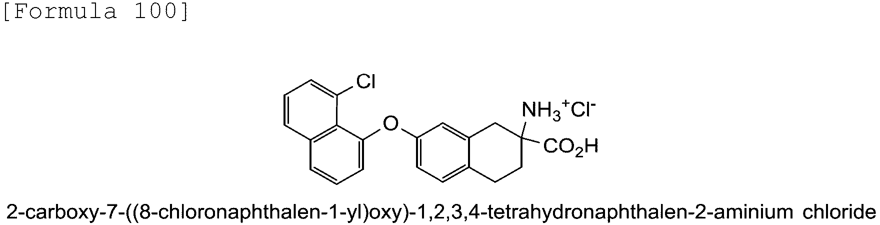

### (Example 1-39)

### 2- Preparation of carboxy-7-((3'-methoxy-[1,1'-biphenyl]-2-yl)oxy)-1,2,3,4-tetrahydronaphthalene-2-aminium chloride (Compound 1-59)

### Step 1 (Steps I-IV)

### Preparation of methyl 2-((tert-butoxycarbonyl)amino)-7-((3'-methoxy-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-methoxy-1,1'-biphenyl, and the reaction temperature was further changed from 90°C. to 120°C.

1H-NMR (CDCl3); 1.42 (s, 9H), 2.05-2.11 (m, 1H), 2.45 (brs, 1H), 2.77-2.78 (m, 2H), 2.84 (d, J = 17.0 Hz, 1H), 3.21 (d, J = 16.5 Hz, 1H), 3.75 (s, 3H), 3.77 (s, 3H), 4.77 (brs, 1H), 6.62 (d, J = 2.0 Hz, 1H), 6.74 (dd, J = 2.0, 8.0 Hz, 1H), 6.84-6.86 (m, 1H), 6.98-7.01 (m, 2H), 7.09 (t, J = 1.5 Hz, 1H), 7.11 (dt, J = 1.5, 7.5 Hz, 1H), 7.20 (td, J = 1.0, 7.5 Hz, 1H), 7.27-7.31 (m, 2H), 7.45 (dd, J = 1.5, 7.0 Hz, 1H).

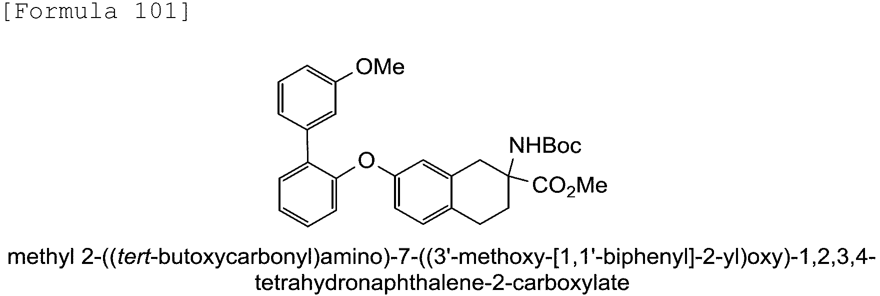

### Step 2 (Steps V-VI)

### 2- Production of carboxy-7-((3'-methoxy-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 1.99-2.03 (m, 1H), 2.27-2.34 (m, 1H), 2.72-2.79 (m, 1H), 2.79 (d, J = 17.0 Hz, 1H), 2.88-2.93 (m, 1H), 3.39 (d, J = 17.0 Hz, 1H), 3.73 (s, 3H), 6.61 (d, J = 3.0 Hz, 1H), 6.68 (dd, J = 2.0, 8.5 Hz, 1H), 6.82 (td, J = 1.5, 8.0 Hz, 1H), 6.98 (dd, J = 1.5, 8.0 Hz, 1H), 7.04-7.07 (m, 3H), 7.20-7.25 (m, 2H), 7.32 (td, J = 1.5, 8.0 Hz, 1H), 7.46 (dd, J = 1.5 , 7.5 Hz, 1H).

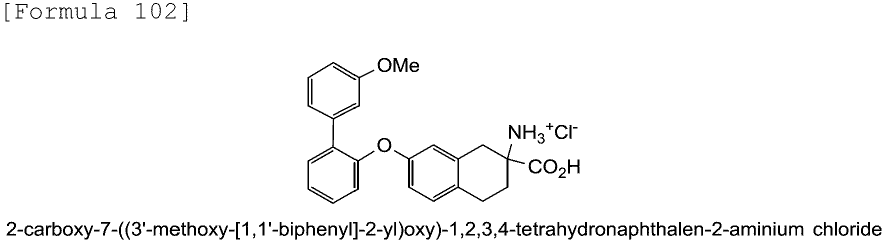

### (Example 1-40)

### Preparation of 2-((7-Ammonio-7-carboxy-5,6,7,8-tetrahydronaphthalen-2-yl)oxy)-3-phenylpyridin-1-ium chloride (Compound 1-69)

### Step 1 (Steps I-IV)

### Preparation of methyl 2-((tert-butoxycarbonyl)amino)-7-((3-phenylpyridin-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3-phenylpyridine, and the reaction temperature was further changed from 90°C. to 120°C.

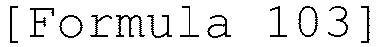

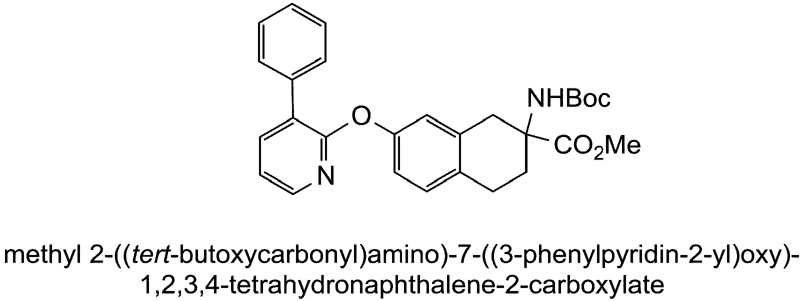

### Step 2 (Steps V-VI)

### Preparation of 2-((7-Ammonio-7-carboxy-5,6,7,8-tetrahydronaphthalen-2-yl)oxy)-3-phenylpyridin-1-ium chloride

The following compound was synthesized from the compound obtained in Step 1 in the same manner as in Steps 2 and 3 of Example 1-26.

1H-NMR (CD3OD); 2.13-2.18 (m, 1H), 2.40-2.46 (m, 1H), 2.89-2.95 (m, 1H), 3.03 (d, J = 17.0 Hz, 1H), 3.01-3.05 (m, 1H), 3.49 (d, J = 17.0 Hz, 1H), 6.97-7.00 (m, 2H), 7.25 (d, J = 8.0 Hz, 1H), 7.39-7.47 (m, 4H), 7.63-7.65 (m, 2H), 8.14-8.45 (m, 2H).

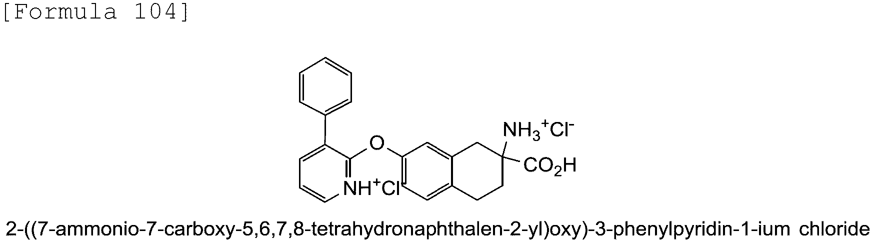

### (Example 1-41)

### 2- Preparation of Carboxy-7-((3'-(trifluoromethyl)-[1,1'-biphenyl]-2-yl)oxy)-1,2,3,4-tetrahydronaphthalene-2-aminium chloride (Compound 1-64).

### Step 1 (Steps I-IV)

### Preparation of methyl 2-((tert-butoxycarbonyl)amino)-7-((3'-(trifluoromethyl)-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-(trifluoromethyl)-1,1'-biphenyl, and the reaction temperature was further changed from 90°C. to 120°C.

1H-NMR (CDCl3); 1.42 (s, 9H), 2.05-2.11 (m, 1H), 2.44 (brs, 1H), 2.76-2.78 (m, 2H), 2.85 (d, J = 16.5 Hz, 1H), 3.22 (d, J = 16.5 Hz, 1H), 3.75 (s, 3H), 4.76 (brs, 1H), 6.61 (d, J = 2.5 Hz, 1H), 6.73 (dd, J = 2.5, 8.0 Hz, 1H), 7.00-7.02 (m, 2H), 7.22 (td, J = 1.0, 8.0 Hz, 1H), 7.33 (td, J = 1.5, 8.0 Hz, 1H), 7.44 (dd, J = 1.5, 7.0 Hz, 1H), 7.48 (d, J = 8.0 Hz, 1H), 7.55 (d, J = 8.0 Hz, 1H), 7.72 (d, J = 7.5 Hz, 1H), 7.80 (s, 1H).

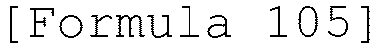

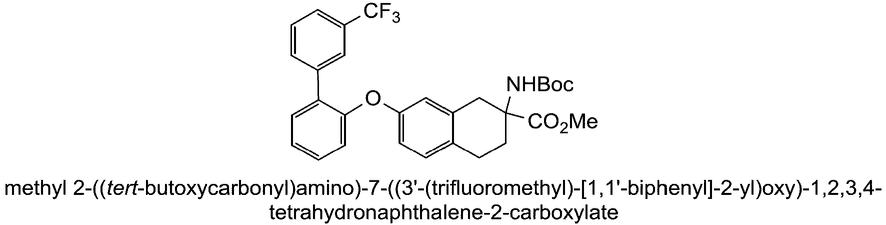

### Step 2 (Steps V-VI)

### 2- Production of carboxy-7-((3'-(trifluoromethyl)-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.07-2.10 (m, 1H), 2.30-2.36 (m, 1H), 2.76-2.83 (m, 1H), 2.87 (d, J = 17.0 Hz, 1H), 2.91-2.96 (m, 1H), 3.40 (d, J = 17.0 Hz, 1H), 6.66 (d, J = 2.0 Hz, 1H), 6.71 (dd, J = 2.0, 8.5 Hz, 1H), 7.02 (d, J = 8.0 Hz, 1H), 7.08 (d, J = 9.0 Hz, 1H), 7.29 (td, J = 1.0, 7.5 Hz, 1H), 7.39 (td, J = 2.0, 8.0 Hz, 1H), 7.50 (dd, J = 2.0, 7.5 Hz, 1H), 7.51-7.58 (m, 2H), 7.76 (d, J = 8.0 Hz, 1H), 7.82 (s, 1H).

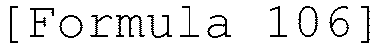

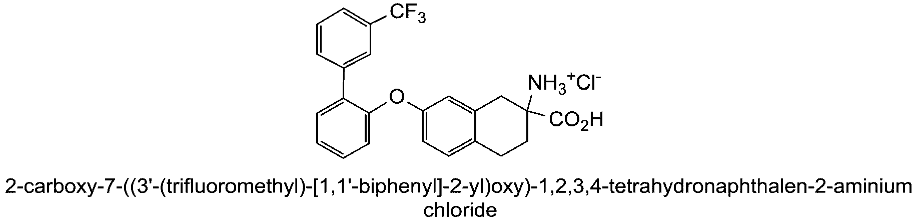

### (Example 1-42)

### 2- Preparation of carboxy-7-((3'-nitro-[1,1'-biphenyl]-2-yl)oxy)-1,2,3,4-tetrahydronaphthalene-2-aminium chloride (Compound 1-74).

### Step 1 (Steps I-IV)

### Preparation of methyl 2-((tert-butoxycarbonyl)amino)-7-((3'-nitro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-nitro-1,1'-biphenyl, and the reaction temperature was further changed from 90°C. to 120°C.

1H-NMR (CDCl3); 1.41 (s, 9H), 2.05-2.12 (m, 1H), 2.43 (brs, 1H), 2.77-2.79 (m, 2H), 2.87 (d, J = 17.5 Hz, 1H), 3.24 (d, J = 17.5 Hz, 1H), 3.75 (s, 3H), 4.77 (brs, 1H), 6.63 (d, J = 3.0 Hz, 1H), 6.74 (dd, J = 2.5, 8.0 Hz, 1H), 7.01-7.03 (m, 2H), 7.24 (td, J = 1.0, 6.5 Hz, 1H), 7.36 (td, J = 1.5, 8.0 Hz, 1H), 7.47 (dd, J = 1.5, 7.5 Hz, 1H), 7.54 (t, J = 8.0 Hz, 1H), 7.90 (dt, J = 2.0, 3.0 Hz, 1H), 8.15-8.17 (m, 1H), 8.44 (t, J = 2.5 Hz, 1H).

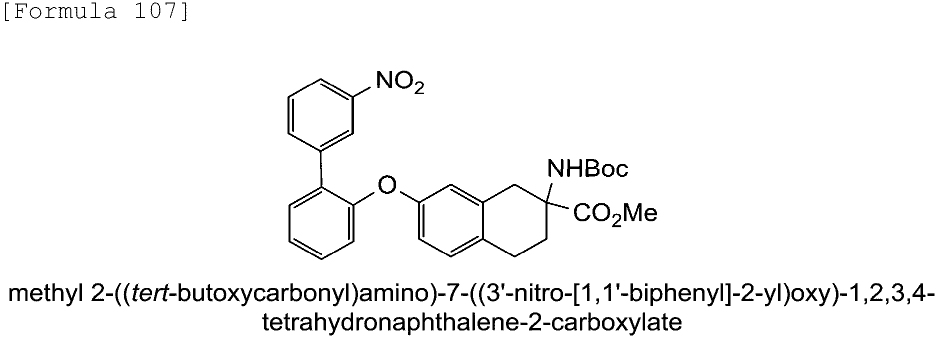

### Step 2 (Steps V-VI)

### 2- Production of carboxy-7-((3'-nitro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.01-2.04 (m, 1H), 2.27-2.33 (m, 1H), 2.73-2.79 (m, 1H), 2.82 (d, J = 17.5 Hz, 1H), 2.88-2.94 (m, 1H), 3.39 (d, J = 17.0 Hz, 1H), 6.66 (d, J = 2.0 Hz, 1H), 6.70 (dd, J = 2.5, 8.0 Hz, 1H), 7.02 (d, J = 8.5 Hz, 1H), 7.06 (d, J = 9.0 Hz, 1H), 7.29 (t, J = 8.0 Hz, 1H), 7.39 (td, J = 2.0, 8.0 Hz, 1H), 7.53 (dd, J = 2.0, 7.5 Hz, 1H), 7.59 (t, J = 8.0 Hz, 1H), 7.93 (d, J = 8.0 Hz, 1H), 8.14 (dd, J = 2.0, 8.5 Hz, 1H), 8.41 (t, J = 2.5 Hz, 1H).

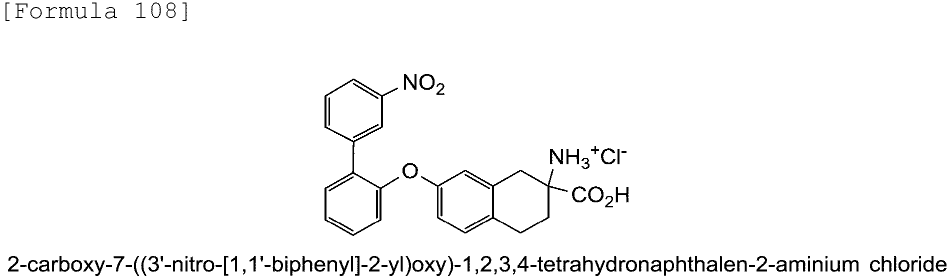

### (Example 1-43)

### 2- Preparation of carboxy-7-((3'-methyl-[1,1'-biphenyl]-2-yl)oxy)-1,2,3,4-tetrahydronaphthalene-2-aminium chloride (Compound 1-76).

### Step 1 (Steps I-IV)

### Preparation of methyl 2-((tert-butoxycarbonyl)amino)-7-((3'-methyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-methyl-1,1'-biphenyl, and the reaction temperature was further changed from 90°C. to 120°C.

1H-NMR (CDCl3); 1.42 (s, 9H), 2.05-2.11 (m, 1H), 2.36 (s, 3H), 2.45 (brs, 1H), 2.77-2.78 (m, 2H), 2.84 (d, J = 17.0 Hz, 1H), 3.21 (d, J = 17.5 Hz, 1H), 3.75 (s, 3H), 4.76 (brs, 1H), 6.62 (d, J = 2.0 Hz, 1H), 6.75 (dd, J = 2.0, 8.0 Hz, 1H), 6.97 (dd, J = 1.5, 8.0 Hz, 1H), 7.00 (d, J = 8.5 Hz, 1H), 7.11 (d, J = 7.5 Hz, 1H), 7.18 (td, J = 1.0, 7.5 Hz, 1H), 7.23-7.29 (m, 2H), 7.32-7.34 (m, 2H), 7.43 (dd, J = 1.5, 7.5 Hz, 1H).

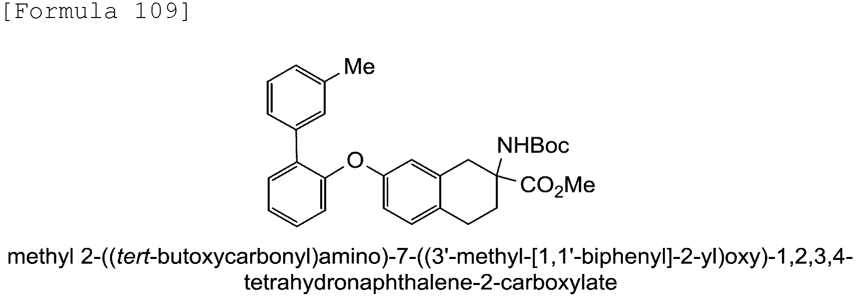

### Step 2 (Steps V-VI)

### 2- Production of carboxy-7-((3'-methyl-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.01-2.04 (m, 1H), 2.28-2.34 (m, 1H), 2.31 (s, 3H), 2.73-2.77 (m, 1H), 2.80 (d, J = 17.5 Hz, 1H), 2.87-2.93 (m, 1H), 3.39 (d, J = 17.5 Hz, 1H), 6.60 (d, J = 2.5 Hz, 1H), 6.67 (dd, J = 2.0, 8.0 Hz, 1H), 6.97 (dd, J = 1.0, 8.5 Hz, 1H), 7.04 (d, J = 8.0 Hz, 1H), 7.06 (d, J = 8.0 Hz, 1H), 7.17 (t, J = 7.0 Hz, 1H), 7.22 (td, J = 1.5, 7.5 Hz, 1H), 7.27-7.32 (m, 3H), 7.42 (dd, J = 2.0, 7.5 Hz, 1H).

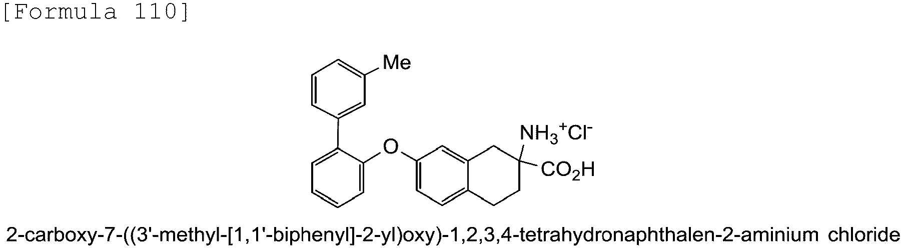

### (Example 1-44)

### 2- Preparation of carboxy-7-((8-fluoronaphthalen-1-yl)oxy)-1,2,3,4-tetrahydronaphthalene-2-aminium chloride (Compound 1-80)

### Step 1 (Steps I-IV)

### Preparation of methyl 2-((tert-butoxycarbonyl)amino)-7-((8-fluoronaphthalen-1-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 1-bromo-8-fluoronaphthalene, and the reaction temperature was further changed from 90°C. to 120°C.

1H-NMR (CDCl3); 1.41 (s, 9H), 2.09-2.14 (m, 1H), 2.47 (brs, 1H), 2.79-2.82 (m, 2H), 2.86 (d, J = 16.5 Hz, 1H), 3.23 (d, J = 17.0 Hz, 1H), 3.75 (s, 3H), 4.80 (brs, 1H), 6.65 (d, J = 2.0 Hz, 1H), 6.79 (dd, J = 2.5, 8.0 Hz, 1H), 7.02-7.12 (m, 3H), 7.39-7.44 (m, 2H), 7.65 (d, J = 8.0 Hz, 2H).

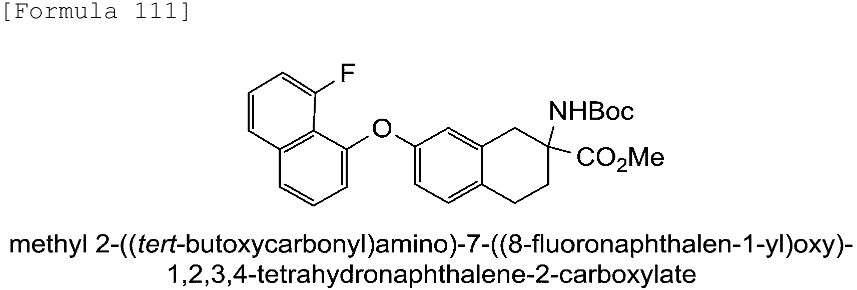

### Step 2 (Steps V-VI)

### 2- Production of carboxy-7-((8-fluoronaphthalen-1-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.13-2.18 (m, 1H), 2.37-2.43 (m, 1H), 2.83-2.90 (m, 1H), 2.94 (d, J = 17.5 Hz, 1H), 2.98-3.04 (m, 1H), 3.45 (d, J = 17.0 Hz, 1H), 6.70 (d, J = 3.0 Hz, 1H), 6.79 (dd, J = 2.5, 8.5 Hz, 1H), 7.05 (d, J = 7.5 Hz, 1H), 7.11 (dd, J = 7.0, 12.5 Hz, 1H), 7.16 (d, J = 8.5 Hz, 1H), 7.44-7.50 (m, 2H), 7.72-7.75 (m, 2H).

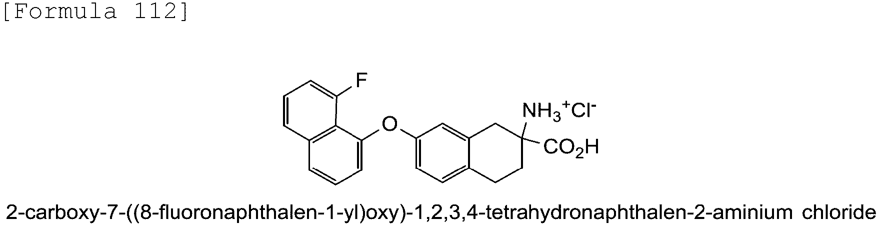

### (Example 1-45)

### 2- Preparation of Carboxy-7-((2',3'-difluoro-[1,1'-biphenyl]-2-yl)oxy)-1,2,3,4-tetrahydronaphthalene-2-aminium chloride (Compound 1-88)

### Step 1 (Steps I-IV)

### Preparation of methyl 2-((tert-butoxycarbonyl)amino)-7-((2',3'-difluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2,3-difluoro-1,1'-biphenyl, and the reaction temperature was further changed from 90°C. to 120°C.

1H-NMR (CDCl3); 1.42 (s, 9H), 2.06-2.12 (m, 1H), 2.45 (brs, 1H), 2.77-2.81 (m, 2H), 2.86 (d, J = 17.5 Hz, 1H), 3.22 (d, J = 17.5 Hz, 1H), 3.75 (s, 3H), 4.75 (brs, 1H), 6.66 (d, J = 2.0 Hz, 1H), 6.77 (dd, J = 2.5, 8.5 Hz, 1H), 6.96 (dd, J = 1.5, 8.0 Hz, 1H), 7.02 (d, J = 8.0 Hz, 1H), 7.05-7.14 (m, 3H), 7.18 (td, J = 1.0, 8.0 Hz, 1H), 7.32-7.38 (m, 2H).

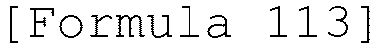

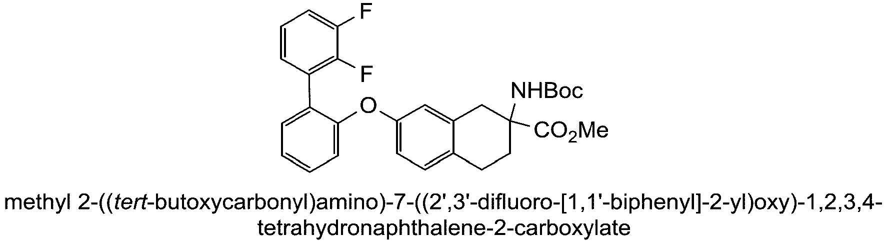

Step 2 (Steps V-VI)

### 2- Production of carboxy-7-((2',3'-difluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.00-2.05 (m, 1H), 2.30-2.36 (m, 1H), 2.75-2.79 (m, 1H), 2.82 (d, J = 17.5 Hz, 1H), 2.91-2.96 (m, 1H), 3.42 (d, J = 17.0 Hz, 1H), 6.68 (d, J = 2.0 Hz, 1H), 6.73 (dd, J = 3.0, 9.0 Hz, 1H), 6.97 (dd, J = 1.0, 8.5 Hz, 1H), 7.08 (d, J = 8.0 Hz, 1H), 7.13-7.24 (m, 3H), 7.36-7.40 (m, 2H).

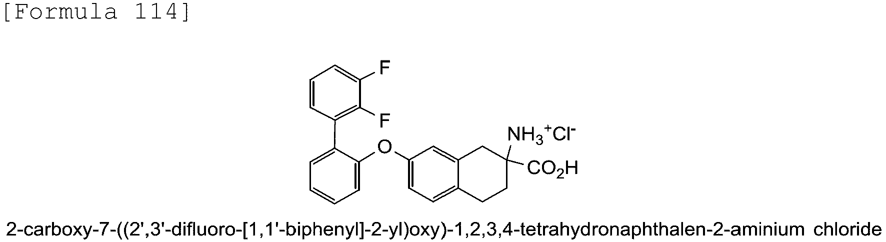

### (Example 1-46)

### 2- Preparation of carboxy-7-((8-methylnaphthalen-1-yl)oxy)-1,2,3,4-tetrahydronaphthalene-2-aminium chloride (Compound 1-86)

### Step 1 (Steps I-IV)

### Preparation of methyl 2-((tert-butoxycarbonyl)amino)-7-((8-methylnaphthalen-1-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 1-bromo-8-methylnaphthalene, and the reaction temperature was further changed from 90°C. to 120°C.

1H-NMR (CDCl3); 1.42 (s, 9H), 2.08-2.14 (m, 1H), 2.47 (brs, 1H), 2.79-2.82 (m, 5H), 2.86 (d, J = 16.0 Hz, 1H), 3.24 (d, J = 17.0 Hz, 1H), 3.75 (s, 3H), 4.78 (brs, 1H), 6.63 (d, J = 2.0 Hz, 1H), 6.76 (dd, J = 3.0, 8.5 Hz, 1H), 6.93 (dd, J = 1.0, 8.0 Hz, 1H), 7.05 (d, J = 8.0 Hz, 1H), 7.24 (d, J = 7.0 Hz, 1H), 7.33-7.37 (m, 2H), 7.62 (dd, J = 1.5, 9.0 Hz, 1H), 7.70 (d, J = 8.0 Hz, 1H).

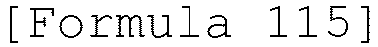

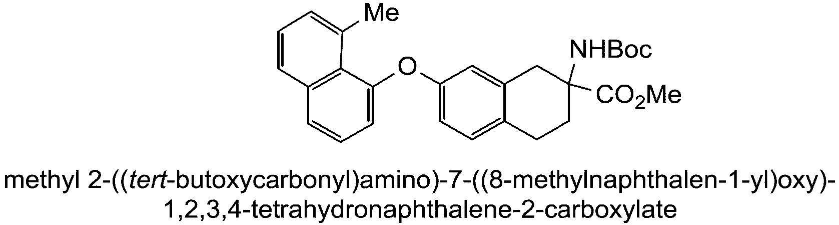

### Step 2 (Steps V-VI)

### 2- Production of carboxy-7-((8-methylnaphthalen-1-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.13-2.19 (m, 1H), 2.38-2.44 (m, 1H), 2.75 (s, 3H), 2.84-2.91 (m, 1H), 2.94 (d, J = 17.0 Hz, 1H), 2.98-3.04 (m, 1H), 3.44 (d, J = 17.0 Hz, 1H), 6.69 (d, J = 2.5 Hz, 1H), 6.78 (dd, J = 3.0, 9.0 Hz, 1H), 6.96 (dd, J = 1.0, 8.0 Hz, 1H), 7.17 (d, J = 8.0 Hz, 1H), 7.24 (d, J = 8.0 Hz, 1H), 7.35-7.39 (m, 2H), 7.68 (dd, J = 1.0, 8.0 Hz, 1H), 7.74 (d, J = 8.0 Hz, 1H).

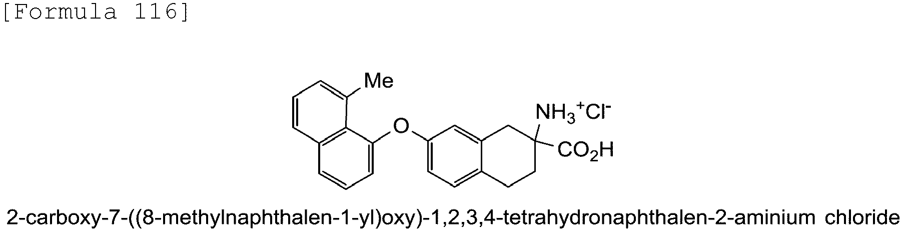

### (Example 1-47)

### 2- Preparation of carboxy-7-((3'-ethyl-[1,1'-biphenyl]-2-yl)oxy)-1,2,3,4-tetrahydronaphthalene-2-aminium chloride (Compound 1-91)

### Step 1 (Steps I-IV)

### Preparation of methyl 2-((tert-butoxycarbonyl)amino)-7-((3'-ethyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-ethyl-1,1'-biphenyl, and the reaction temperature was further changed from 90°C. to 120°C.

1H-NMR (CDCl3); 1.22 (t, J = 8.0 Hz, 3H), 1.42 (s, 9H), 2.05-2.11 (m, 1H), 2.45 (brs, 1H), 2.65 (q, J = 7.5 Hz, 2H), 2.76-2.78 (m, 2H), 2.84 (d, J = 17.0 Hz, 1H), 3.20 (d, J = 17.0 Hz, 1H), 3.75 (s, 3H), 4.75 (brs, 1H), 6.61 (d, J = 2.0 Hz, 1H), 6.74 (dd, J = 2.5, 8.0 Hz, 1H), 6.98-7.00 (m, 2H), 7.13 (d, J = 7.5 Hz, 1H), 7.19 (td, J = 2.0, 7.5 Hz, 1H), 7.25-7.29 (m, 2H), 7.33-7.36 (m, 2H), 7.45 (dd, J = 2.0, 8.0 Hz, 1H).

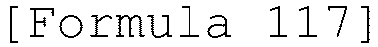

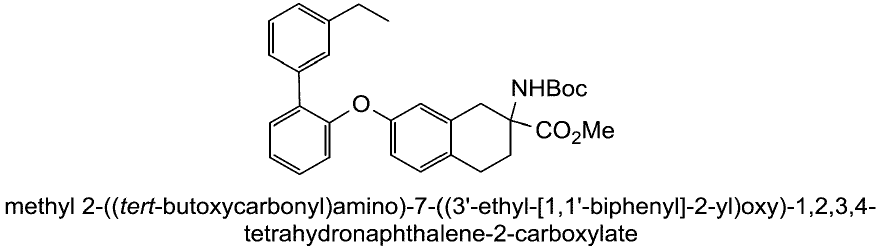

### Step 2 (Steps V-VI)

### 2- Production of carboxy-7-((3'-ethyl-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 1.19 (t, J = 7.5 Hz, 3H), 2.00-2.03 (m, 1H), 2.27-2.34 (m, 1H), 2.61 (q, J = 7.5 Hz, 2H), 2.72-2.78 (m, 1H), 2.79 (d, J = 17.0 Hz, 1H), 2.87-2.93 (m, 1H), 3.39 (d, J = 17.5 Hz, 1H), 6.60 (d, J = 2.5 Hz, 1H), 6.67 (dd, J = 2.5, 8.0 Hz, 1H), 6.98 (dd, J = 1.0, 8.0 Hz, 1H), 7.04 (d, J = 8.5 Hz, 1H), 7.09 (d, J = 7.5 Hz, 1H), 7.19-7.24 (m, 2H), 7.27-7.34 (m, 3H), 7.52 (dd, J = 1.5, 7.5 Hz, 1H).

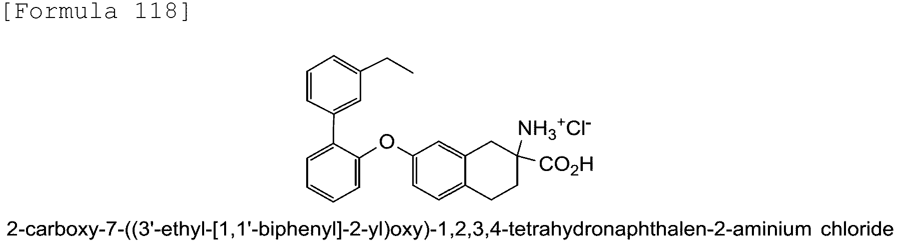

### (Example 1-48)

### 2- Preparation of carboxy-7-((3'-Chloro-4'-fluoro-[1,1'-biphenyl]-2-yl)oxy)-1,2,3,4-tetrahydronaphthalene-2-aminium chloride (Compound 1-93)

### Step 1 (Steps I-IV)

### Preparation of methyl 2-((tert-butoxycarbonyl)amino)-7-((3'-Chloro-4'-fluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-chloro-4'-fluoro-1,1'-biphenyl, and the reaction temperature was further changed from 90°C. to 120°C.

1H-NMR (CDCl3); 1.42 (s, 9H), 2.06-2.12 (m, 1H), 2.43 (brs, 1H), 2.77-2.79 (m, 2H), 2.87 (d, J = 17.0 Hz, 1H), 3.24 (d, J = 17.0 Hz, 1H), 3.75 (s, 3H), 4.76 (brs, 1H), 6.61 (d, J = 2.5 Hz, 1H), 6.72 (dd, J = 2.5, 8.0 Hz, 1H), 6.98 (d, J = 8.0 Hz, 1H), 7.02 (d, J = 8.0 Hz, 1H), 7.12 (t, J = 9.0 Hz, 1H), 7.19 (td, J = 1.5, 7.5 Hz, 1H), 7.30 (td, J = 1.5, 8.0 Hz, 1H), 7.38-7.42 (m, 2H), 7.58 (dd, J = 2.5, 7.5 Hz, 1H).

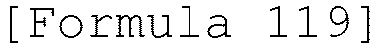

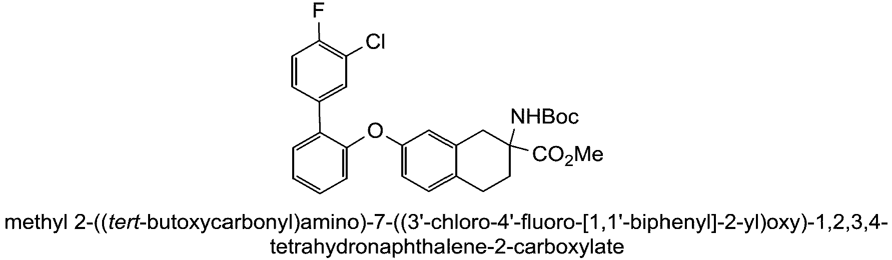

### Step 2 (Steps V-VI)

### 2- Production of carboxy-7-((3'-Chloro-4'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.01-2.04 (m, 1H), 2.28-2.34 (m, 1H), 2.71-2.76 (m, 1H), 2.80 (d, J = 17.5 Hz, 1H), 2.83-2.93 (m, 1H), 3.39 (d, J = 17.0 Hz, 1H), 6.60 (d, J = 2.5 Hz, 1H), 6.66-6.71 (m, 2H), 6.82 (dd, J = 1.0, 6.5 Hz, 1H), 6.91 (t, J = 1.5 Hz, 1H), 6.98 (dd, J = 1.0, 8.0 Hz, 1H), 7.04 (d, J = 8.0 Hz, 1H), 7.15 (t, J = 8.0 Hz, 1H), 7.23 (td, J = 1.0, 7.0 Hz, 1H), 7.31 (td, J = 1.5, 8.0 Hz, 1H), 7.46 (dd, J = 2.0, 7.5 Hz, 1H).

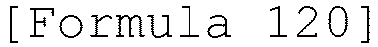

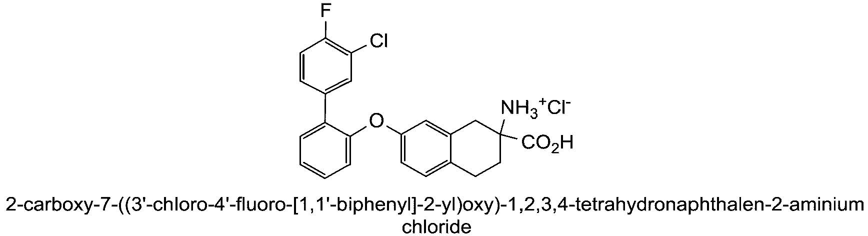

### (Example 1-49)

### 2- Preparation of Carboxy-7-((2'-Chloro-3'-fluoro-[1,1'-biphenyl]-2-yl)oxy)-1,2,3,4-tetrahydronaphthalene-2-aminium chloride (Compound 1-89)

### Step 1 (Steps I-IV)

### Preparation of methyl 2-((tert-butoxycarbonyl)amino)-7-((2'-Chloro-3'-fluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-2-chloro-3-fluoro-1,1'-biphenyl, and the reaction temperature was further changed from 90°C. to 120°C.

1H-NMR (CDCl3); 1.42 (s, 9H), 2.05-2.11 (m, 1H), 2.44 (brs, 1H), 2.75-2.78 (m, 2H), 2.83 (d, J = 16.5 Hz, 1H), 3.20 (d, J = 17.0 Hz, 1H), 3.75 (s, 3H), 4.74 (brs, 1H), 6.61 (d, J = 2.0 Hz, 1H), 6.73 (dd, J = 2.5, 8.0 Hz, 1H), 6.97-6.99 (m, 2H), 7.10-7.12 (m, 2H), 7.16-7.23 (m, 2H), 7.30 (dd, J = 1.5, 7.5 Hz, 1H), 7.35 (td, J = 1.5, 8.0 Hz, 1H).

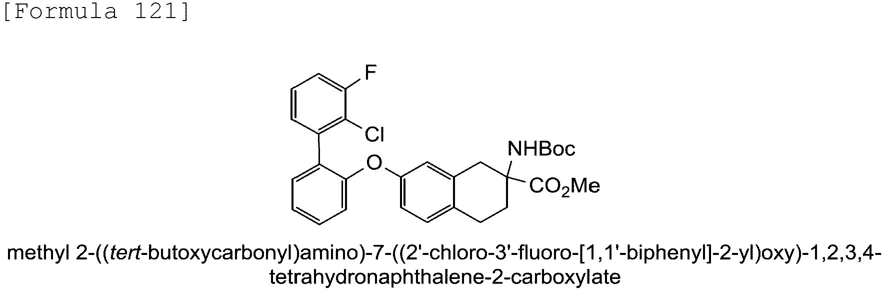

### Step 2 (Steps V-VI)

### 2- Production of carboxy-7-((2'-Chloro-3'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.00-2.04 (m, 1H), 2.29-2.35 (m, 1H), 2.74-2.80 (m, 1H), 2.80 (d, J = 17.0 Hz, 1H), 2.90-2.95 (m, 1H), 3.40 (d, J = 17.0 Hz, 1H), 6.66 (d, J = 1.5 Hz, 1H), 6.71 (dd, J = 2.0, 8.0 Hz, 1H), 6.97 (d, J = 8.0 Hz, 1H), 7.06 (d, J = 8.5 Hz, 1H), 7.13-7.23 (m, 3H), 7.26-7.31 (m, 2H), 7.38 (td, J = 1.0, 7.0 Hz, 1H).

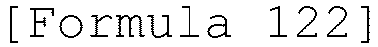

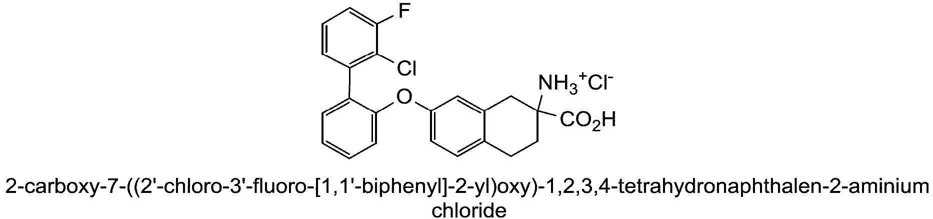

### (Example 1-50)

### 2- Preparation of Carboxy-7-((3'-(Dimethylammonio)-[1,1'-biphenyl]-2-yl)oxy)-1,2,3,4-tetrahydronaphthalene-2-aminium chloride (Compound 1-67).

### Step 1 (Steps I-IV)

### Preparation of methyl 2-((tert-butoxycarbonyl)amino)-7-((3'-(dimethylamino)-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-N,N-dimethyl-[1,1'-biphenyl]-3-amine, and the reaction temperature was further changed from 90°C. to 120°C.

1H-NMR (CDCl3); 1.42 (s, 9H), 2.04-2.11 (m, 1H), 2.46 (brs, 1H), 2.75-2.79 (m, 2H), 2.83 (d, J = 16.5 Hz, 1H), 2.87 (s, 6H), 3.20 (d, J = 17.0 Hz, 1H), 3.75 (s, 3H), 4.76 (brs, 1H), 6.61 (d, J = 2.5 Hz, 1H), 6.69-6.70 (m, 1H), 6.74 (dd, J = 3.0, 9.0 Hz, 1H), 6.86-6.89 (m, 2H), 6.99-7.00 (m, 2H), 7.18-7.29 (m, 3H), 7.47 (dd, J = 2.0, 7.5 Hz, 1H).

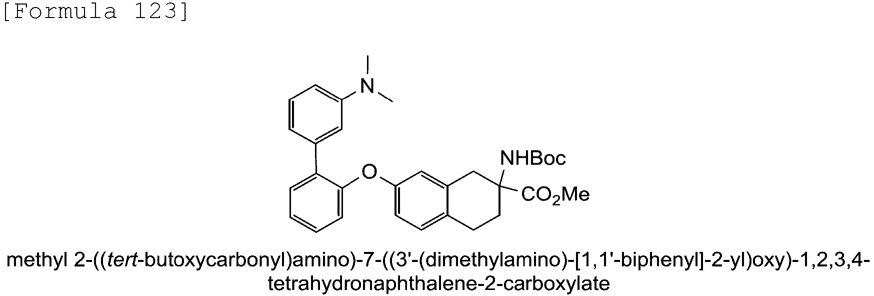

### Step 2 (Steps V-VI)

### 2- Production of carboxy-7-((3'-(Dimethylammonio)-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.12-2.17 (m, 1H), 2.33-2.39 (m, 1H), 2.81-2.88 (m, 1H), 2.96 (d, J = 17.0 Hz, 1H), 2.92-2.98 (m, 1H), 3.28 (s, 6H), 3.40 (d, J = 17.5 Hz, 1H), 6.72 (d, J = 2.5 Hz, 1H), 6.76 (dd, J = 1.5, 8.0 Hz, 1H), 7.04 (d, J = 8.0 Hz, 1H), 7.11 (d, J = 8.5 Hz, 1H), 7.31 (t, J = 7.0 Hz, 1H), 7.42 (td, J = 1.5, 9.0 Hz, 1H), 7.54 (dd, J = 1.5, 8.0 Hz, 1H), 7.58-7.65 (m, 2H), 7.71 (d, J = 7.5 Hz, 1H), 7.89 (s, 1H).

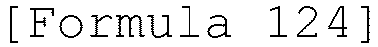

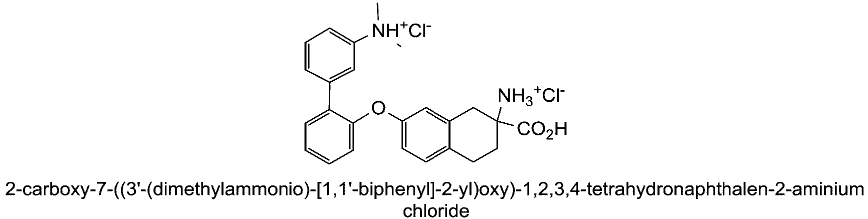

### (Example 1-51)

### Preparation of 7-((2'-Bromo-3-nitro-[1,1'-biphenyl]-2-yl)oxy)-2-carboxy-1,2,3,4-tetrahydronaphthalene-2-aminium chloride (Compound 1-99)

### Step 1 (Steps I-IV)

### Preparation of methyl 7-((2'-Bromo-3-nitro-[1,1'-biphenyl]-2-yl)oxy)-2-((tert-butoxycarbonyl)amino)-1,2,3,4-tetrahydronaphthalene-2-carboxylate methyl 7-((2'-bromo-3-nitro-[1,1'-biphenyl]-2-yl)oxy)-2-((tert-butoxycarbonyl)amino)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2-fluoro-3-nitro-1,1'-biphenyl, and the reaction temperature was further changed from 90°C. to 120°C.

1H-NMR (CDCl3); 1.43 (s, 9H), 1.98-2.02 (m, 1H), 2.43 (brs, 1H), 2.66-2.71 (m, 2H), 2.69 (d, J = 18.0 Hz, 1H), 3.05 (d, J = 17.5 Hz, 1H), 3.74 (s, 3H), 4.69 (brs, 1H), 6.30 (s, 1H), 6.47 (t, J = 8.5 Hz, 1H), 6.79 (d, J = 8.5 Hz, 1H), 7.10-7.24 (m, 3H), 7.40 (t, J = 8.0 Hz, 1H), 7.48-7.56 (m, 2H), 7.96 (dd, J = 1.5, 8.0 Hz, 1H).

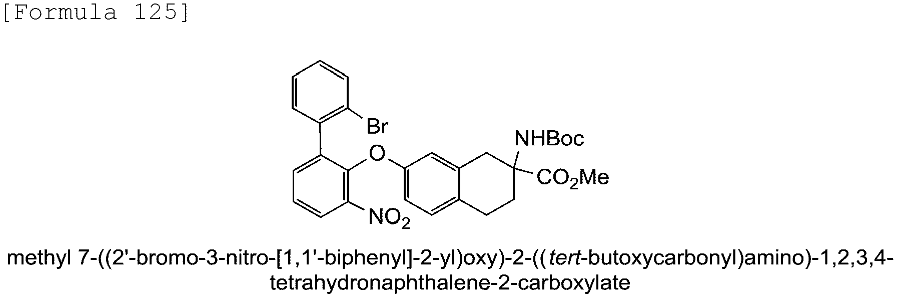

### Step 2 (Steps V-VI)

### Preparation of 7-((2'-Bromo-3-nitro-[1,1'-biphenyl]-2-yl)oxy)-2-carboxy-1,2,3,4-tetrahydronaphthalene-2-aminium chloride

The following compound was synthesized from the compound obtained in Step 1 in the same manner as in Steps 2 and 3 of Example 1-26.

1H-NMR (DMSO-d6); 1.99-2.12 (m, 2H), 2.65-2.75 (m, 2H), 2.87-2.92 (m, 1H), 3.12-3.39 (a proton overlapped with water protons, 1H), 6.45-6.46 (m, 2H), 6.90 (dd, J = 4.5, 9.0 Hz, 1H), 7.19-7.30 (m, 3H), 7.59-7.63 (m, 2H), 7.71-7.73 (m, 1H), 8.15 (dd, J = 1.5, 8.0 Hz, 1H).

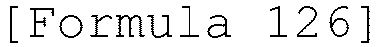

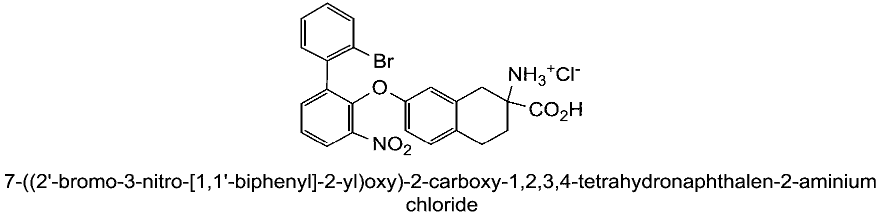

### (Example 1-52)

### 2- Preparation of Carboxy-7-((3'-(methylthio)-[1,1'-biphenyl]-2-yl)oxy)-1,2,3,4-tetrahydronaphthalene-2-aminium chloride (Compound 1-94)

### Step 1 (Steps I-IV)

### Preparation of methyl 2-((tert-butoxycarbonyl)amino)-7-((3'-(methylthio)-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-methylthio-1,1'-biphenyl, and the reaction temperature was further changed from 90°C. to 120°C.

1H-NMR (CDCl3); 1.42 (s, 9H), 2.05-2.11 (m, 1H), 2.41 (s, 3H), 2.46 (brs, 1H), 2.76-2.78 (m, 2H), 2.85 (d, J = 16.5 Hz, 1H), 3.21 (d, J = 17.0 Hz, 1H), 3.75 (s, 3H), 4.76 (brs, 1H), 6.61 (d, J = 2.5 Hz, 1H), 6.73 (dd, J = 3.0, 8.5 Hz, 1H), 6.99-7.01 (m, 2H), 7.18-7.22 (m, 2H), 7.25-7.32 (m, 3H), 7.39 (m, 1H), 7.43 (dd, J = 2.0, 7.5 Hz, 1H).

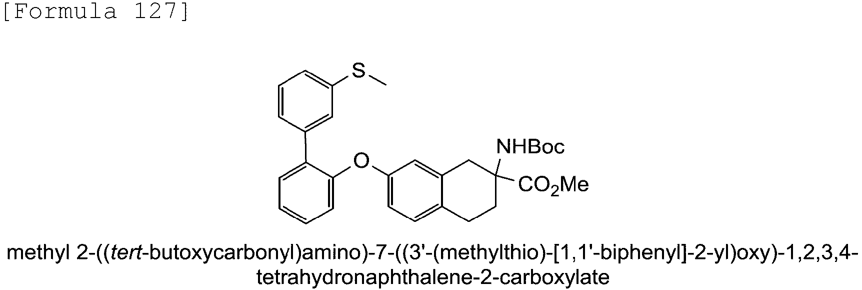

### Step 2 (Steps V-VI)

### 2- Production of carboxy-7-((3'-(methylthio)-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.10-2.15 (m, 1H), 2.33-2.38 (m, 1H), 2.39 (s, 3H), 2.78-2.85 (m, 1H), 2.91 (d, J = 16.5 Hz, 1H), 2.94-2.99 (m, 1H), 3.41 (d, J = 17.0 Hz, 1H), 6.65 (d, J = 2.0 Hz, 1H), 6.71 (dd, J = 2.0, 8.0 Hz, 1H), 7.02 (dd, J = 1.0, 8.0 Hz, 1H), 7.09 (d, J = 8.0 Hz, 1H), 7.15-7.18 (m, 1H), 7.23-7.28 (m, 3H), 7.35 (dd, J = 2.0, 8.0 Hz, 1H), 7.37 (dd, J = 2.0, 3.5 Hz, 1H), 7.46 (dd, J = 1.5, 8.0 Hz, 1H).

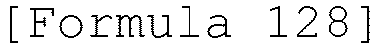

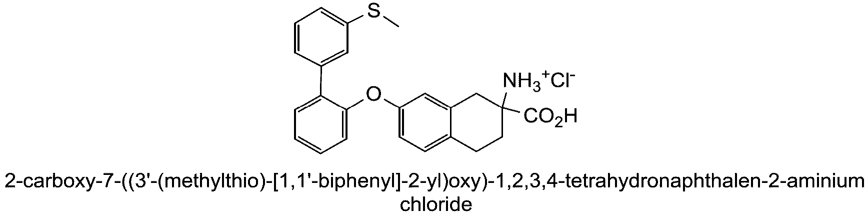

### (Example 1-53)

### 2- Preparation of carboxy-7-((2',3'-dichloro-[1,1'-biphenyl]-2-yl)oxy)-1,2,3,4-tetrahydronaphthalene-2-aminium chloride (Compound 1-100)

### Step 1 (Steps I-IV)

### Preparation of methyl 2-((tert-butoxycarbonyl)amino)-7-((2',3'-dichloro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2,3-dichloro-1,1'-biphenyl, and the reaction temperature was further changed from 90°C. to 120°C.

1H-NMR (CDCl3); 1.42 (s, 9H), 2.05-2.11 (m, 1H), 2.46 (brs, 1H), 2.77-2.78 (m, 2H), 2.83 (d, J = 16.5 Hz, 1H), 3.19 (d, J = 16.5 Hz, 1H), 3.76 (s, 3H), 4.75 (brs, 1H), 6.61 (brs, 1H), 6.73 (brs, 1H), 6.96-6.98 (m, 2H), 7.16-7.22 (m, 3H), 7.28 (dd, J = 2.0, 7.5 Hz, 1H), 7.35 (td, J = 1.5, 8.5 Hz, 1H), 7.41 (dd, J = 2.0, 7.5 Hz, 1H).

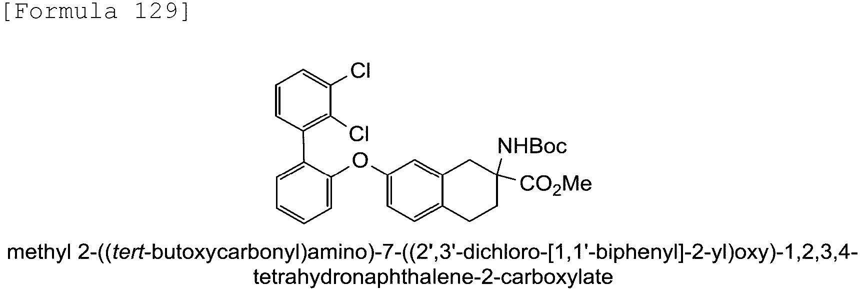

### Step 2 (Steps V-VI)

### 2- Production of carboxy-7-((2',3'-dichloro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.10-2.15 (m, 1H), 2.34-2.40 (m, 1H), 2.79-2.85 (m, 1H), 2.91 (d, J = 17.5 Hz, 1H), 2.94-3.00 (m, 1H), 3.42 (d, J = 17.0 Hz, 1H), 6.69 (d, J = 2.0 Hz, 1H), 6.75 (dd, J = 2.5, 8.0 Hz, 1H), 6.98 (d, J = 8.0 Hz, 1H), 7.09 (d, J = 8.5 Hz, 1H), 7.21-7.26 (m, 3H), 7.30 (dd, J = 2.0, 7.5 Hz, 1H), 7.39 (td, J = 1.7, 8.0 Hz, 1H), 7.47 (dd, J = 3.0, 7.0 Hz, 1H).

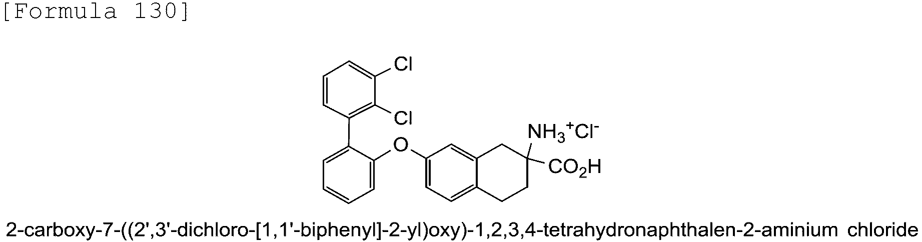

### (Example 1-54)

### 2- Preparation of carboxy-7-((3'-Chloro-2'-fluoro-[1,1'-biphenyl]-2-yl)oxy)-1,2,3,4-tetrahydronaphthalene-2-aminium chloride (Compound 1-101)

### Step 1 (Steps I-IV)

### Preparation of methyl 2-((tert-butoxycarbonyl)amino)-7-((3'-Chloro-2'-fluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-3-chloro-2-fluoro-1,1'-biphenyl, and the reaction temperature was further changed from 90°C. to 120°C.

1H-NMR (CDCl3); 1.42 (s, 9H), 2.06-2.12 (m, 1H), 2.47 (brs, 1H), 2.78-2.79 (m, 2H), 2.85 (d, J = 17.5 Hz, 1H), 3.22 (d, J = 17.0 Hz, 1H), 3.76 (s, 3H), 4.77 (brs, 1H), 6.65 (d, J = 2.5 Hz, 1H), 6.76 (dd, J = 2.5, 8.5 Hz, 1H), 6.95-6.97 (m, 1H), 7.02 (d, J = 9.0 Hz, 1H), 7.09 (td, J = 1.0, 8.0 Hz, 1H), 7.17 (td, J = 1.0, 7.0 Hz, 1H), 7.25-7.29 (m, 1H), 7.32-7.36 (m, 3H).

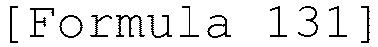

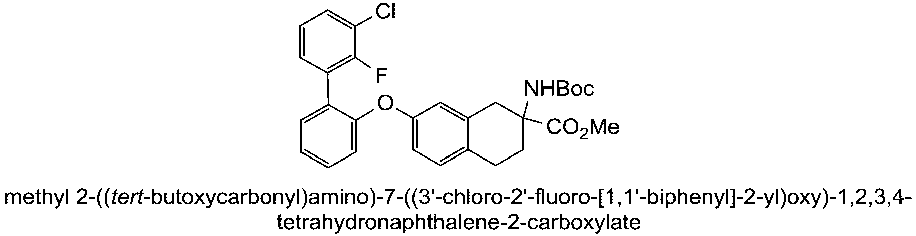

### Step 2 (Steps V-VI)

### 2- Production of carboxy-7-((3'-Chloro-2'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.11-2.16 (m, 1H), 2.35-2.41 (m, 1H), 2.80-2.87 (m, 1H), 2.94 (d, J = 17.3 Hz, 1H), 2.97-3.01 (m, 1H), 3.44 (d, J = 17.2 Hz, 1H), 6.72 (d, J = 2.6 Hz, 1H), 6.77 (dd, J = 2.6, 8.4 Hz, 1H), 6.99 (d, J = 8.1 Hz, 1H), 7.12 (d, J = 8.4 Hz, 1H), 7.15 (t, J = 7.9 Hz, 1H), 7.24 (t, J = 7.5 Hz, 1H), 7.29 (td, J = 1.9, 7.9 Hz, 1H), 7.38-7.43 (m, 3H).

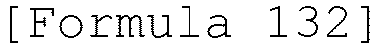

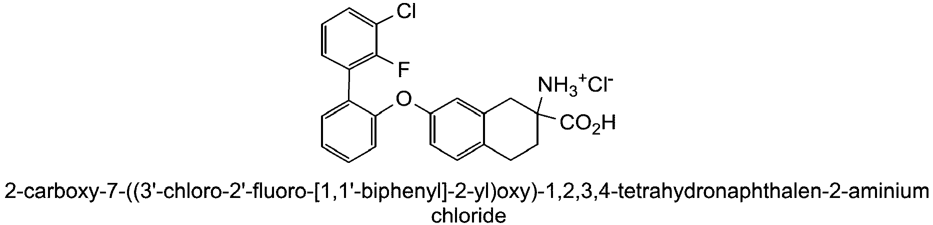

### (Example 1-55)

### 2- Preparation of Carboxy-7-((2'-fluoro-3'-methyl-[1,1'-biphenyl]-2-yl)oxy)-1,2,3,4-tetrahydronaphthalene-2-aminium chloride (Compound 1-102)

### Step 1 (Steps I-IV)

### Preparation of methyl 2-((tert-butoxycarbonyl)amino)-7-((2'-fluoro-3'-methyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2-fluoro-3-methyl-1,1'-biphenyl, and the reaction temperature was further changed from 90°C. to 120°C.

1H-NMR (CDCl3); 1.42 (s, 9H), 2.04-2.11 (m, 1H), 2.28 (s, 3H), 2.46 (brs, 1H), 2.75-2.78 (m, 2H), 2.84 (d, J = 17.0 Hz, 1H), 3.20 (d, J = 17.0 Hz, 1H), 3.75 (s, 3H), 4.77 (brs, 1H), 6.65 (d, J = 2.5 Hz, 1H), 6.77 (dd, J = 2.0, 8.0 Hz, 1H), 6.95 (dd, J = 1.0, 8.0 Hz, 1H), 7.00 (d, J = 8.5 Hz, 1H), 7.03 (d, J = 7.5 Hz, 1H), 7.12-7.19 (m, 3H), 7.28-7.34 (m, 1H), 7.53 (dd, J = 1.0, 8.0 Hz, 1H).

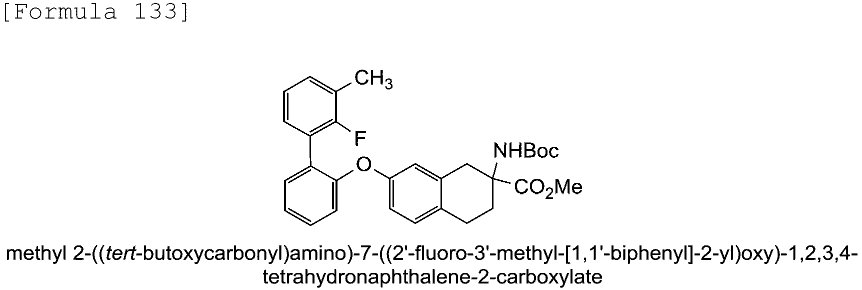

### Step 2 (Steps V-VI)

### 2- Production of carboxy-7-((2'-fluoro-3'-methyl-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.10-2.16 (m, 1H), 2.24 (s, 3H), 2.33-2.40 (m, 1H), 2.79-2.86 (m, 1H), 2.92 (d, J = 17.9 Hz, 1H), 2.94-3.00 (m, 1H), 3.43 (d, J = 17.2 Hz, 1H), 6.69 (d, J = 2.6 Hz, 1H), 6.75 (dd, J = 2.6, 8.2 Hz, 1H), 6.95-6.97 (m, 1H), 7.02 (t, J = 7.5 Hz, 1H), 7.10 (d, J = 8.5 Hz, 1H), 7.12-7.17 (m, 2H), 7.21 (td, J = 0.9, 7.0 Hz, 1H), 7.33-7.37 (m, 2H) .

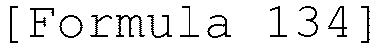

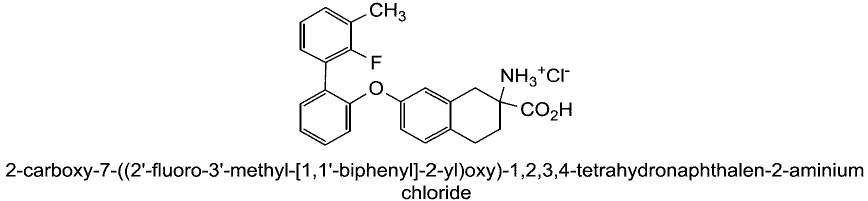

### (Example 1-56)

### 2- Preparation of carboxy-7-((3',4'-Difluoro-[1,1'-Biphenyl]-2-yl)oxy)-1,2,3,4-tetrahydronaphthalene-2-aminium chloride (Compound 1-103)

### Step 1 (Steps I-IV)

### Preparation of methyl 2-((tert-butoxycarbonyl)amino)-7-((3',4'-Difluoro-[1,1'-Biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3',4'-difluoro-1,1'-biphenyl, and the reaction temperature was further changed from 90°C. to 120°C.

1H-NMR (CDCl3); 1.42 (s, 9H), 2.06-2.12 (m, 1H), 2.44 (brs, 1H), 2.77-2.80 (m, 2H), 2.87 (d, J = 16.5 Hz, 1H), 3.24 (d, J = 16.5 Hz, 1H), 3.75 (s, 3H), 4.78 (brs, 1H), 6.61 (d, J = 2.5 Hz, 1H), 6.73 (dd, J = 2.0, 8.0 Hz, 1H), 6.97 (dd, J = 1.5, 9.0 Hz, 1H), 7.02 (d, J = 9.0 Hz, 1H), 7.12-7.21 (m, 2H), 7.24-7.27 (m, 1H), 7.30 (td, J = 1.5, 8.0 Hz, 1H), 7.37-7.41 (m, 2H).

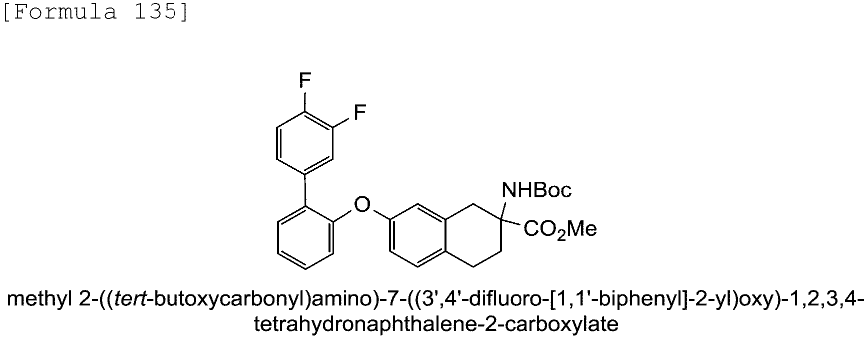

### Step 2 (Steps V-VI)

### 2- Production of carboxy-7-((3',4'-Difluoro-[1,1'-Biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.05-2.10 (m, 1H), 2.31-2.37 (m, 1H), 2.76-2.83 (m, 1H), 2.87 (d, J = 17.5 Hz, 1H), 2.92-2.97 (m, 1H), 3.42 (d, J = 17.5 Hz, 1H), 6.66 (d, J = 2.6 Hz, 1H), 6.71 (dd, J = 2.7, 8.5 Hz, 1H), 7.00 (d, J = 8.4 Hz, 1H), 7.09 (d, J = 8.5 Hz, 1H), 7.20-7.27 (m, 2H), 7.30-7.33 (m, 1H), 7.36 (td, J = 1.8, 7.8 Hz, 1H), 7.41-7.45 (m, 1H), 7.52 (dd, J = 1.7, 7.5 Hz, 1H).

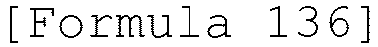

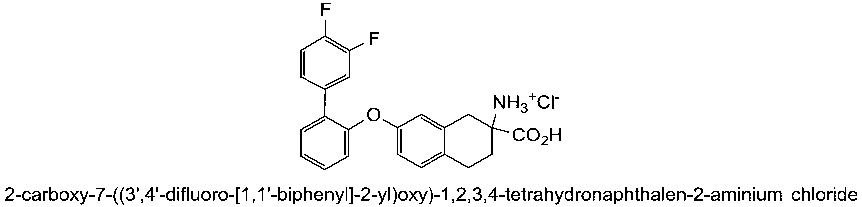

### (Example 1-57)

### 2- Preparation of carboxy-7-((2'-Chloro-3'-methyl-[1,1'-biphenyl]-2-yl)oxy)-1,2,3,4-tetrahydronaphthalene-2-aminium chloride (Compound 1-104)

### Step 1 (Steps I-IV)

### Preparation of methyl 2-((tert-butoxycarbonyl)amino)-7-((2'-Chloro-3'-methyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2-chloro-3-methyl-1,1'-biphenyl, and the reaction temperature was further changed from 90°C. to 120°C.

1H-NMR (CDCl3); 1.42 (s, 9H), 2.04-2.10 (m, 1H), 2.45 (brs, 1H), 2.74-2.77 (m, 2H), 2.82 (d, J = 16.5 Hz, 1H), 3.18 (d, J = 16.6 Hz, 1H), 3.75 (s, 3H), 4.75 (brs, 1H), 6.61-6.62 (m, 1H), 6.73-6.76 (m, 1H), 6.96-6.98 (m, 2H), 7.13-7.19 (m, 4H), 7.28-7.34 (m, 2H).

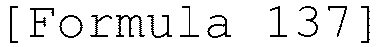

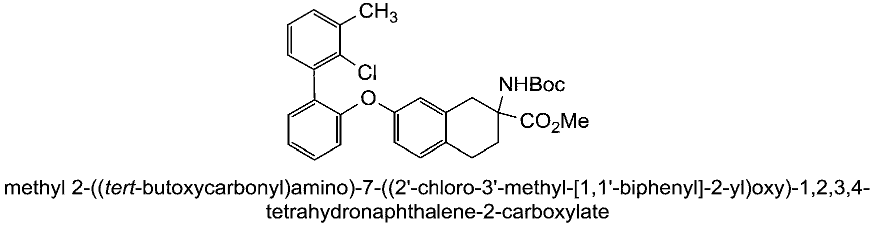

### Step 2 (Steps V-VI)

### 2- Production of carboxy-7-((2'-Chloro-3'-methyl-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.09-2.15 (m, 1H), 2.33-2.39 (m, 1H), 2.37 (s, 3H), 2.78-2.84 (m, 1H), 2.90 (d, J = 17.3 Hz, 1H), 2.93-2.99 (m, 1H), 3.41 (d, J = 17.3 Hz, 1H), 6.67 (d, J = 2.7 Hz, 1H), 6.95-6.97 (m, 1H), 7.06-7.16 (m, 3H), 7.18-7.23 (m, 2H), 7.26-7.29 (m, 2H), 7.36 (td, J = 1.8, 7.8 Hz, 1H).

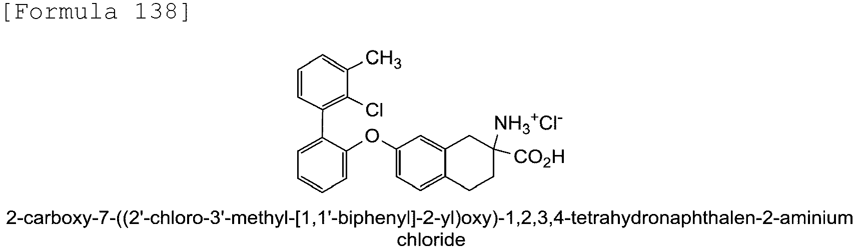

### (Example 1-58)

### 2- Preparation of carboxy-7-((2',3',4'-trifluoro-[1,1'-biphenyl]-2-yl)oxy)-1,2,3,4-tetrahydronaphthalene-2-aminium chloride (Compound 1-105)

### Step 1 (Steps I-IV)

### Preparation of methyl 2-((tert-butoxycarbonyl)amino)-7-((2',3',4'-trifluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-2,3,4-trifluoro-1,1'-biphenyl, and the reaction temperature was further changed from 90°C. to 120°C.

1H-NMR (CDCl3); 1.42 (s, 9H), 2.08-2.13 (m, 1H), 2.45 (brs, 1H), 2.78-2.80 (m, 2H), 2.87 (d, J = 17.0 Hz, 1H), 3.24 (d, J = 16.7 Hz, 1H), 3.76 (s, 3H), 4.77 (brs, 1H), 6.66 (d, J = 2.6 Hz, 1H), 6.77 (dd, J = 2.5, 8.3 Hz, 1H), 6.94-7.01 (m, 2H), 7.03 (d, J = 8.4 Hz, 1H), 7.06-7.11 (m, 1H), 7.18 (td, J = 1.0, 7.5 Hz, 1H), 7.33-7.36 (m, 2H).

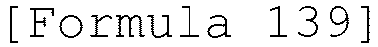

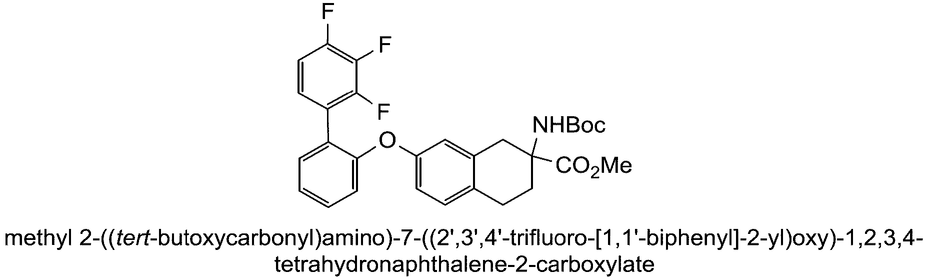

### Step 2 (Steps V-VI)

### 2- Production of carboxy-7-((2',3',4'-trifluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.03-2.07 (m, 1H), 2.30-2.36 (m, 1H), 2.76-2.81 (m, 1H), 2.85 (d, J = 17.8 Hz, 1H), 2.91-2.97 (m, 1H), 3.42 (d, J = 17.2 Hz, 1H), 6.68 (d, J = 2.6 Hz, 1H), 6.73 (dd, J = 2.6, 8.5 Hz, 1H), 6.96 (d, J = 8.3 Hz, 1H), 7.09 (d, J = 8.6 Hz, 1H), 7.12-7.18 (m, 2H), 7.22 (m, 1H), 7.37-7.38 (m, 2H).

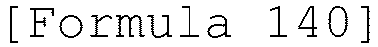

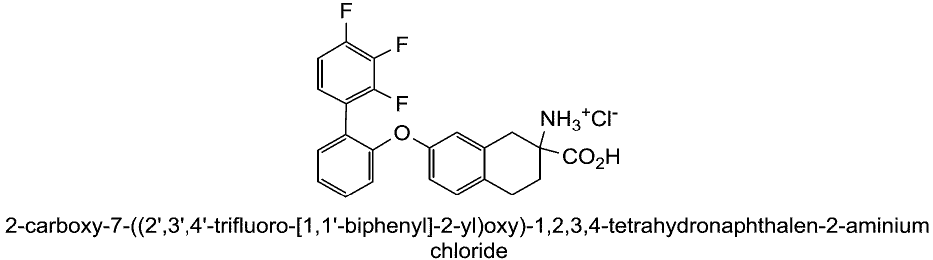

### (Example 1-59)

### 2- Preparation of Carboxy-7-((2',4'-difluoro-[1,1'-biphenyl]-2-yl)oxy)-1,2,3,4-tetrahydronaphthalene-2-aminium chloride (Compound 1-106)

### Step 1 (Steps I-IV)

### Preparation of methyl 2-((tert-butoxycarbonyl)amino)-7-((2',4'-difluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2'-bromo-2,4-difluoro-1,1'-biphenyl, and the reaction temperature was further changed from 90°C. to 120°C.

1H-NMR (CDCl3); 1.42 (s, 9H), 2.06-2.12 (m, 1H), 2.46 (brs, 1H), 2.77-2.89 (m, 2H), 2.86 (d, J = 16.9 Hz, 1H), 3.22 (d, J = 17.0 Hz, 1H), 3.75 (s, 3H), 4.77 (brs, 1H), 6.64 (d, J = 2.5 Hz, 1H), 6.76 (dd, J = 2.6, 8.4 Hz, 1H), 6.84 (td, J = 2.4, 9.3 Hz, 1H), 6.87-6.91 (m, 1H), 6.96 (dd, J = 0.3, 7.7 Hz, 1H), 7.01 (d, J = 8.5 Hz, 1H), 7.17 (td, J = 0.5, 7.5 Hz, 1H), 7.30-7.36 (m, 3H).

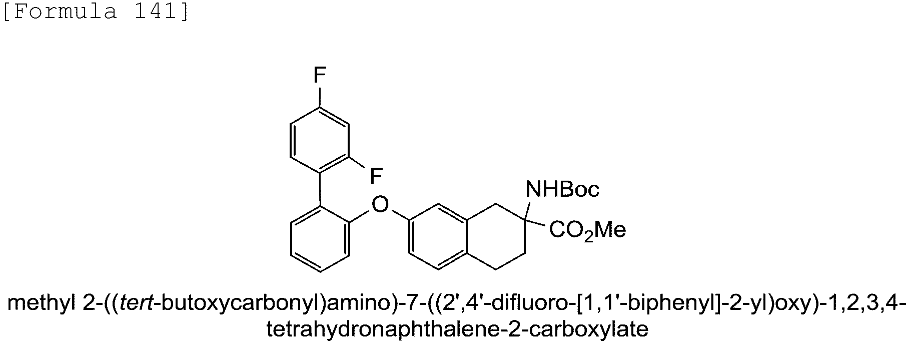

### Step 2 (Steps V-VI)

### 2- Production of carboxy-7-((2',4'-difluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.11-2.16 (m, 1H), 2.34-2.40 (m, 1H), 2.80-2.86 (m, 1H), 2.93 (d, J = 17.3 Hz, 1H), 2.96-3.00 (m, 1H), 3.42 (d, J = 17.2 Hz, 1H), 6.68 (d, J = 2.6 Hz, 1H), 6.74 (dd, J = 2.7, 8.5 Hz, 1H), 6.91-6.98 (m, 3H), 7.10 (d, J = 8.3 Hz, 1H), 7.23 (t, J = 7.5 Hz, 1H), 7.35-7.40 (m, 3H).

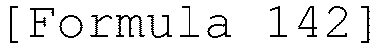

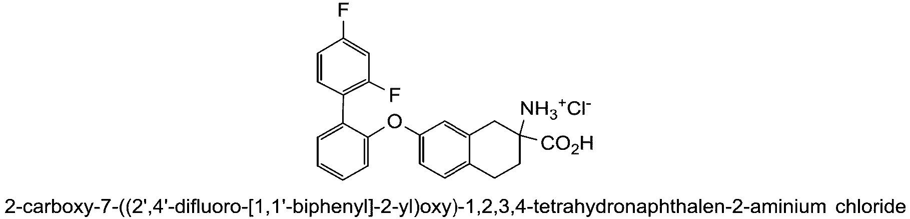

### (Example 1-60)

### 2- Preparation of carboxy-7-((8-methoxynaphthalen-1-yl)oxy)-1,2,3,4-tetrahydronaphthalene-2-aminium chloride (Compound 1-107)

### 2-carboxy-7-((8-methoxynaphthalen-1-yl)oxy)-1,2,3,4-tetrahydronaphthalen-2-aminium chloride

### Step 1 (Steps I-IV)

### Preparation of methyl 2-((tert-butoxycarbonyl)amino)-7-((8-methoxynaphthalen-1-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 1-bromo-8-methoxynaphthalene, and the reaction temperature was further changed from 90°C. to 120°C.

1H-NMR (CDCl3); 1.41 (s, 9H), 2.08-2.12 (m, 1H), 2.45 (brs, 1H), 2.77-2.79 (m, 2H), 2.83 (d, J = 16.5 Hz, 1H), 3.20 (d, J = 16.5 Hz, 1H), 3.74 (s, 3H), 4.79 (brs, 1H), 6.53 (d, J = 2.0 Hz, 1H), 6.68 (dd, J = 2.0, 9.0 Hz, 1H), 6.80 (d, J = 8.0 Hz, 1H), 6.99 (d, J = 8.5 Hz, 1H), 7.06 (dd, J = 1.5, 7.5 Hz, 1H), 7.37-7.47 (m, 3H), 7.64 (d, J = 1.0, 8.0 Hz, 1H).

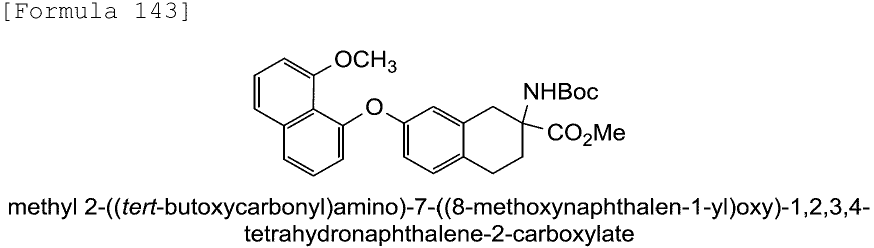

### Step 2 (Steps V-VI)

### 2- Production of carboxy-7-((8-methoxynaphthalen-1-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.11-2.16 (m, 1H), 2.34-2.40 (m, 1H), 2.81-2.87 (m, 1H), 2.88 (d, J = 17.4 Hz, 1H), 2.94-3.00 (m, 1H), 3.39 (d, J = 17.2 Hz, 1H), 3.65 (s, 3H), 6.52 (d, J = 2.7 Hz, 1H), 6.67 (dd, J = 2.7, 8.5 Hz, 1H), 7.04 (dd, J = 0.9, 7.4 Hz, 1H), 7.09 (d, J = 8.5 Hz, 1H), 7.38-7.49 (m, 3H), 7.70 (dd, J = 0.8, 8.0 Hz, 1H).

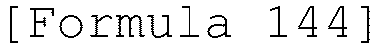

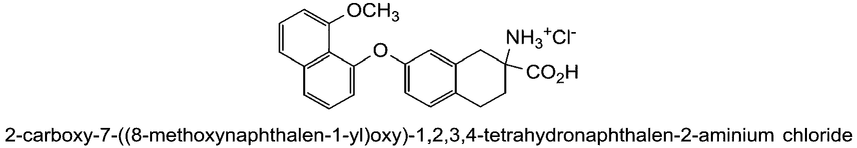

### (Example 1-61)

### 2- Preparation of carboxy-7-((8-phenylnaphthalen-1-yl)oxy)-1,2,3,4-tetrahydronaphthalene-2-aminium chloride (Compound 1-108)

### 2-carboxy-7-((8-phenylnaphthalen-1-yl)oxy)-1,2,3,4-tetrahydronaphthalen-2-aminium chloride

### Step 1 (Steps I-IV)

### Preparation of methyl 2-((tert-butoxycarbonyl)amino)-7-((8-phenylnaphthalen-1-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 1-bromo-8-phenylnaphthalene, and the reaction temperature was further changed from 90°C. to 120°C.

1H-NMR (CDCl3); 1.43 (s, 9H), 2.01-2.07 (m, 1H), 2.41 (brs, 1H), 2.65-2.71 (m, 3H), 3.05 (d, J = 17.0 Hz, 1H), 3.76 (s, 3H), 4.73 (brs, 1H), 5.95 (d, J = 2.0 Hz, 1H), 6.07 (dd, J = 3.0, 9.0 Hz, 1H), 6.75 (d, J = 8.5 Hz, 1H), 7.05 (dd, J = 1.5, 7.5 Hz, 1H), 7.14-7.20 (m, 5H), 7.25-7.27 (m, 1H), 7.44-7.52 (m, 2H), 7.77 (dd, J = 1.0, 8.5 Hz, 1H), 7.90 (dd, J = 1.0, 8.5 Hz, 1H).

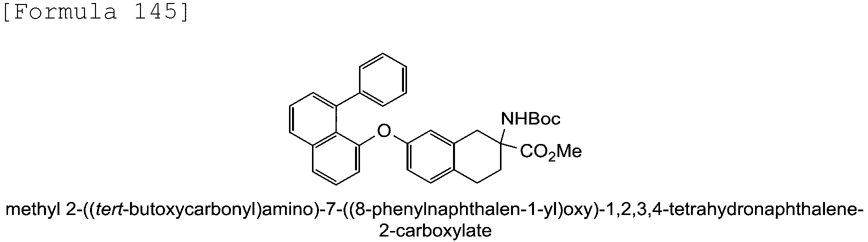

### Step 2 (Steps V-VI)

### 2- Production of carboxy-7-((8-phenylnaphthalen-1-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.07-2.12 (m, 1H), 2.30-2.36 (m, 1H), 2.73-2.80 (m, 1H), 2.77 (d, J = 17.1 Hz, 1H), 2.87-2.93 (m, 1H), 3.27 (d, J = 17.2 Hz, 1H), 6.05 (d, J = 2.7 Hz, 1H), 6.15 (dd, J = 2.7, 8.5 Hz, 1H), 6.87 (d, J = 8.5 Hz, 1H), 6.99 (dd, J = 1.0, 7.5 Hz, 1H), 7.11-7.16 (m, 5H), 7.22 (dd, J = 1.2, 7.3 Hz, 1H), 7.45-7.53 (m, 2H), 7.80 (dd, J = 0.8, 8.5 Hz, 1H), 7.94 (dd, J = 1.0, 8.3 Hz, 1H).

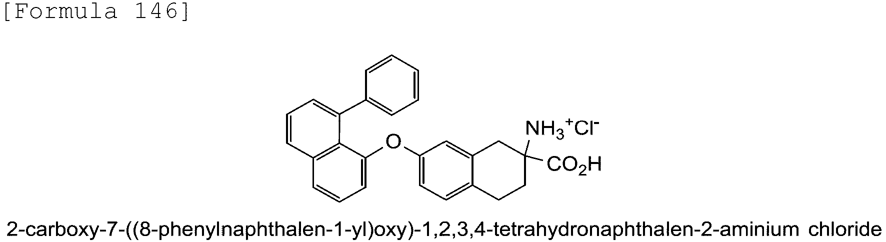

### (Example 1-62)

### 2- Preparation of carboxy-7-((3'-vinyl-[1,1'-biphenyl]-2-yl)oxy)-1,2,3,4-tetrahydronaphthalene-2-aminium chloride (Compound 1-110).

### Step 1 (Steps I-IV)

### Preparation of methyl 2-((tert-butoxycarbonyl)amino)-7-((3'-vinyl-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-vinyl-1,1'-biphenyl, and the reaction temperature was further changed from 90°C. to 120°C.

1H-NMR (CDCl3); 1.42 (s, 9H), 2.05-2.11 (m, 1H), 2.46 (brs, 1H), 2.75-2.78 (m, 2H), 2.84 (d, J = 17.0 Hz, 1H), 3.21 (d, J = 16.8 Hz, 1H), 3.75 (s, 3H), 4.76 (brs, 1H), 5.23 (d, J = 11.1 Hz, 1H), 5.71 (d, J = 17.3 Hz, 1H), 6.62 (d, J = 2.6 Hz, 1H), 6.69-6.75 (m, 2H), 6.99-7.01 (m, 2H), 7.20 (td, J = 1.1, 7.5 Hz, 1H), 7.28-7.35 (m, 3H), 7.43 (dt, J = 1.6, 7.1 Hz, 1H), 7.46 (dd, J = 1.8, 7.5 Hz, 1H), 7.56 (m, 1H).

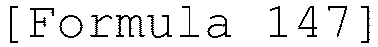

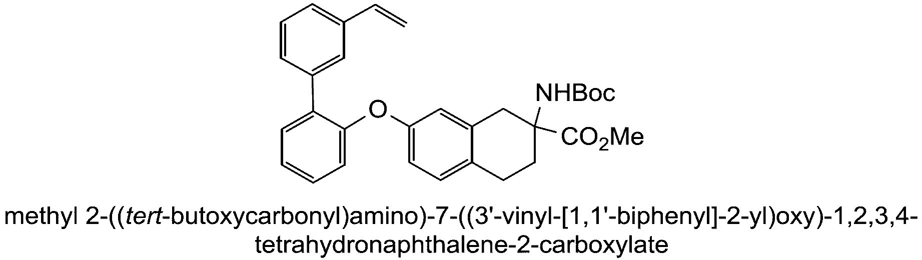

### Step 2 (Steps V-VI)

### 2- Production of carboxy-7-((3'-vinyl-[1,1'-biphenyl]-2-yl)oxy)-1,2,3,4-tetrahydronaphthalene-2-aminium chloride The following compound was synthesized from the compound obtained in Step 1 in the same manner as in Steps 2 and 3 of Example 1-26.

1H-NMR (CD3OD); 2.01-2.06 (m, 1H), 2.28-2.34 (m, 1H), 2.72-2.77 (m, 1H), 2.79 (d, J = 17.8 Hz, 1H), 2.83-2.94 (m, 1H), 3.39 (d, J = 17.4 Hz, 1H), 5.20 (d, J = 10.9 Hz, 1H), 5.72 (d, J = 17.7 Hz, 1H), 6.63 (d, J = 2.6 Hz, 1H), 6.67-6.73 (m, 2H), 6.99-7.02 (m, 1H), 7.05 (d, J = 8.4 Hz, 1H), 7.23-7.43 (m, 5H), 7.45-7.47 (m, 1H), 7.55 (s, 1H).

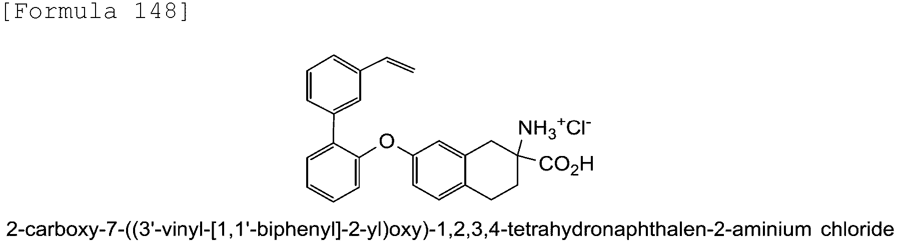

### (Example 1-63)

### 2- Preparation of carboxy-7-((2',3',5'-trifluoro-[1,1'-biphenyl]-2-yl)oxy)-1,2,3,4-tetrahydronaphthalene-2-aminium chloride (Compound 1-111).

### Step 1 (Steps I-IV)

### Preparation of methyl 2-((tert-butoxycarbonyl)amino)-7-((2',3',5'-trifluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-2,3,5-trifluoro-1,1'-biphenyl, and the reaction temperature was further changed from 90°C. to 120°C.

1H-NMR (CDCl3); 1.42 (s, 9H), 2.07-2.13 (m, 1H), 2.46 (brs, 1H), 2.78-2.81 (m, 2H), 2.88 (d, J = 16.5 Hz, 1H), 3.24 (d, J = 16.5 Hz, 1H), 3.76 (s, 3H), 4.78 (brs, 1H), 6.68 (d, J = 2.5 Hz, 1H), 6.78 (dd, J = 2.5, 9.0 Hz, 1H), 6.87-6.96 (m, 3H), 7.04 (d, J = 8.0 Hz, 1H), 7.18 (td, J = 1.0, 7.5 Hz, 1H), 7.33-7.37 (m, 2H).

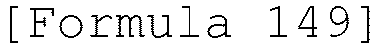

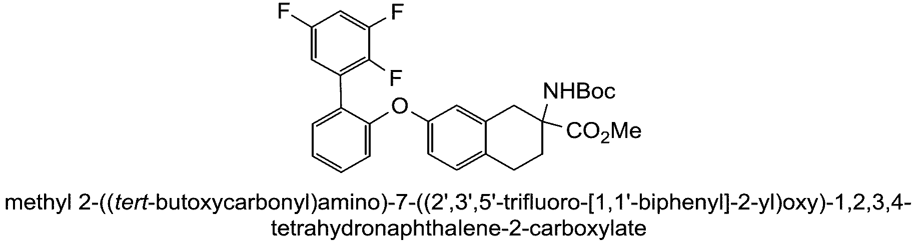

### Step 2 (Steps V-VI)

### 2- Production of carboxy-7-((2',3',5'-trifluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.12-2.17 (m, 1H), 2.36-2.42 (m, 1H), 2.81-2.88 (m, 1H), 2.95 (d, J = 18.0 Hz, 1H), 2.98-3.02 (m, 1H), 3.45 (d, J = 17.0 Hz, 1H), 6.74 (d, J = 2.0 Hz, 1H), 6.78 (dd, J = 2.0, 8.5 Hz, 1H), 6.96-7.00 (m, 2H), 7.09-7.15 (m, 2H), 7.26 (td, J = 1.0, 8.5 Hz, 1H), 7.41-7.44 (m, 2H).

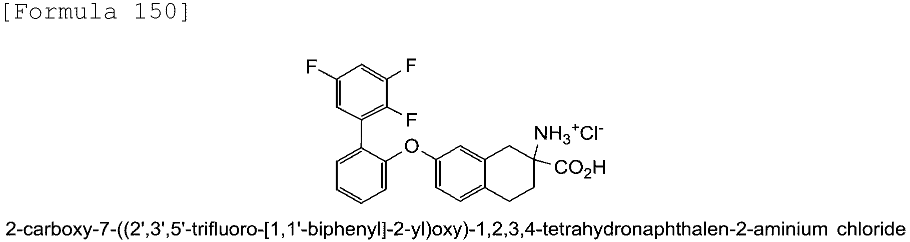

### (Example 1-64)

### 2- Preparation of carboxy-7-((2',3',4',5',6'-pentafluoro-[1,1'-biphenyl]-2-yl)oxy)-1,2,3,4-tetrahydronaphthalene-2-aminium chloride (Compound 1-113).

### Step 1 (Steps I-IV)

### Preparation of methyl 2-((tert-butoxycarbonyl)amino)-7-((2',3',4',5',6'-pentafluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-2,3,4,5,6-pentafluoro-1,1'-biphenyl, and the reaction temperature was further changed from 90°C. to 120°C.

1H-NMR (CDCl3); 1.42 (s, 9H), 2.09-2.15 (m, 1H), 2.45 (brs, 1H), 2.81-2.84 (m, 2H), 2.97 (d, J = 16.5 Hz, 1H), 3.32 (d, J = 16.5 Hz, 1H), 3.77 (s, 3H), 4.81 (brs, 1H), 6.73 (d, J = 2.5 Hz, 1H), 6.85 (dd, J = 2.0, 8.5 Hz, 1H), 7.10 (d, J = 8.0 Hz, 1H), 7.35-7.38 (m, 2H), 7.44-7.47 (m, 1H), 7.75 (d, J = 8.0 Hz, 1H).

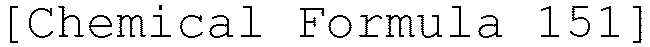

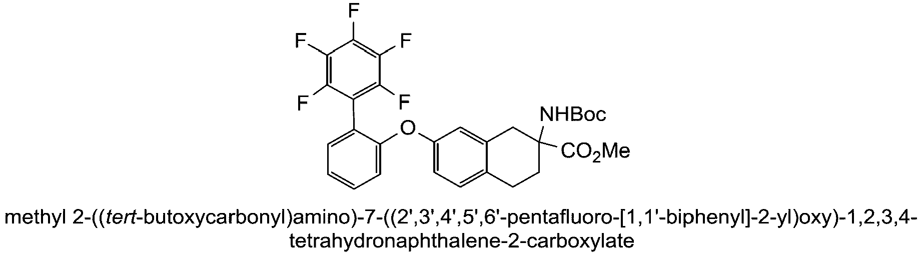

### Step 2 (Steps V-VI)

### 2- Production of carboxy-7-((2',3',4',5',6'-pentafluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.15-2.20 (m, 1H), 2.40-2.46 (m, 1H), 2.86-2.93 (m, 1H), 3.04 (d, J = 17.0 Hz, 1H), 3.03-3.08 (m, 1H), 3.53 (d, J = 17.0 Hz, 1H), 6.93-6.96 (m, 2H), 7.24 (d, J = 8.5 Hz, 1H), 7.43-7.48 (m, 2H), 7.53 (td, J = 1.0, 8.0 Hz, 1H), 7.80-7.81 (m, 1H).

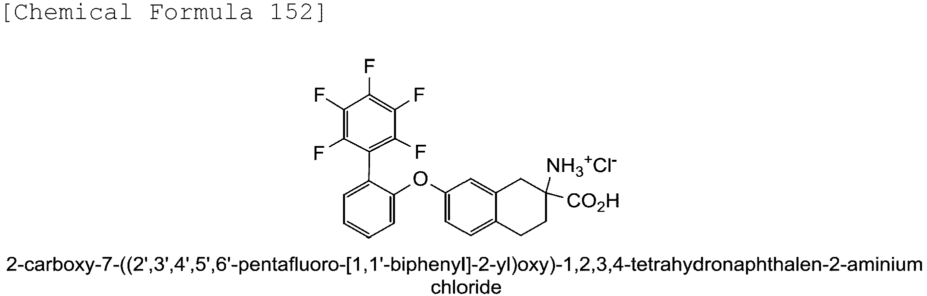

### (Example 1-65)

### 2- Preparation of Carboxy-7-((3',5'-difluoro-[1,1'-biphenyl]-2-yl)oxy)-1,2,3,4-tetrahydronaphthalene-2-aminium chloride (Compound 1-114).

### Step 1 (Steps I-IV)

### Preparation of methyl 2-((tert-butoxycarbonyl)amino)-7-((3',5'-difluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3',5'-difluoro-1,1'-biphenyl, and the reaction temperature was further changed from 90°C. to 120°C.

1H-NMR (CDCl3); 1.42 (s, 9H), 2.06-2.13 (m, 1H), 2.45 (brs, 1H), 2.78-2.80 (m, 2H), 2.87 (d, J = 16.5 Hz, 1H), 3.24 (d, J = 17.0 Hz, 1H), 3.76 (s, 3H), 4.78 (brs, 1H), 6.63 (d, J = 2.0 Hz, 1H), 6.72-6.77 (m, 2H), 6.97 (dd, J = 1.0, 8.0 Hz, 1H), 7.03 (d, J = 8.0 Hz, 1H), 7.07-7.12 (m, 2H), 7.20 (td, J = 1.5, 7.5 Hz, 1H), 7.32 (td, J = 2.0, 8.5 Hz, 1H), 7.41 (dd, J = 2.0, 8.5 Hz, 1H).

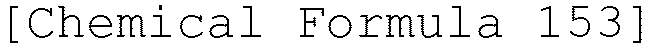

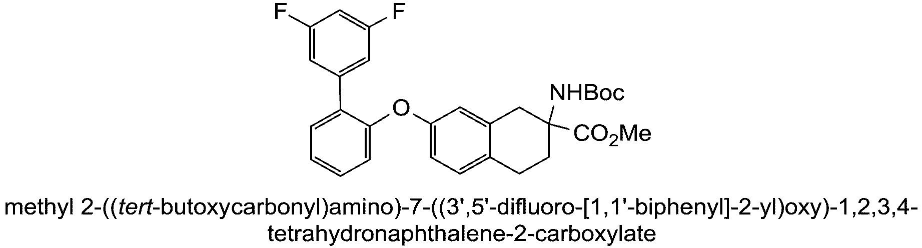

### Step 2 (Steps V-VI)

### 2- Production of carboxy-7-((3',5'-difluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.09-2.12 (m, 1H), 2.33-2.39 (m, 1H), 2.78-2.85 (m, 1H), 2.90 (d, J = 18.0 Hz, 1H), 2.94-3.00 (m, 1H), 3.43 (d, J = 17.5 Hz, 1H), 6.69 (brs, 1H), 6.74 (dd, J = 2.5, 8.5 Hz, 1H), 6.84-6.89 (m, 1H), 7.01 (d, J = 8.0 Hz, 1H), 7.11-7.15 (m, 3H), 7.27 (t, J = 7.5 Hz, 1H), 7.39 (td, J = 1.0, 9.0 Hz, 1H), 7.49 (dd, J = 1.0, 8.0 Hz, 1H).

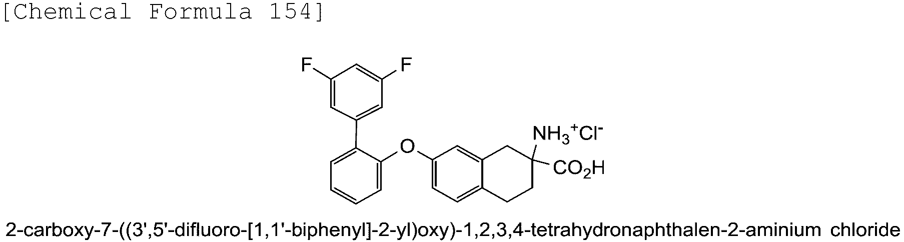

### (Example 1-66)

### 2- Preparation of Carboxy-7-((3'-chloro-5'-fluoro-[1,1'-biphenyl]-2-yl)oxy)-1,2,3,4-tetrahydronaphthalene-2-aminium chloride (Compound 1-115).

### Step 1 (Steps I-IV)

### Preparation of methyl 2-((tert-butoxycarbonyl)amino)-7-((3'-chloro-5'-fluoro-[1,1'-biphenyl]-2-yl)oxy)-1,2,3,4-tetrahydronaphthalene-2-carboxylate

The following compounds were obtained in the same manner as in Steps 1 to 4 of Example 1-1, except that 2-iodonaphthalene in Step 4 of Example was changed to 2-bromo-3'-chloro-5'-fluoro-1,1'-biphenyl, and the reaction temperature was further changed from 90°C. to 120°C.

1H-NMR (CDCl3); 1.42 (s, 9H), 2.06-2.13 (m, 1H), 2.45 (brs, 1H), 2.78-2.80 (m, 2H), 2.87 (d, J = 17.0 Hz, 1H), 3.24 (d, J = 17.0 Hz, 1H), 3.76 (s, 3H), 4.78 (brs, 1H), 6.63 (d, J = 2.0 Hz, 1H), 6.74 (dd, J = 2.0, 8.0 Hz, 1H), 6.97 (d, J = 7.5 Hz, 1H), 7.01-7.05 (m, 2H), 7.18-7.21 (m, 2H), 7.30-7.34 (m, 2H), 7.40 (dd, J = 2.0, 7.5 Hz, 1H).

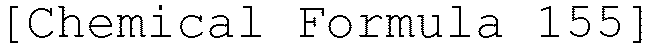

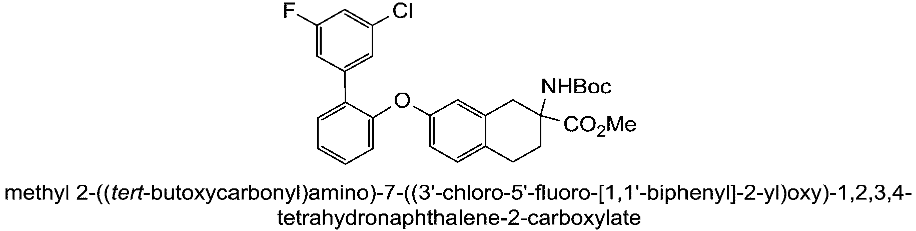

### Step 2 (Steps V-VI)

### 2- Production of carboxy-7-((3'-chloro-5'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.11-2.16 (m, 1H), 2.35-2.41 (m, 1H), 2.79-2.86 (m, 1H), 2.93 (d, J = 18.0 Hz, 1H), 2.97-3.01 (m, 1H), 3.44 (d, J = 17.5 Hz, 1H), 6.70 (d, J = 2.0 Hz, 1H), 6.75 (dd, J = 3.0, 8.5 Hz, 1H), 7.02 (d, J = 8.0 Hz, 1H), 7.11-7.14 (m, 2H), 7.23 (d, J = 9.5 Hz, 1H), 7.28 (t, J = 7.0 Hz, 1H), 7.38-7.42 (m, 2H), 7.49 (dd, J = 1.0, 7.0 Hz, 1H).

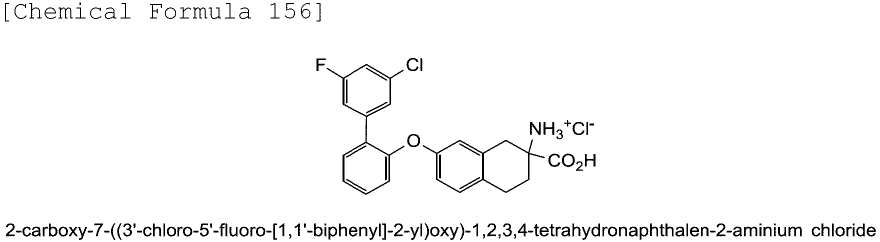

### (Example 1-67)

### 2- Preparation of carboxy-7-((2',3'-dichloro-5'-fluoro-[1,1'-biphenyl]-2-yl)oxy)-1,2,3,4-tetrahydronaphthalene-2-aminium chloride (Compound 1-116).

### Step 1 (Steps I-IV)

### Preparation of methyl 2-((tert-butoxycarbonyl)amino)-7-((2',3'-dichloro-5'-fluoro-[1,1'-biphenyl]-2-yl)oxy)-1,2,3,4-tetrahydronaphthalene-2-carboxylate

The following compound was obtained in the same manner as in Steps 1 to 4 of Example 1-1, except that 2-iodonaphthalene in Step 4 of Example was changed to 2'-bromo-2,3-dichloro-5-fluoro-1,1'-biphenyl, and the reaction temperature was further changed from 90°C. to 120°C.

1H-NMR (CDCl3); 1.42 (s, 9H), 2.05-2.12 (m, 1H), 2.46 (brs, 1H), 2.78-2.79 (m, 2H), 2.85 (d, J = 16.5 Hz, 1H), 3.22 (d, J = 16.5 Hz, 1H), 3.76 (s, 3H), 4.77 (brs, 1H), 6.63 (brs, 1H), 6.75 (d, J = 7.5 Hz, 1H), 6.96 (d, J = 8.0 Hz, 1H), 6.99-7.02 (m, 2H), 7.15-7.20 (m, 2H), 7.26-7.27 (m, 1H), 7.36 (td, J = 2.0, 8.0 Hz, 1H).

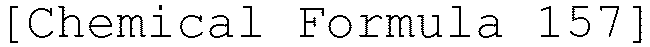

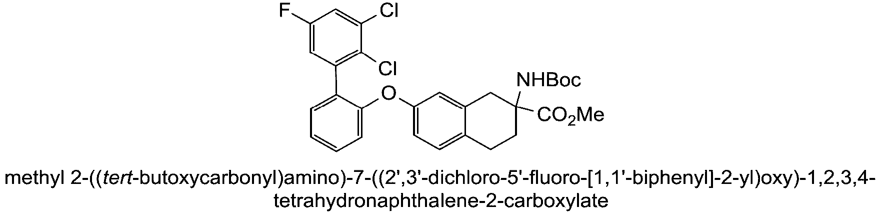

### Step 2 (Steps V-VI)

### 2- Production of carboxy-7-((2',3'-dichloro-5'-fluoro-[1,1'-biphenyl]-2-yl)oxy)-1,2,3,4-tetrahydronaphthalene-2-aminium chloride

The following compound was synthesized from the compound obtained in Step 1 in the same manner as in Steps 2 and 3 of Example 1-26.

1H-NMR (CD3OD); 2.11-2.16 (m, 1H), 2.35-2.41 (m, 1H), 2.80-2.87 (m, 1H), 2.93 (d, J = 18.0 Hz, 1H), 2.97-3.01 (m, 1H), 3.44 (d, J = 17.5 Hz, 1H), 6.72 (brs, 1H), 6.77 (dd, J = 2.5, 8.0 Hz, 1H), 6.98 (d, J = 8.5 Hz, 1H), 7.09-7.13 (m, 2H), 7.24 (t, J = 8.0 Hz, 1H), 7.32 (dd, J = 2.0, 7.5 Hz, 1H), 7.37 (dd, J = 2.5, 8.0 Hz, 1H), 7.41 (td, J = 1.5, 8.0 Hz, 1H).

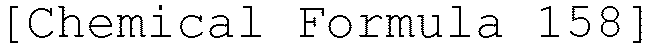

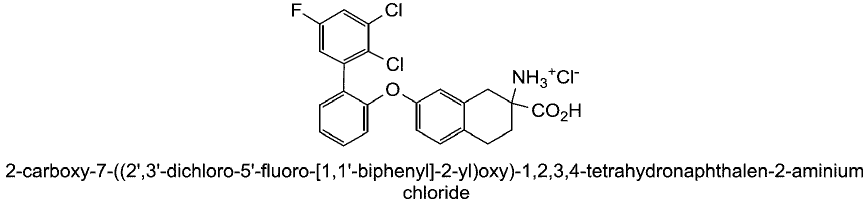

### (Example 1-68)

### 2- Preparation of carboxy-7-(4-nitrophenoxy)-1,2,3,4-tetrahydronaphthalene-2-aminium chloride (Compound 1-117) Step 1 (Steps I-IV)

### Preparation of methyl 2-((tert-butoxycarbonyl)amino)-7-(4-nitrophenoxy)-1,2,3,4-tetrahydronaphthalene-2-carboxylate

A phenolic substance (2.00 g, 6.22 mmol) obtained by Step 3 of Example 1-1, 1-fluoro-4-nitrobenzene (1.05 g, 7.47 mmol), cesium carbonate (4.05 g, 12.4 mmol), and DMSO (15 mL) were added. The reaction was allowed to proceed at 100°C. for about 1 hour. The reaction was then cooled in an ice bath and ethyl acetate (50 mL) and distilled water (50 mL) were added. After stirring for about 5 minutes, the mixture was filtered through Celite, and the obtained filtrate was transferred to a separatory funnel. The aqueous phase was further extracted with ethyl acetate (50 mL), and the resulting organic phases were combined and washed with saturated brine (50 mL). The mixture was dried over anhydrous magnesium sulfate, removed by filtration, and the residue obtained by filtration was purified by silica gel column chromatography to give the object product (2.59 g, yield 94%).

1H-NMR (CDCl3); 1.43 (s, 9H), 2.12-2.18 (m, 1H), 2.45 (brs, 1H), 2.85-2.88 (m, 2H), 2.98 (d, J = 17.2 Hz, 1H), 3.35 (d, J = 17.0 Hz, 1H), 3.77 (s, 3H), 4.83 (brs, 1H), 6.81 (d, J = 2.5 Hz, 1H), 6.89 (dd, J = 2.5, 8.3 Hz, 1H), 6.99-7.02 (m, 2H), 7.17 (d, J = 8.3 Hz, 1H), 8.18-8.21 (m, 2H).

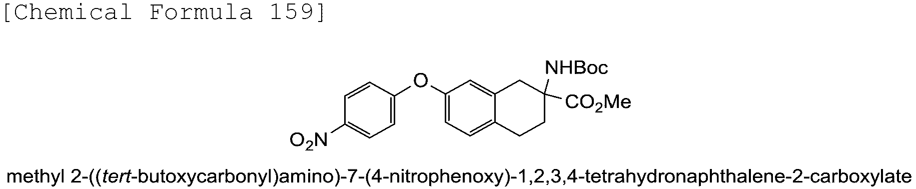

### Step 2 (Steps V-VI)

### 2- Production of carboxy-7-(4-nitrophenoxy)-1,2,3,4-tetrahydronaphthalene-2-aminium chloride

The following compound was synthesized from the compound obtained in Step 1 in the same manner as in Steps 5 and 6 of Example 1-1.

1H-NMR (CD3OD); 2.17-2.22 (m, 1H), 2.41-2.47 (m, 1H), 2.90-2.97 (m, 1H), 3.05 (d, J = 17.5 Hz, 1H), 3.04-3.10 (m, 1H), 3.53 (d, J = 17.0 Hz, 1H), 6.98-7.01 (m, 2H), 7.06-7.09 (m, 2H), 7.30 (d, J = 8.0 Hz, 1H), 8.21-8.24 (m, 2H).

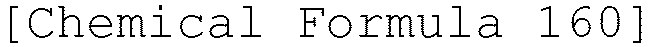

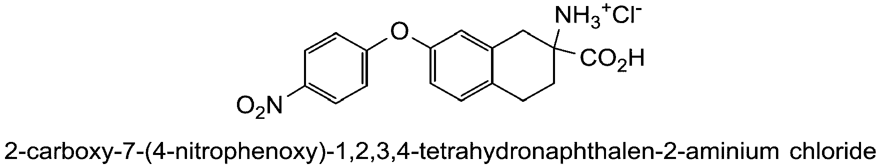

### (Example 1-69)

### 2- Preparation of Carboxy-7-(2-(naphthalen-1-yl)-4-nitrophenoxy)-1,2,3,4-tetrahydronaphthalene-2-aminium chloride (Compound 1-118)

### Step 1 (Steps I-IV)

### Preparation of methyl 2-((tert-butoxycarbonyl)amino)-7-(2-(naphthalen-1-yl)-4-nitrophenoxy)-1,2,3,4-tetrahydronaphthalene-2-carboxylate

The following compound was obtained in the same manner as in Step 1 of Example 1-68, except that 1-fluoro-4-nitrobenzene was changed to 1-(2-fluoro-5-nitrophenyl) naphthalene.

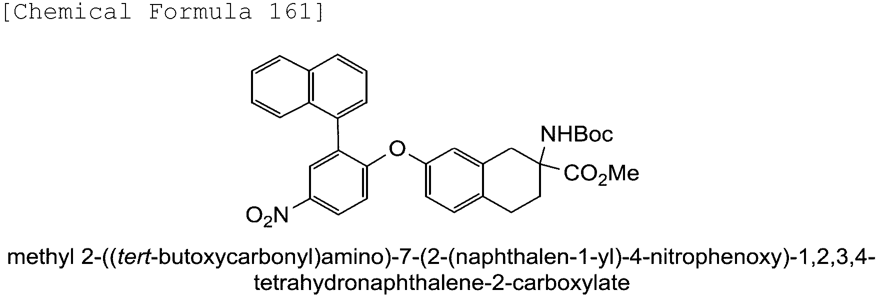

### Step 2 (Steps V-VI)

### 2- Production of carboxy-7-(2-(naphthalen-1-yl)-4-nitrophenoxy)-1,2,3,4-tetrahydronaphthalene-2-aminium chloride

The following compound was synthesized from the compound obtained in Step 1 in the same manner as in Steps 5 and 6 of Example 1-1.

HRMS (ESI) m/z calcd for C27H23N2O5 [M H] 455.1607 found 455.1908.

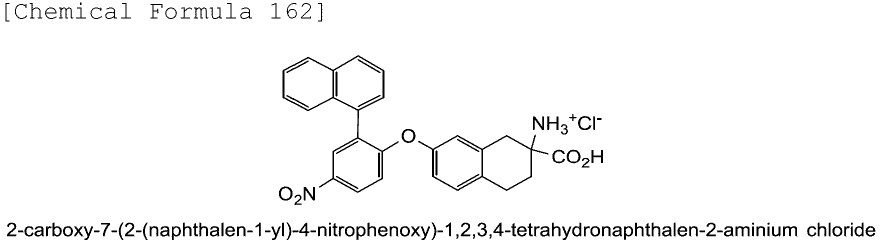

### (Example 2-1)

### Production of 2-carboxy-6-(naphthalene-2-yloxy)-1,2,3,4-tetrahydronaphthalene-2-aminium chloride

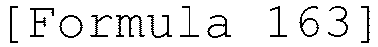

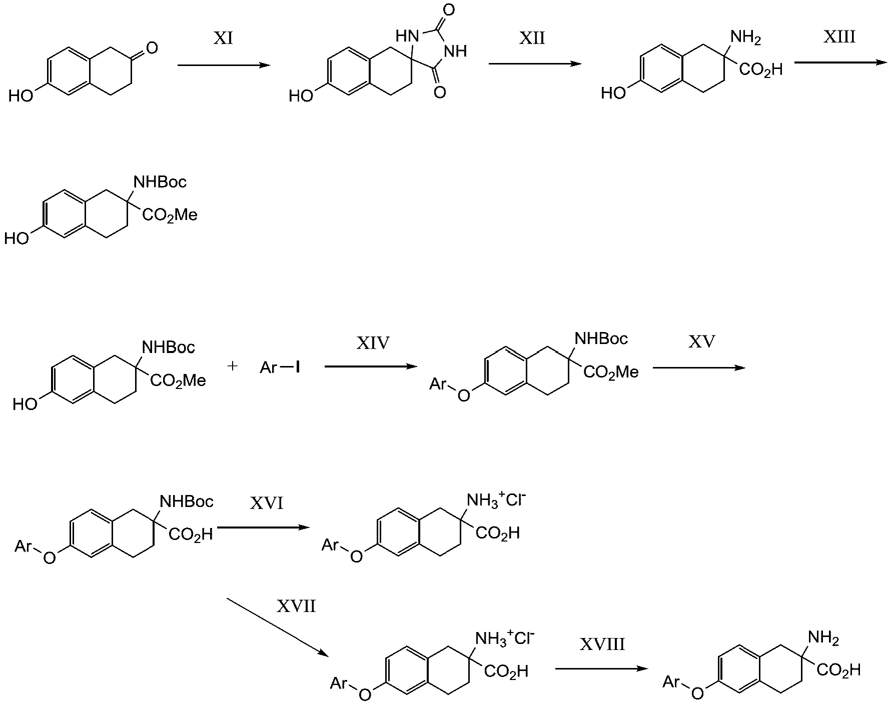

### Step 1 (Step XI-XIII)

### Production of methyl 2-((tert-butoxycarbonyl)amino)-6-hydroxy-1,2,3,4-tetrahydronaphthalene-2-carboxylate (Compound 2-1)

The following compound was obtained in the same manner as in Steps 1 to 3 in Example 1, except that 7-hydroxy-2-tetralone in Step 1 of Example was changed to 6-hydroxy-2-tetralone.

¹H-NMR (CDCl₃); 1.42 (s, 9H), 2.05-2.13 (m, 1H), 2.50 (brs, 1H), 2.76-2.79 (m, 2H), 2.85 (d, J = 15.5 Hz, 1H), 3.15 (d, J = 16.0 Hz, 1H), 3.77 (s, 3H), 4.80 (brs, 1H), 6.59 (s, 1H), 6.62 (dd, J = 2.0, 8.0 Hz, 1H), 6.90 (d, J = 8.0 Hz, 1H).

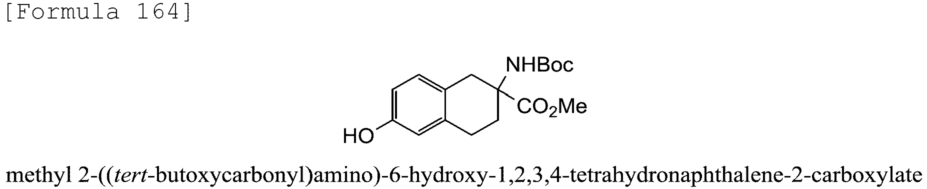

### Step 2 (Step XIV)

### Production of methyl 2-((tert-butoxycarbonyl)amino)-6-(naphthalene-2-yloxy)-1,2,3,4-tetrahydronaphthalene-2-carboxylate

The following compound was obtained in the same manner as in Step 4 in Example 1-1.

¹H-NMR (CDCl₃); 1.43 (s, 9H), 2.10-2.16 (m, 1H), 2.49 (brs, 1H), 2.79-2.82 (m, 2H), 2.94 (d, J = 16.5 Hz, 1H), 3.25 (d, J = 16.5 Hz, 1H), 3.78 (s, 3H), 4.81 (brs, 1H), 6.81 (d, J = 2.5 Hz, 1H), 6.86 (dd, J = 2.0, 8.0 Hz, 1H), 7.04 (d, J = 8.0 Hz, 1H), 7.26 (dd, J = 2.5, 7.0 Hz, 1H), 7.32 (d, J = 2.5 Hz, 1H), 7.39-7.42 (m, 1H), 7.44-7.47 (m, 1H), 7.72 (d, J = 8.0 Hz, 1H), 7.81-7.84 (m, 2H).

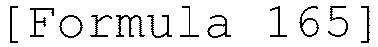

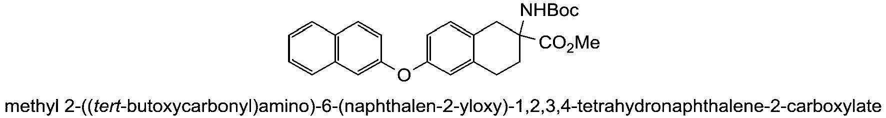

### Step 3 (Step XV-XVI)

### Production of 2-carboxy-6-(naphthalene-2-yloxy)-1,2,3,4-tetrahydronaphthalene-2-aminium chloride

The compound obtained in Step 2 was treated in the same manner as in Steps 5 and 6 in Example 1 to synthesize the following compound.

¹H-NMR (CD₃OD) ; 2.13-2.18 (m, 1H), 2.39-2.45 (m, 1H), 2.85-2.92 (m, 1H), 2.99-3.05 (m, 1H), 3.04 (d, J = 17.0 Hz, 1H), 3.50 (d, J = 16.5 Hz, 1H), 6.90 (d, J = 2.0 Hz, 1H), 6.93 (dd, J = 2.0, 8.0 Hz, 1H), 7.20 (d, J = 8.5 Hz, 1H), 7..23 (dd, J = 2.0, 9.0 Hz, 1H), 7.29 (d, J = 2.0, 1H), 7.38-7.41 (m, 1H), 7.43-7.46 (m, 1H), 7.70 (d, J = 8.0 Hz, 1H), 7.83-7.88 (m, 2H).

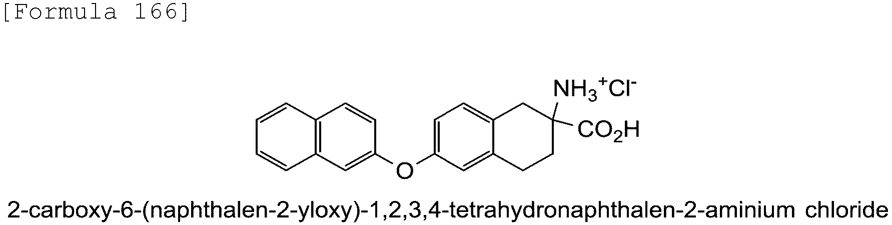

### (Example 2-2)

### Production of 3-(4-((6-ammonio-6-carboxy-5,6,7,8-tetrahydronaphthalene-2-yl)oxy)phenyl)pyridine-1-ium chloride (Compound 1-22)

### Step 1 (Step I-IV)

### Production of methyl 2-((tert-butoxycarbonyl)amino)-6-(4-(pyridine-3-yl)phenoxy)-1,2,3,4-tetrahydronaphthalene-2-carboxylate

The following compound was obtained in the same manner as in Steps 1 to 4 in Example 1-1, except that 2-iodonaphthalene in Step 4 of Example was changed to 3-(3-iodophenyl)pyridine.

¹H-NMR (CDCl₃); 1.43 (s, 9H), 2.10-2.16 (m, 1H), 2.48 (brs, 1H), 2.80-2.82 (m, 2H), 2.94 (d, J = 16.5 Hz, 1H), 3.25 (d, J = 16.5 Hz, 1H), 3.78 (s, 3H), 4.81 (brs, 1H), 6.81 (d, J = 2.0 Hz, 1H), 6.85 (dd, J = 2.5, 8.0 Hz, 1H), 7.02-7.05 (m, 2H), 7.23 (t, J = 2.0 Hz, 1H), 7.32 (dd, J = 2.0, 7.0 Hz, 1H), 7.35-7.37 (m, 1H), 7.44 (t, J = 8.0 Hz, 1H), 7.85 (dt, J = 2.0, 8.0 Hz, 1H), 8.59 (dd, J = 2.0, 5.5 Hz, 1H), 8.82 (d, J = 1.5 Hz, 1H).

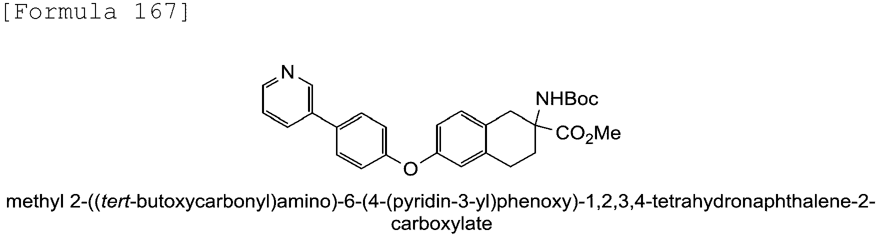

### Step 2 (Step V)

### Production of 2-((tert-butoxycarbonyl)amino)-6-(4-(pyridine-3-yl)phenoxy)-1,2,3,4-tetrahydronaphthalene-2-carboxylic acid

The above compound was treated in the same manner as in Step 5 in Example 1-1 to synthesize the following compound. This carboxylic acid compound was used as it was for the next step without purification.

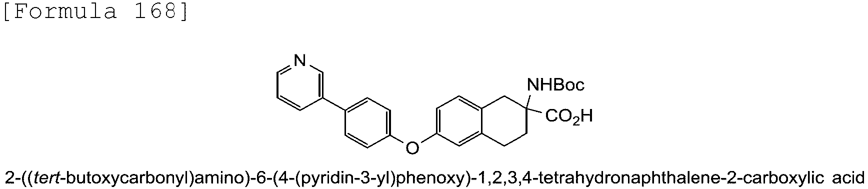

### Step 3 (Step VII)

### Production of 3-(4-((6-ammonio-6-carboxy-5,6,7,8-tetrahydronaphthalene-2-yl)oxy)phenyl)pyridine-1-ium chloride

To the above compound (300 mg, 0.651 mmol), 10% hydrochloric acid (15 mL) was added, and the mixture was stirred under reflux for 1 hour. The precipitate obtained after cooling was collected by filtration to obtain a target compound (190 mg, yield 67%) described below.

¹H-NMR (CD₃OD); 2.15-2.20 (m, 1H), 2.38-2.44 (m, 1H), 2.88-2.95 (m, 1H), 3.00-3.06 (m, 1H), 3.06 (d, J = 17.0 Hz, 1H), 3.49 (d, J = 16.5 Hz, 1H), 6.93-6.94 (m, 2H), 7.14-7.16 (m, 1H), 7.22 (dd, J = 2.0, 9.0 Hz, 1H), 7.50 (t, J = 1.0 Hz, 3H), 7.57-7.59 (m, 2H), 8.18 (dd, J = 6.0, 7.5 Hz, 1H), 8.86 (d, J = 5.5 Hz, 1H), 8.90-8.93 (m, 1H), 9.19 (d, J = 2.5 Hz, 1H).

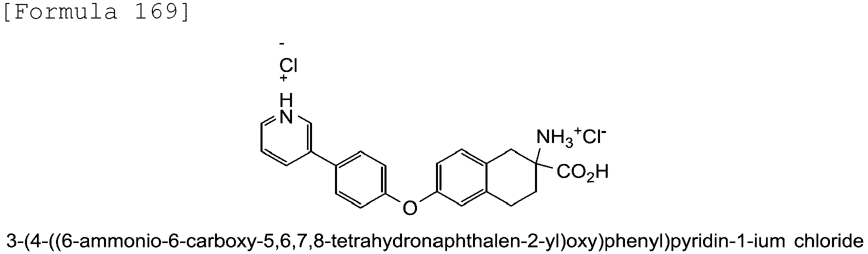

### (Example 3)

### Synthesis of tetrahydronaphthalene derivative for BNCT

As a compound that can be used for BNCT, for example, compounds as described below can be synthesized.

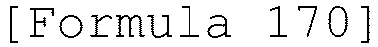

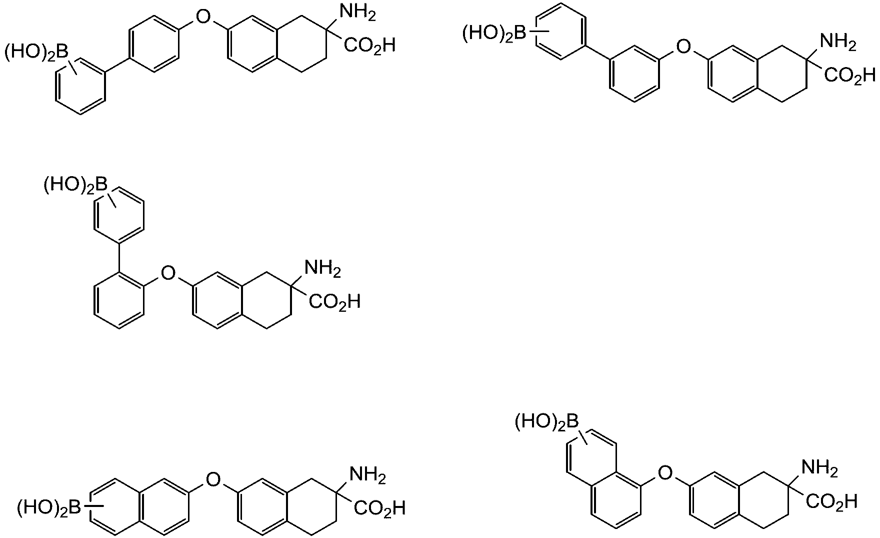

As a route of synthesis, the following protocol can be adopted.

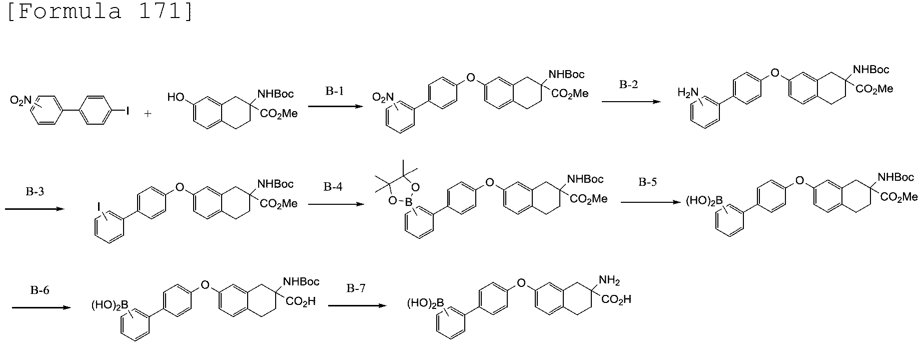

Step B-1 can employ the same method as in Step 4 (Step IV) of Example 1-1.

Step B-2 is a step of reducing by a standard method such as hydrogenation in the presence of Pd-C, and Step B-3 is a step of iodinating by a Sandmeyer type synthesis method. Step B-4 is a step of reacting with bis(pinacolato)diboron in the presence of a Pd catalyst such as a base or Pd(PPh₃)₄. The compound thus obtainable can be depinacolated by a standard method as shown in Step B-5. Synthesis can be performed by alkali saponification in Step B-6, and de-Boc reaction by an acid such as HCl-dioxane in Step B-7.

### (Example 4)

### Synthesis of ²¹¹At-labeled tetrahydronaphthalene derivative

As a compound that can be used for cancer treatment using a radioisotope, for example, compounds as described below can be synthesized.

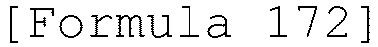

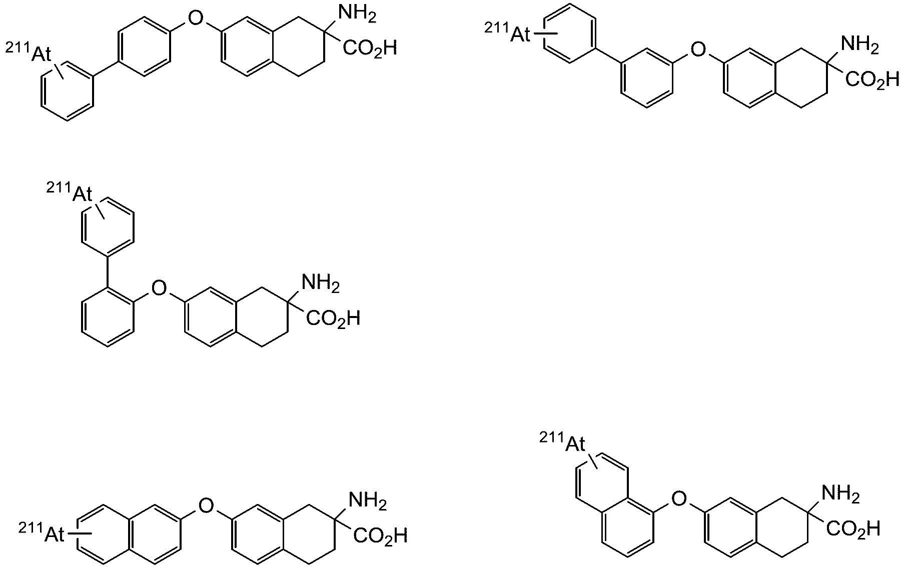

### (Example 5)

### Synthesis of F-labeled tetrahydronaphthalene derivative

As a compound that can be used for cancer diagnosis using a radioisotope, for example, compounds as described below can be synthesized.

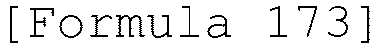

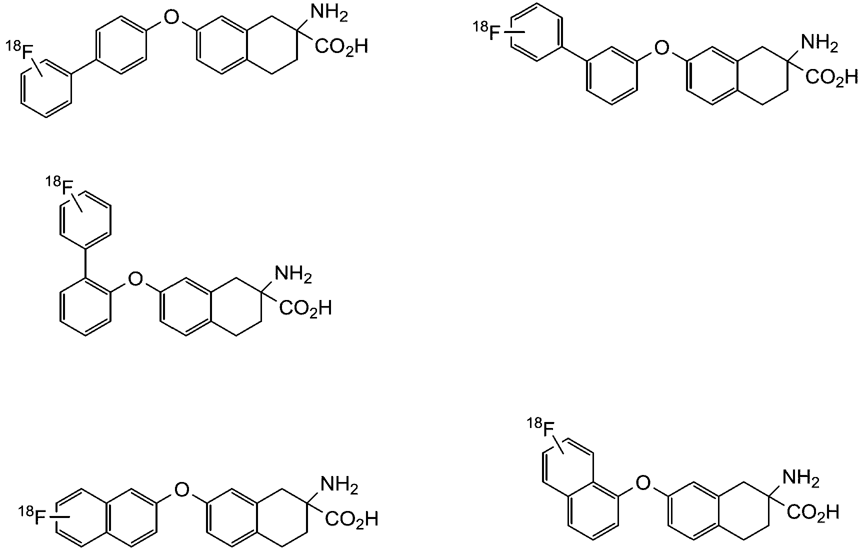

### (Example 6)

### Synthesis of tetrahydronaphthalene derivative for BNCT

As a compound that can be used for BNCT, for example, compounds as described below can be synthesized.

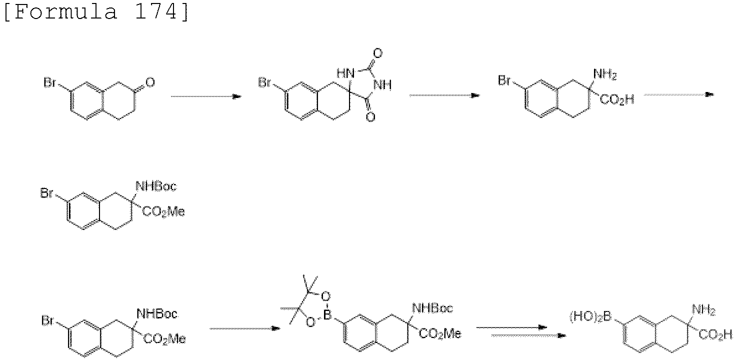

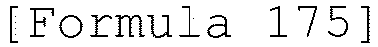

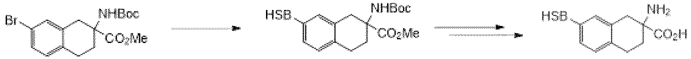

### (Example 7)

### Synthesis of ²¹¹At-labeled tetrahydronaphthalene derivative

As a compound that can be used for cancer treatment using a radioisotope, for example, a solution containing ²¹¹At can be reacted with the compound having boronic acid obtained in Example 6, and ²¹¹At can be introduced instead of boronic acid at the same position.

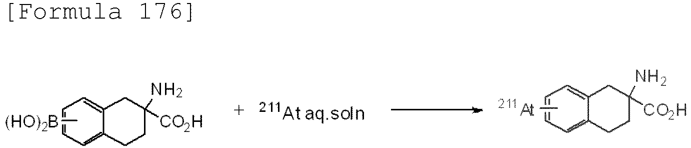

### (Example 8)

### Synthesis of labeled tetrahydronaphthalene derivative

As a compound that can be used for cancer diagnosis using a radioisotope, for example, compounds as described below can be synthesized.

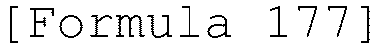

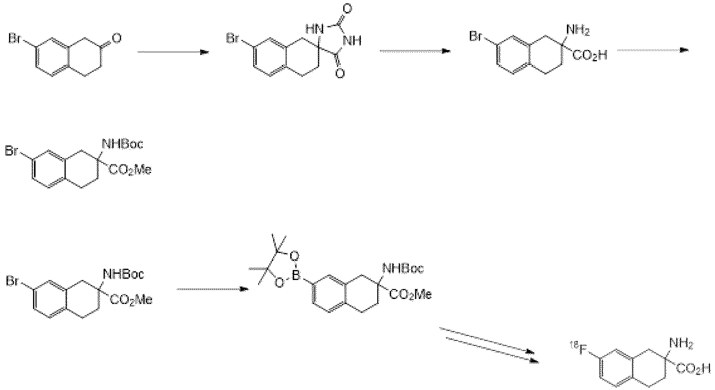

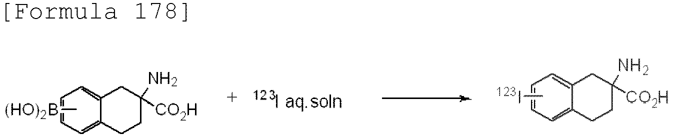

### [Test Example]

### (1) Production of human-LAT1 and LAT2 stably expressing HEK293 cell lines

HEK293 cells stably expressing human LAT1 and LAT2 were produced, respectively.

A shuttle vector DNA (LAT1: pcDNA3.1(+)-hLAT1, LAT2: pcDNA3.1(+)-hLAT2; expression vector pcDNA3.1(+) was produced by Invitrogen Corp.) having ampicillin and neomycin resistance markers and having full-length cDNA of human LAT1 or LAT2 inserted under a promoter of CMV (cytomegalovirus) was constructed. This vector was transfected into the HEK293 cells by a method using Lipofectamine (registered trademark) 2000 (Invitrogen Corp.) according to the manufacturer's instructions. Thereafter, stably expressing cell clones of transgene were selected by a limiting dilution method in the presence of 0.9 mg/mL of Geneticin (registered trademark), and among the obtained cell clones, cell clones having a high uptake of [¹⁴C]L-leucine and maintaining stable expression even after 10 passages were obtained. These cells were used for evaluating affinity and selectivity for LAT1 and LAT2.

### <Test Example 1> L-leucine uptake inhibition test using human-LAT1 stably expressing HEK293 cell line

1-1. It has been confirmed that LAT1 is responsible for L-leucine uptake in the absence of sodium ions in the human-LAT1 stably expressing HEK293 cell line. The human-LAT1 stably expressing HEK293 cell line was seeded in a 24-well plate at 1×10⁵ Cell/Well, and the [¹⁴C]L-leucine uptake inhibition test using the compound was performed after 48 hours. After the medium was removed and the cells were washed with Hank's balanced salt solution (Na⁺-free HBSS) not containing sodium ions, the cells were cultured in Na⁺-free HBSS for 10 minutes. Thereafter, 1 µM of [¹⁴C]L-leucine was taken up in Na⁺-free HBSS in the presence of a test compound (0.1, 1.0, 10, 0 µM in each case), which is a racemic form, for 1 minute, and then the cells were washed three times with ice-cooled Na⁺-free HBSS. To the well, 500 µL of 1 M NaOH was added, and after 30 minutes, the cells were lysed, and the cell lysate was collected. The amount of [¹⁴C]L-leucine in the cell lysate was determined using a liquid scintillator. The amount of protein in the cell lysate was measured using BCA Assay Kit (Thermofisher Scientific), and the uptake amount of [¹⁴C]L-leucine per 1 mg of protein in the cell per minute was calculated.

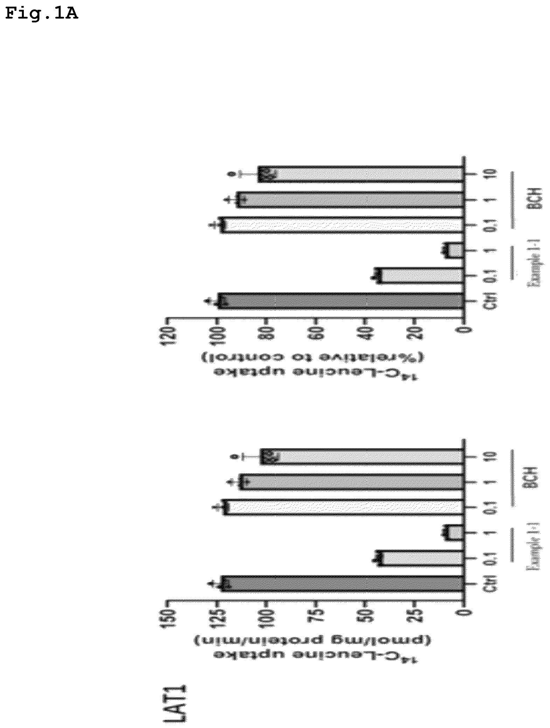

As a result, it was revealed that the test compound suppressed the uptake of [¹⁴C]L-leucine in the human-LAT1 stably expressing HEK293 cell line. As can be seen from Fig. 1A, the compound of Example 1-1 (Compound 1-5) suppressed the uptake of labeled leucine in the human-LAT1 stably expressing HEK293 cell line in a concentration-dependent manner, the suppression rate was as strong as about 70% at 0.1 µM of the compound (racemic form), and the 50% suppression concentration (racemic form) was 0.1 µM or less.

The degree of LAT1 inhibition of the compound obtained in the present invention was compared with BCH (BCH, 2-aminobicyclo-(2,2,1)-heptane-2-carboxylic acid), which is an existing LAT1 inhibitor. In the [¹⁴C]L-leucine uptake inhibition test described above, the degrees of inhibition of [¹⁴C]L-leucine uptake inhibition using the compound (racemic form) obtained in Examples and BCH (0.1, 1.0, 10, 0 µM for both) were compared. As shown in Fig. 1A, 0.1 to 10 µM of BCH showed weak [¹⁴C]L-leucine uptake inhibition via LAT1. In the table, a test compound having a higher inhibitory effect than BCH is denoted by A, a test compound having the same inhibitory effect as BCH is denoted by B, and a test compound having a lower inhibitory effect than BCH is denoted by C.

**[Table 1]**

| Example | Structural Formula | Assay Data |
|---|---|---|
| Example 1-1 | 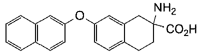 | A |
| Example 1-2 | 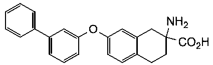 | A |
| Example 1-3 | 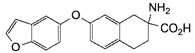 | A |
| Example 1-4 | 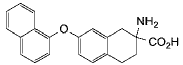 | A |
| Example 1-5 | 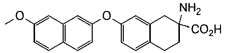 | A |
| Example 1-6 | 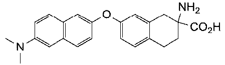 | A |
| Example 1-7 | 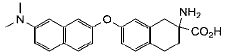 | A |
| Example 1-8 | 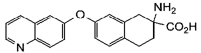 | A |
| Example 1-9 | 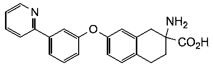 | A |
| Example 1-10 | 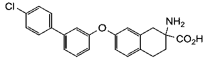 | A |
| Example 1-11 | 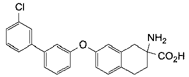 | A |
| Example 1-12 | 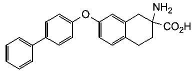 | A |
| Example 1-13 | 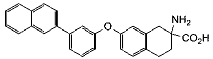 | A |
| Example 1-16 | 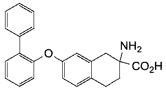 | A |
| Example 1-30 | 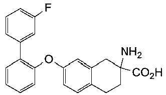 | A |
| Example 1-32 | 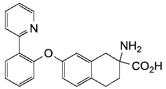 | A |
| Example 1-33 | 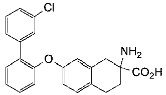 | A |
| Example 1-35 | 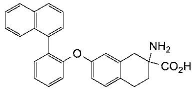 | A |
| Example 1-39 | 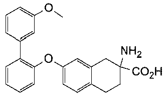 | A |
| Example 1-42 | 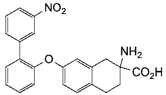 | A |
| Example 1-43 | 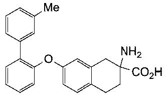 | A |
| Example 1-45 | 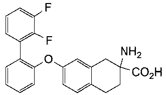 | A |
| Example 1-46 | 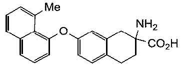 | A |
| Example 1-47 | 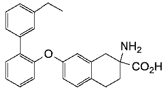 | A |
| Example 1-48 | 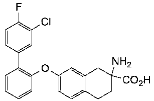 | A |
| Example 1-49 | 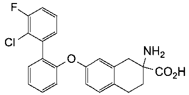 | A |
| Example 1-50 | 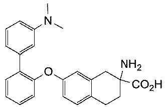 | A |
| Example 1-52 | 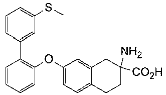 | A |
| Example 1-53 | 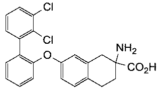 | A |
| Example 1-54 | 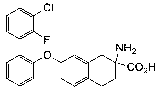 | A |
| Example 1-55 | 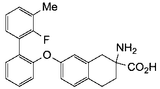 | A |
| Example 1-56 | 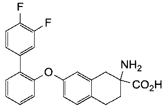 | A |
| Example 1-57 | 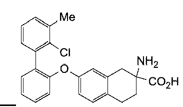 | A |
| Example 1-58 | 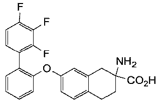 | A |
| Example 1-59 | 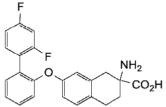 | A |
| Example 1-60 | 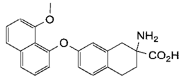 | A |
| Example 1-61 | 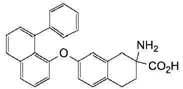 | A |
| Example 1-62 | 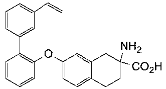 | A |
| Example 1-63 | 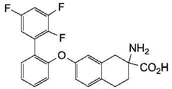 | A |
| Example 1-64 | 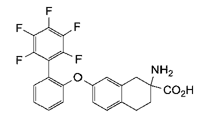 | A |
| Example 1-65 | 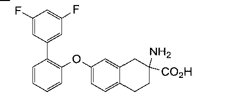 | A |
| Example 1-66 | 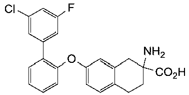 | A |
| Example 1-67 | 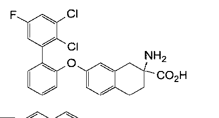 | A |
| Example 1-69 | 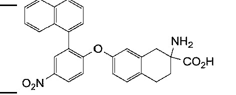 | A |
| Example 2-1 | 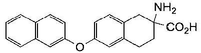 | A |
| Example 2-2 | 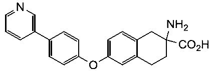 | B |

All the compounds of Example 1-14 to Example 1-64 were tested similarly and the activity was A rank for all the compounds. A plurality of compounds having extremely excellent effects were also found.

The compounds having particularly remarkable effects were, for example, the compounds of Example 1-1, Example 1-4, Example 1-45, Example 1-46, Example 1-53, Example 1-54, Example 1-55, Example 1-57, Example 1-58, and Example 1-63.

### <Test Example 2> Alanine uptake inhibition test using human-LAT2 stably expressing HEK293 cell line

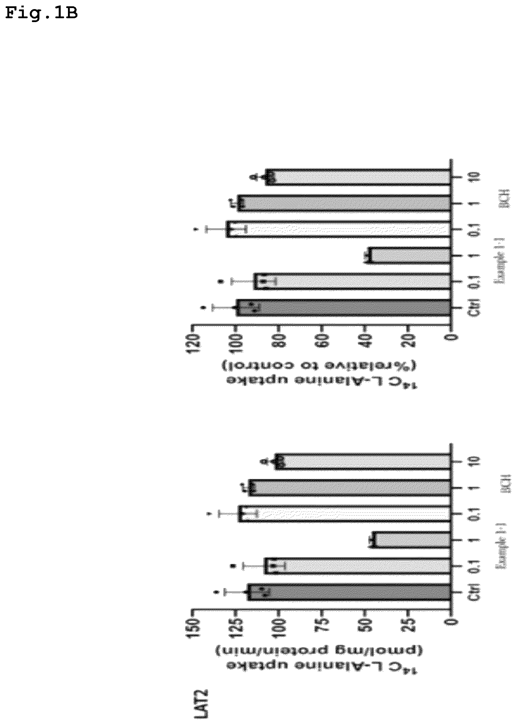

[¹⁴C]L-alanine uptake inhibition using the compound of Example 1-1 was performed using the human-LAT2 stably expressing HEK293 cell line. In the same manner as in the [¹⁴C]L-leucine uptake inhibition experiment in the human-LAT1 stably expressing HEK293 cell line, the human-LAT2 stably expressing HEK293 cell line was seeded in a 24-well plate at 1×10⁵ Cell/Well, and after 48 hours, 1 µM of [¹⁴C]L-alanine was taken up in Na⁺-free HBSS in the presence of the compound (0.1, 1.0, 10, 0 µM in each case), which is a racemic form, of Example 1-1 for 1 minute, then the uptake amount of [¹⁴C]L-alanine and the protein amount of the cell lysate were measured, and the uptake amount of [¹⁴C]L-alanine per 1 mg of protein in the cell per minute was determined. As a result, in this cell, the compound suppressed the alanine uptake at a 50% suppression concentration of about 1 µM, and was found to have a weaker inhibitory action than [¹⁴C]L- leucine uptake inhibition in the LAT1 stably expressing HEK293 cell line (Fig. 1B).

### <Test Example 3> Leucine uptake persistence inhibitory effect after compound removal

The uptake of [¹⁴C]L-leucine remaining after the compound of the present invention was caused to act in advance and removed in the human-LAT1 highly expressing cell line was examined.

In the same manner as in the uptake inhibition test, the cell line was seeded in a 24-well plate, and an MEM (Minimum Essential Medium) medium containing 20 µM of the compound (racemic form) was added to the wells and allowed to act for 30 minutes. An MEM medium containing no compound was used as a control.

After the compound was caused to act, the cells were washed 3 times with the MEM medium and cultured in the MEM medium for an additional 30 minutes. Thereafter, washing was performed 3 times with Na⁺Free-HBSS, and 1 µM of [¹⁴C]L-leucine dissolved in Na⁺Free-HBSS was taken up for 1 minute. The cells were washed 3 times with ice-cooled Na⁺Free-HBSS, and the amount of [¹⁴C]L-leucine taken up in the cells was determined with a liquid scintillator. In the same manner as in other tests, the amount of protein in the cell fluid was measured, and the uptake amount of [¹⁴C]L-leucine per 1 mg of protein in the cell fluid per minute was determined.

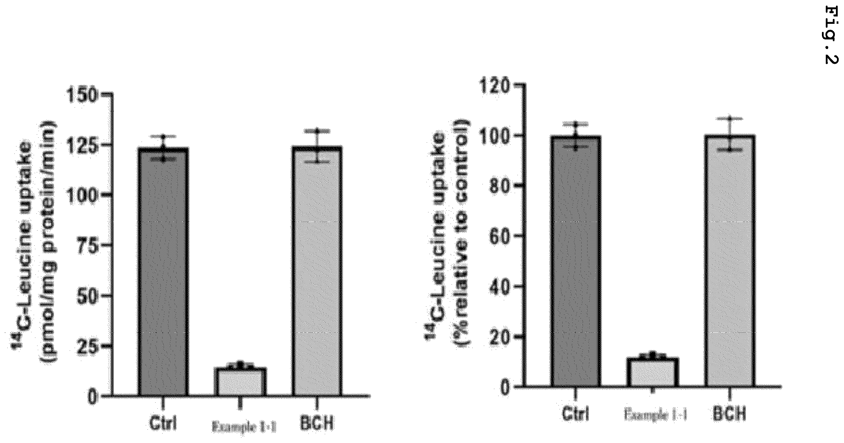

Fig. 2 shows comparison results between Example 1-1 and BHC.

It was found that in the cells to which each of the compounds of Examples had been previously caused to act, the uptake of [¹⁴C]L-leucine was strongly suppressed, and the inhibitory effect has been sustained for 30 minutes.

All the compounds synthesized in Examples were created so as to bind to a distal pocket (see Non-Patent Document 2), which is a binding pocket of LAT1 revealed by cryoelectron microscopic analysis.

The strong LAT1 inhibitory effect that has been sustained even after the end of compound exposure, as verified in Test Example 3, is considered to be the property that the compound is endowed by binding to the distal pocket of LAT1.

When similar tests were also performed in other Examples, a compound having a more remarkable effect was found particularly as compared with Example 1-1. Examples of such a compound include the following compounds:
compounds of Example 1-30, Example 1-33, Example 1-35, Example 1-42, Example 1-43, Example 1-45, Example 1-46, Example 1-48, Example 1-49, Example 1-50, Example 1-52, Example 1-53, Example 1-54, Example 1-55, Example 1-57, Example 1-58, Example 1-59, Example 1-62, and Example 1-63.

### <Test Example 4> Cell proliferation inhibitory effect using mouse colorectal cancer cells

Mouse colorectal cancer-derived CT26 cells were adjusted to 0.5×10⁴ cells/mL, seeded in a 96-well plate at 1000 cells/well, and cultured in a 10% fetal bovine serum-added RPMI 1640 medium at 5% CO₂ and 37°C. The culture medium was changed 24 hours after the start of the culture, and the medium containing the compound of Example was replaced to examine the action of the compound on cell proliferation. As the compound, a concentration of 0.01, 0.03, 0.1, 0.3, 1,3, 10, or 30 µM was used. As a control, the cells were cultured in a medium containing no compound. The number of cells was evaluated by CCK-8 assay (Cell Counting Kit-8, DOJINDO LABORATORIES) 72 hours after the start of the compound treatment, and the compound concentration (IC₅₀ value) giving a proliferation inhibition rate of 50% as compared with the control was calculated.

Suppression of cell proliferation was observed depending on the concentration of the compound. The compound concentration (IC₅₀ value) giving a proliferation inhibition rate of 50% as compared with the control was calculated. It was revealed that the compounds of Examples exhibited a strong cytostatic effect at a low concentration.

In Table 2, with respect to the compounds of Examples, a compound having an IC₅₀ value of 0.9 µmol/L or less was evaluated as A, a compound having an IC₅₀ value of 0.9 to 5 µmol/L was evaluated as B, and a compound having an IC₅₀ value higher than 5 µmol/L was evaluated as C. Of the evaluated compounds of Examples, in many compounds, an extremely low IC₅₀ value of 0.9 µmol/L or less was obtained.

### [Table 2]

**[Table 2]**

| Example | Evaluation of IC50 |
|---|---|
| Example1-1 | A |
| Example1-4 | A |
| Example1-16 | A |
| Example1-30 | A |
| Example1-32 | B |
| Example1-33 | A |
| Example1-35 | A |
| Example1-39 | A |
| Example1-42 | A |
| Example1-43 | A |
| Example1-45 | A |
| Example1-46 | A |
| Example1-47 | A |
| Example1-48 | A |
| Example1-49 | A |
| Example1-50 | A |
| Example1-52 | A |
| Example1-53 | A |
| Example1-54 | A |
| Example1-55 | A |
| Example1-56 | A |
| Example1-57 | A |
| Example1-58 | A |
| Example1-59 | A |
| Example1-60 | A |
| Example1-61 | A |
| Example1-62 | A |
| Example1-63 | A |
| Example1-64 | A |
| Example1-65 | A |
| Example1-66 | A |
| Example1-67 | A |
| Example1-68 | B |
| Example1-69 | A |

### <Test Example 5> BNCT-enhancing effect by L-BPA confinement

### · Examination of BNCT-enhancing effect using cultured cells

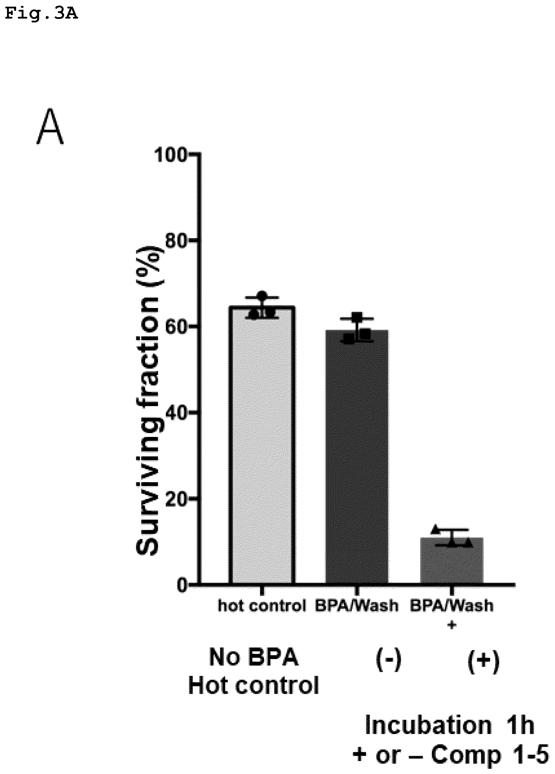

Human breast cancer cells MCF7 were plate-cultured in an RPMI medium for 48 hours, then replaced with an RPMI medium containing 100 µM of L-[10B]BPA, and cultured for 15 minutes to allow the L-[10B]BPA to be taken up in the cells. After washing with HBSS, the cells were cultured in an RPMI medium for 60 minutes, and after further washing with HBSS, the cells were peeled off and stored in HBSS at room temperature for 1.5 hours ((-) in Fig. 3A). L-BPA was taken up in the cells, washed with HBSS and then cultured in an RPMI medium containing the compound of Example 1-1 (10 µM) obtained in the present invention for 60 minutes, and after further washing with HBSS, the cells were peeled off and stored in HBSS at room temperature for 1.5 hours ((+) in Fig. 3A). The cells were cultured in an RPMI medium containing no L-[10B]BPA for 15 minutes, and after washing with HBSS, the cells were peeled off and stored in HBSS at room temperature for 1.5 hours ((No BPA Hot control) in Fig. 3A). These three cell samples were irradiated with neutron rays (5 MW for 4 minutes). After irradiation, in order to measure viable cells, the cells were plate-cultured and the number of colonies generated after 2 weeks was measured. The number of viable cells was evaluated as surviving fraction (%) (Fig. 3A).

When L-[10B]BPA was taken up in the cells and then not treated with the compound of Example 1-1, the L-[10B]BPA has leaked from the cells, and cell death caused by neutron irradiation was about No BPA Hot control, however, when LAT1 was inhibited by treatment with the compound of Example 1 -1 obtained in the present invention after the L-[10B]BPA was taken up in the cells, the L-[10B]BPA was retained in the cells, and the neutron irradiation effect was enhanced, so that a large BNCT-enhancing effect was obtained.

### Examination of BNCT-enhancing effect using mouse allograft tumor model

Mouse triple negative breast cancer cells 4T1 and mouse colorectal cancer cells CT26 were plate-cultured and then transplanted subcutaneously into the lower limbs of BALB/c mice. After 1 week, 400 mg/kg of L-[10B]BPA or a solvent as a control was intravenously administered through the tail vein. In Group 1, the compound of Example 1-45 was administered intravenously at a dose of 25 mg/kg after 1 hour and 1.5 hours from the administration of L-[10B]BPA. In Group 2, the solvent was administered instead of L-[10B]BPA, and after 1 hour and 1.5 hours, the compound of Example 1-45 obtained in the present invention was administered intravenously at a dose of 25 mg/kg. In Group 3, the solvent was administered intravenously instead of the compound of Example 1-45 after 1 hour and 1.5 hours from the administration of L-[10B]BPA. For Groups 1, 2, and 3, the lower limbs were irradiated with neutron rays under anesthesia after 2.5 hours from L-[10B]BPA administration or solvent administration as the control (5 MW for 30 minutes). The tumors were excised after 11 days from irradiation and the volumes thereof were compared.

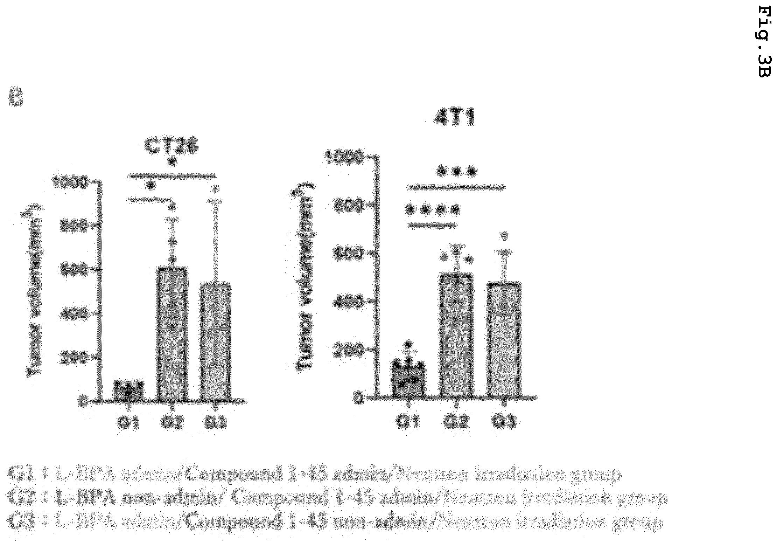

The results are shown in Fig. 3B. When L-[10B]BPA was administered and then not treated with the compound of Example 1-45 (Group 3), the L-[10B]BPA has leaked from tumor cells after being taken up, and the antitumor effect by neutron irradiation was about the same as that in a case where the L-[10B]BPA was not administered (Group 2). Therefore, in Group 3, no BNCT effect was obtained despite the administration of L-[10B]BPA. On the other hand, when the L-[10B]BPA was administered and then treated with the compound of Example 1-45 (Group 1), the L-[10B]BPA was taken up and then retained in the tumor cells, and a large antitumor effect by neutron irradiation was obtained. The compound obtained in the present invention having a strong sustained inhibitory effect on LAT1 has shown to have a BNCT-enhancing effect.

## Claims

1. A compound represented by Formula (I) below or a pharmaceutically acceptable salt thereof: 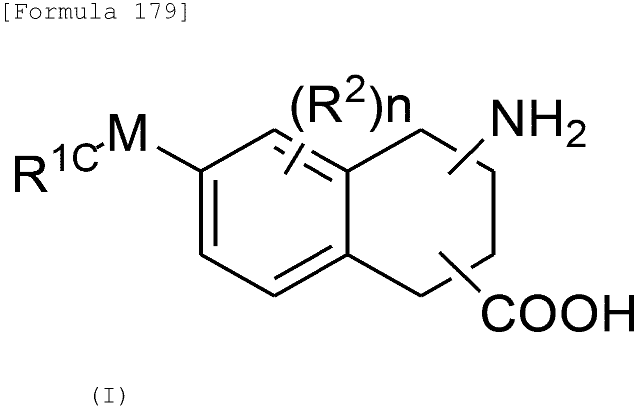
wherein in Formula (I),
M represents S, O, or NH;
R^{1C} represents any structure selected from the group consisting of a 5- to 7-membered aromatic heterocyclic group having a substituent, a C5- to 7-membered aromatic cyclic group having a substituent, and a substituted or unsubstituted 8- to 16-membered polycyclic group,
where, the substituent of the 5- to 7-membered aromatic heterocyclic group having the substituent or the C5- to 7-membered aromatic cyclic group having the substituent is 1 to 5 groups independently selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted biphenyl, substituted or unsubstituted pyridyl, substituted or unsubstituted naphthyl, halogen, hydroxy, cyano, amino, C1-6 alkylamino, di-(C1-6 alkyl)amino, C1-6 alkyl, C2-6 alkenyl, C2-6 alkynyl, C1-6 alkoxyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, sulfamoyl, ¹⁸F, ¹²³I, boron containing substituents, alpha-emitting nuclides and beta-emitting nuclides,
where, the substituent of the polycyclic group is 1 to 6 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, sulfamoyl, ¹⁸F, ¹²³I, boron containing substituents, alpha-emitting nuclides and beta-emitting nuclides;
or,
M is S, O, NH, or absent (simply represents a single bond), R^{1C} represents ¹⁸F, ¹²³I, a substituent including boron, an α radioactive nucleus and/or a β radioactive nucleus;
R² represents a group independently selected from the group consisting of halogen, hydroxy, cyano, amino, C1-6 alkylamino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl; and
n represents any integer of 0 to 5.

2. The compound or pharmaceutically acceptable salt thereof of claims 1, wherein the R^{1C} is a substituted or unsubstituted 8- to 16-membered polycyclic group, and the polycyclic group is a ring which may optionally contain N, O and/or S in addition to a C atom.

3. The compound or pharmaceutically acceptable salt thereof of claim 1, wherein
the R^{1C} is a 5- to 7-membered aromatic group having a substituent, and necessarily contains, as the substituent, at least one group selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted pyridyl, substituted or unsubstituted naphthyl, and substituted or unsubstituted biphenyl, and
where, when the benzyloxy, benzyl, phenyl, phenyl C1-6 alkyl, pyridyl, naphthyl, or biphenyl is substituted, the benzyloxy, benzyl, phenyl, phenyl C1-6 alkyl, pyridyl, naphthyl, or biphenyl is substituted with 1 to 5 groups independently selected from the group consisting of halogen, hydroxy, cyano, amino, C1-6 alkylamino, di-(C1-6 alkyl)amino, C1-6 alkyl, C2-6 alkenyl, C2-6 alkynyl, C1-6 alkoxyl, benzyloxy, benzyl, phenyl, phenyl C1-6 alkyl, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino, or di-(C1-6 alkyl)amino), a 3- to 8-membered non-aromatic heterocycle, morpholinocarbonyl, C3-8 cycloalkyl, C3-8 cycloalkylamino, 3- to 8-membered non-aromatic heterocycle-substituted amino, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, and sulfamoyl.

4. The compound or pharmaceutically acceptable salt thereof of claim 1, wherein
the R^{1C} is one selected from the group consisting of substituted or unsubstituted pyridylphenyl, substituted or unsubstituted biphenyl, substituted or unsubstituted biphenyl-substituted phenyl, substituted or unsubstituted naphthyl-substituted phenyl, substituted or unsubstituted naphthyl, substituted or unsubstituted quinoline-substituted phenyl, substituted or unsubstituted isoquinoline, substituted or unsubstituted isoquinoline-substituted phenyl, substituted or unsubstituted anthracene, substituted or unsubstituted anthracene-substituted phenyl, substituted or unsubstituted dithiazole ylideneaminonaphthyl, substituted or unsubstituted dithiazole ylideneaminobiphenyl, substituted or unsubstituted benzofuranyl, substituted or unsubstituted benzothiophenyl, substituted or unsubstituted acenaphthene, and substituted or unsubstituted dithiazole ylideneaminophenyl, and
the substituent in the case of the substitution is 1 to 5 groups selected from the group consisting of halogen, hydroxy, cyano, amino, C1-6 alkylamino, C1-6 alkyl, C2-6 alkenyl, C2-6 alkynyl, C1-6 alkoxy, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino), morpholinocarbonyl, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, C3-8 cycloalkyl, 3- to 8-membered non-aromatic heterocycle, C3-8 cycloalkylamino, 3- to 8-membered non-aromatic heterocycle-substituted amino, dialkylamino, and sulfamoyl.

5. The compound or pharmaceutically acceptable salt thereof of claim 1, wherein in Formula (I),
M represents O,
R^{1C} is a 5- to 7-membered aromatic group having a substituent, and necessarily contains, as the substituent, at least one group selected from the group consisting of substituted or unsubstituted benzyloxy, substituted or unsubstituted benzyl, substituted or unsubstituted phenyl, substituted or unsubstituted phenyl C1-6 alkyl, substituted or unsubstituted pyridyl, substituted or unsubstituted naphthyl, and substituted or unsubstituted biphenyl,
the substituent in the case of substitution is 1 to 5 groups selected from the group consisting of halogen, hydroxy, cyano, amino, C1-6 alkylamino, C1-6 alkyl, C2-6 alkenyl, C2-6 alkynyl, C1-6 alkoxy, C1-6 alkoxy C1-6 alkyl, nitro, C1-6 haloalkyl, carbamoyl, C1-6 alkylaminocarbonyl, di-(C1-6 alkyl)aminocarbonyl, C1-6 alkoxycarbonyl, C1-6 alkylcarbonyl, COOR¹⁰ (R¹⁰ is H or C1-6 alkyl, amino, C1-6 alkylamino), morpholinocarbonyl, C1-6 haloalkylsulfanyl, C1-6 haloalkylsulfinyl, C1-6 haloalkylsulfonyl, C1-6 alkylthio, C1-6 alkylsulfinyl, C1-6 alkylsulfonyl, aminosulfonyl, sulfo, C3-8 cycloalkyl, 3- to 8-membered non-aromatic heterocycle, C3-8 cycloalkylamino, 3- to 8-membered non-aromatic heterocycle-substituted amino, dialkylamino, and sulfamoyl, and
n represents 0.

6. An L-type amino acid transporter LAT1 selective inhibitor containing the compound or pharmaceutically acceptable salt thereof described in any one of claims 1 to 5.

7. A pharmaceutical composition for cancer treatment, containing the compound or pharmaceutically acceptable salt thereof described in any one of claims 1 to 5.

8. A composition for retaining L-BPA in a cell, containing the compound or pharmaceutically acceptable salt thereof of any one of claims 1 to 5.

9. A composition for cancer treatment, containing a compound represented by Formula (II) below or a pharmaceutically acceptable salt thereof: 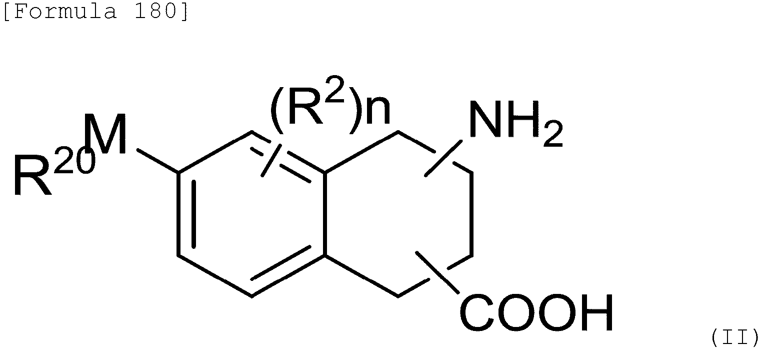
wherein, in Formula (II),
M represents S, O, NH, or absent (single bond); and
R²⁰ represents a radioisotope.

10. The composition for cancer treatment of claims 9, wherein the R²⁰ represents an α radioactive nucleus and/or a β radioactive nucleus.

11. The composition for cancer treatment of claim 10, wherein the α radioactive nucleus is ²¹¹At, and the β radioactive nucleus is ¹³¹I.

12. A drug for BNCT, containing a compound represented by Formula (III) below or a pharmaceutically acceptable salt thereof: 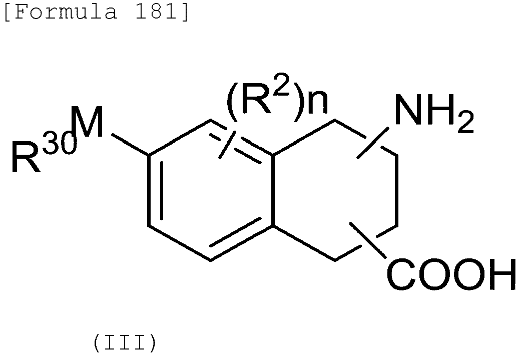
wherein, in Formula (III),
M represents S, O, NH, or absent (single bond); and
R³⁰ represents a substituent including boron.

13. The drug for BNCT of claim 12, wherein the R³⁰ represents boronic acid (-B(OH)₂), a boronic acid ester, a boronic acid amide, or a boron cluster.

14. A cancer diagnostic agent, containing a compound represented by Formula (IV) below or a pharmaceutically acceptable salt thereof: 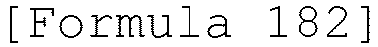 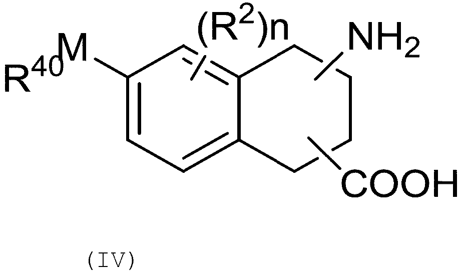
wherein, in Formula (IV),
M represents S, O, NH, or absent (single bond); and
R⁴⁰ represents a radioisotope.

15. The cancer diagnostic agent of claim 14, wherein the R⁴⁰ is ¹⁸F or ¹²³I .

16. The cancer diagnostic agent of claim 14 or 15, wherein the cancer diagnostic agent is a probe for PET or a probe for SPECT.

17. A sustained inhibitor of LAT1, containing the compound or pharmaceutically acceptable salt thereof described in any one of claims 1 to 5.

18. A composition for enhancing a BNCT effect, containing the compound or pharmaceutically acceptable salt thereof described in any one of claims 1 to 5.
